# EUROPEAN PATENT SPECIFICATION

(11) **EP 0 460 169 B1**
(45) Date of publication and mention of the grant of the patent: **05.06.1996**
(21) Application number: 91901496.9
(22) Date of filing: 19.12.1990
(51) Int. Cl.: C07D 311/58, C07D 335/06, A61K 31/35, A61K 31/38

(54) **NEW CHROMAN AND THIOCHROMAN DERIVATIVES**
NEUE CHROMAN- UND THIOCHROMANDERIVATE
NOUVEAUX DERIVES DU CHROMAN ET DU THIOCHROMAN

(30) Priority: 22.12.1989 SE 8904361
(43) Date of publication of application: 11.12.1991
(73) Proprietor: Astra Aktiebolag, 151 85 Södertälje (SE)
(72) Inventor: LARSSON, Lars-Gunnar, S-150 16 Hölö (SE); NOR EN, Rolf, S-151 54 Södertälje (SE); RENYI, Lucy, Anna, S-127 37 Skärholmen (SE); ROSS, Svante, Bertil, S-151 52 Södertälje (SE); SOHN, Daniel, Dungan, S-151 55 Södertälje (SE); SVENSSON, Björn, Eric, S-151 59 Södertälje (SE); THORBERG, Seth, Olov, S-153 00 Järna (SE)
(86) International application number: SE9000863
(87) International publication number: WO9109853

(56) References cited:
- EP-A- 0 222 996
- EP-A- 0 280 269
- WO-A-88/04654
- CHEMICAL ABSTRACT, Vol. 107, 1987, "Preparation of 3-aminodihydro/1/benzopyran and benzothiopyran derivatives as serotoninergic agonists", Abstract 39617j, & JP 62 59273 (CIBA-GEIGY A.G.) see especially reg.No. 109140-40-1.
- J. Med. Chem., Vol. 15, 1972, I.M. LOCKHART et al: "3-Chromanamine Hydrochlorides with Central Stimulant Activity", pages 863-865 see the whole article.

## Description

### Field of the Invention

The present invention relates to new substituted-3-aminochromans and thiochromans, enantiomers and salts thereof, processes for their preparation, pharmaceutical compositions containing said therapeutically active compounds as well as new intermediates useful in the preparation of the therapeutically active compounds and to the use of said active compounds in therapy.

An object of the invention is to provide compounds for therapeutic use, especially compounds having a therapeutic activity via the central nervous system (CNS). A further object is to provide compounds having a selective effect on the 5-hydroxy-tryptamine receptors in mammals including man.

### Prior art

Therapeutically useful 3-amino-dihydro-[1]-benzopyran and benzothiopyran having effect on 5-hydroxy tryptamine neurons in mammals are disclosed in EP 0222 996.

These compounds are defined by the formula 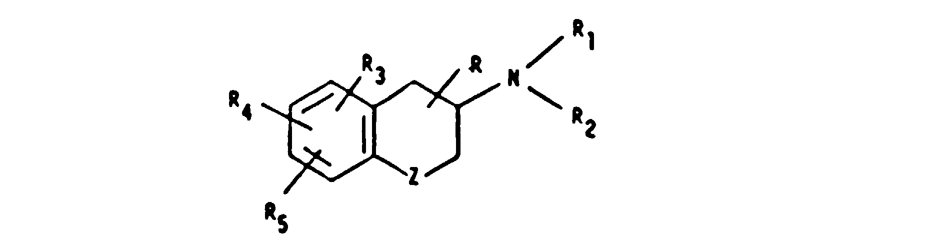 wherein Z is 0 or S;
R is hydrogen or loweralkyl;
R₁ is hydrogen, loweralkyl or arylloweralkyl;
R₂ is hydrogen, loweralkyl or arylloweralkyl; or R₁ and R₂ together form a ring with 4 - 6 carbon atoms;
R₃ is hydrogen, hydroxy, loweralkoxy, arylloweralkoxy, acyloxy or aryloxy when Z is S and R₃ is hydroxy, loweralkoxy, arylloweralkoxy, acyloxy or aryloxy when Z is 0 and R₃ is in 5- or 8-position when Z is O;
R₄ and R₅ are independently hydrogen, lower alkyl or halogen, and mono- or di-S-oxide thereof when Z is S, and pharmaceutically acceptable salts thereof.
3-Chromanamine hydrochlorides with two alkyl groups in the aromatic ring having central stimulating activities are described in J. Med. Chem. 15, p. 863-65 (1972).

### Disclosure of the Invention

The object of the present invention is to obtain new compounds which have a high affinity to the 5-hydroxy-tryptamine receptors in the central nervous system at the same time as they act as agonists, partial agonists or antagonists on the serotonin receptors.

Thus, a group of new compounds of the formula I of the present invention as well as the enantiomers and salts thereof are useful in therapeutic treatment of 5-hydroxytryptamine mediated states and disorders such as depression, anxiety, anorexia, senile dementia, Alzheimer's disease, migraine, termoregulator and sexual disturbances. Further aspects of the invention are related to the use of the compounds, enantiomers and salts thereof in pain control and in modulation of the cardiovascular system.

Thus, the invention provides compounds of the formula 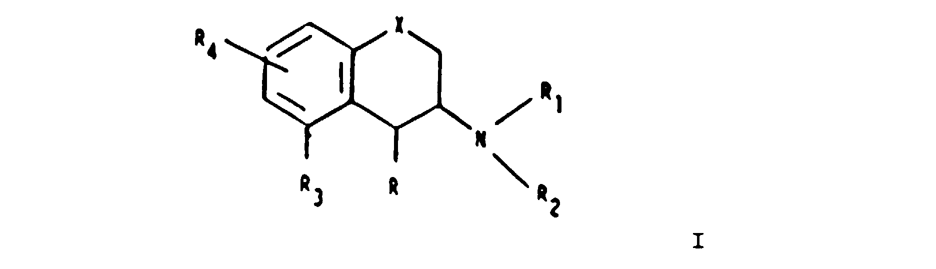 wherein
- X: is O or 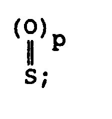
- p: is an integer 0, 1 or 2;
- R: is hydrogen, fluoro or C₁-C₆ alkyl;
- R₁: is hydrogen, C₁-C₆ alkyl or C₂-C₆ alkenyl;
- R₂: is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy;
- R₃: is CN, SO₃CF₃, N₃, NR₅R₆, COR₇, 5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy;
- R₄: is hydrogen or halogen;
- R₅: is hydrogen, C₁-C₆ alkyl or C₂-C₆ alkenyl;
- R₆: is C₁-C₆ alkyl or C₂-C₆ alkenyl; or
- R₅: and R₆ may together form a 5- or 6- membered ring which may contain 1 or 2 heteroatoms selected from N, O or S;
- R₇: is hydrogen, hydroxy, chloro, bromo, C₁-C₆ alkyl, C₂-C₆ alkenyl, C₁-C₄ alkoxy; NR₈ R₉ or 5-or 6- membered aryl which may contain 1 or 2 heteroatoms selected from N, O or S optionally substituted by one or more of halogen, CN, CF₃, C₁-C₆ alkyl, C₂-C₆ alkenyl or C₁-C₄ alkoxy;
- R₈: and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy, or may together form a 5- or 6- membered ring containing 1 or 2 heteroatoms selected from N, O or S;
enantiomers or salts thereof.

A further aspect of the invention is a pharmaceutical preparation containing as active ingredient a compound according to formula I 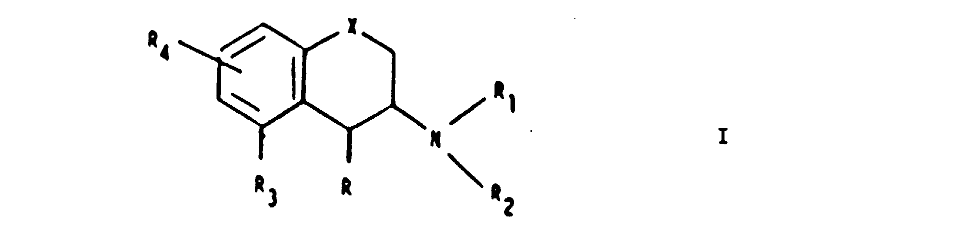 wherein
- X: is O or 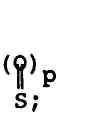
- p: is an integer 0, 1 or 2;
- R: is hydrogen, fluoro or C₁-C₆ alkyl;
- R₁: is hydrogen, C₁-C₆ alkyl or C₂-C₆ alkenyl;
- R₂: is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy; or
- R₃: is NR₅R₆, COR₇, 5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy;
- R₄: is hydrogen or halogen;
- R₅: is hydrogen, C₁-C₆ alkyl or C₂-C₆ alkenyl;
- R₆: is C₁-C₆ alkyl or C₂-C₆ alkylen; or
- R₅: and R₆ may together form a 5- or 6- membered ring which may contain 1 or 2 heteroatoms selected from N, O or S;
- R₇: is hydrogen, C₁-C₆ alkyl, C₂-C₆ alkenyl, C₁-C₄ alkoxy, NR₈ R₉ or 5- or 6- membered aryl which may contain 1 or 2 heteroatoms selected from N, O or S optionally substituted by one or more of halogen, CN, CF₃, C₁-C₆ alkyl, C₂-C₆ alkenyl or C₁-C₄ alkoxy;
- R₈: and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy or may together form a 5- or 6- membered ring containing 1 or 2 heteroatoms selected from N, O or S;
an enantiomer or a pharmaceutically acceptable salt thereof.

A preferred group of therapeutically active compounds of formula I are those wherein R₁ and R₂ are each independently hydrogen, n-propyl, i-propyl or cyclopropyl and R₃ is a carbonylgroup COR₇. Among these groups are the definition of R₇ as alkyl, aminoalkyl e.g. methyl, ethyl, n-propyl, i-propyl, cyclopropyl, n-butyl, i-butyl, t-butyl and cyclobutyl or aryl, aminoaryl e.g. phenyl, thienyl, fluorophenyl and furanyl. Another preferred group is when R₃ is aryl e.g. phenyl, thienyl, furanyl, or fluorophenyl. Another preferred group of active compounds are those wherein R₄ is halogen in 8 position as well as enantiomers thereof.

Compounds of formula I wherein R₃ is CN, COOH, COCl, COBr, N₃ or sO₃CF₃ are new intermediates for preparation of the therapeutically active compounds of formula I.

C₁-C₆ alkyl in formula I representing straight, branched and cyclic alkyl groups having 1 to 6 carbon atoms, for example methyl, ethyl, n-propyl, i-propyl, n-butyl, i-butyl, t-butyl, n-pentyl, i-pentyl, t-pentyl, neo-pentyl, n-hexyl, i-hexyl, cyclopropyl, cyclobytyl, cyclopentyl, cyclohexyl, methylcyclopropyl, ethylcyclopropyl, methylcyclobutyl. Preferred alkyl groups have 1 to 4 carbon atoms.

C₂-C₆ alkenyl in formula I representing straight or branched carbon atoms chains having 2 to 6 carbon atoms and containing one or two double bond, for example allyl, propenyl, isopropenyl, butenyl, isobutenyl, pentenyl, isopentenyl. Preferred alkenyl groups have 2 to 4 carbon atoms and one double bond.

C₁-C₄ alkoxy in formula I representing a straight alkoxy group having 1 to 4 carbon atoms, for example methoxy, ethoxy, propoxy or butoxy, preferably methoxy and ethoxy.

C₁-C₄ alkylaryl where aryl may contain 1 or 2 heteroatoms selected from N. O or S in the definition of R₂ in formula I representing an aryl residue having 3 to 12 carbon atoms in the aromatic ring and optionally 1 or 2 heteroatoms selected from N, O or S in the aromatic ring, bond by a straight or branched alkylen chain having 1 to 4 carbon atoms in the aliphatic chain. The aromatic ring may be substituted by one or more of nitrile, trifluoromethyl, halogen such as fluoro, chloro, bromo, iodo, C₁-C₆ alkyl, e.g. methyl, ethyl, propyl, C₂-C₆ alkenyl e.g. allyl, propenyl, or C₁-C₄ alkoxy preferably in meta and/or para position. Examples of suitable aryl groups in C₁-C₄ alkylaryl are phenyl, naphtyl, biphenyl, thienyl, furyl, pyryl, pyrimidyl and pyrridinyl. Preferred C₁-C₄ alkylaryl groups are unsubstituted and substituted phenylalkyl groups wherein the alkyl group is a straight or branched alkyl having 1 to 4 carbon atoms and the aromatic ring may be substituted by one or more of fluoro, chloro, bromo, iodo, nitrile, trifluoromethyl, methyl or ethyl in meta and/or para position. For example benzyl, phenethyl and phenylpropyl, especially preferred is phenylpropyl.

Halogen in formula I representing fluoro, chloro, bromo, iodo, preferably fluoro, chloro and bromo.

5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy; in the definition of R₃ in formula I representing either (i) substituted or unsubstituted phenyl, thienyl, furyl, pyridyl, pyrimidyl, pyrazinyl, pyradazinyl, thiozolyl, isothiozolyl, oxazolyl, isoxazolyl, imidazolyl, pyrazolyl, piperazinyl or morpholinyl or either (ii) substituted or unsubstituted quinolyl, isoquinolyl, quinazolyl, quinaxazolyl or indolyl.

5- or 6-membered aryl which may contain 1 or 2 heteroatoms selected from N, O or S in the definition of R₇, R₈ and R₉ in formula I representing phenyl, thienyl, furyl, pyridyl, pyrimidyl, pyrazinyl, pyridazinyl, thiazolyl, isothiazolyl, oxazolyl, isoxazolyl, imidazolyl, pyrazolyl, piperazinyl, morpholinyl.

Examples of suitable 5- or 6-membered ring structures formed by R₁ and R₂ or R₅ and R₆ or R₇ and R₈ respectively and the nitrogen atom and which may contain a further heteroatom selected from N, O or S are piperazine, morpholine, pyrrolidine, pyrrole, pyrroline, imidazole, imidazoline, imidazolidine, pyrazole, pyridine, pyrazine, pyrimidine, pyridazine.

The compounds of the invention have one or two assymetric carbon atoms. When R is hydrogen the compounds have an assymetric carbon atom adjacent to the nitrogen atom i.e. C₃ and when R is C₁-C₆ alkyl the compounds have an assymetric carbon atom adjacent to the nitrogen atom and an assymetric carbon atom adjacent to the alkyl group I.e, C₄. Thus, the compounds exist as two or four optical isomers i.e. enantiomers. Both the pure enantiomers, racemic mixtures are within the scope of the present invention. The therapeutic properties of the compounds may to a greater or lesser degree be ascribed to the racemate or to the enantiomers occurring.

Both organic and inorganic acids can be employed to form non-toxic pharmaceutically acceptable acid addition salts of the compounds of this invention. Illustrative acids are sulfuric, nitric, phosphoric, oxalic, hydrochloric, formic, hydrobromic, citric, acetic, lactic, tartaric, pamoic, ethanedisulfonic, sulfamic, succinic, methylsulphonic, propionic, glycollic, malic, gluconic, pyruvic, phenylacetic, 4-aminobenzoic, anthranilic, salicylic, 4-amino-salicylic, 4-hydroxybenzoic, nicotinic, methanesulfonic, ethanesulfonic, hydroxyethanesulfonic, benzenesulfonic, p-toluenesulfonic, sulfanilic, naphtalenesulfonic, ascorbinic, cyclohexylsulfamic, fumaric, maleic and benzoic acids. These salts are readily prepared by methods known in the art.

### Methods of Preparation

The compounds of the formula I may be prepared by the following processes constituting a further aspect of the invention.

### a. Converting a compound of formula II

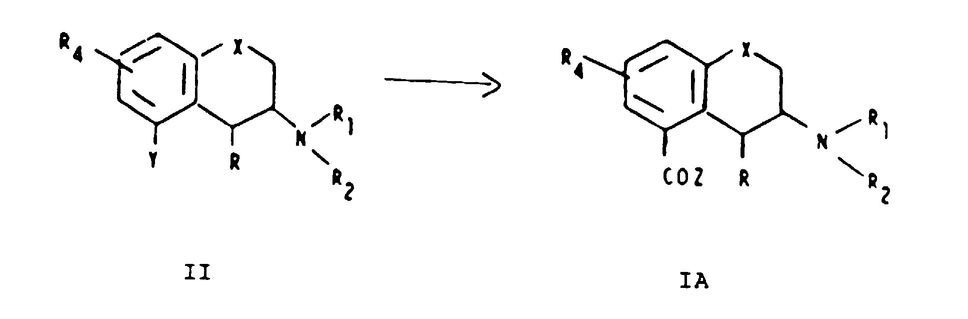

wherein Y is a leaving group such as trifluoromethane sulfonate (OSO₂CF₃), halide e.g. Cl or Br, and X, R, R₁, R₂ and R₄ are defined as above by substitution of the group Y to a carboxy group COZ, wherein Z is Cl, Br, OH, ORₚ where Rₚ is C₁-C₆ alkyl to formation of a compound of formula I wherein R₃ is COZ, (IA).

The compound of formula II can be converted to the compound of formula IA by the following catalytic cycle. Metal M^{o} should be a zerovalent transition metal, such as Pd or Ni with ability to undergo oxidative addition to aryl-Y-bonds e.g. the aryl-SO₃CF₃ bonds. M^{o} may be generated in situ from MII. The aryl-CO-M^{II}-Y are formed by treatment with carbon monoxide (CO). 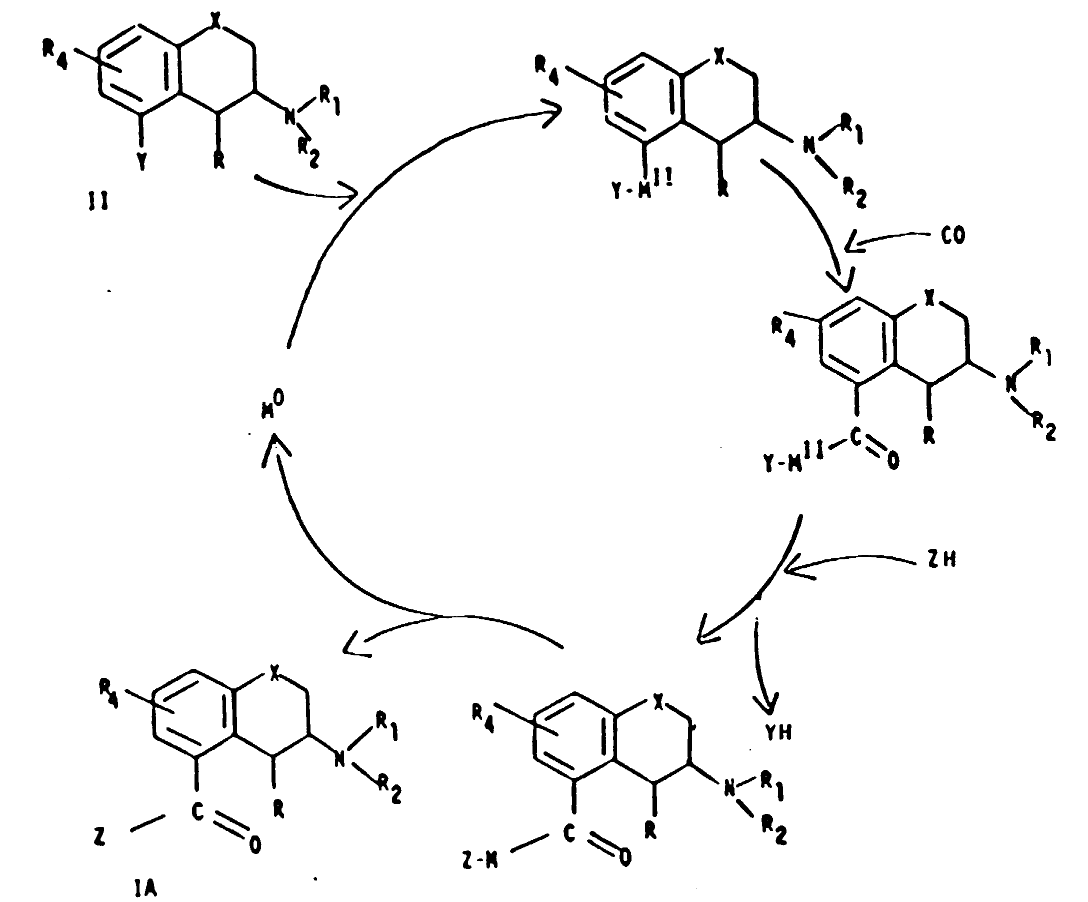 Further reagants are an alcohol such as alkanol e.g. methanol, ethanol, an amine base such as a trialkylamine e.g. triethylamine in an inert organic solvent preferentially a polar aprotic solvent such as dimethylformamide (DMF), dimethylsulfoxide (DMSO), aceton, acetonitrile etc. The reaction is normally performed at a temperature between +40 to + 120°C and at a pressure between 100 to 500 KPa. Optionally followed by hydrolyze and treatment with a thionyl halide e.g. thionylchloride to obtain the corresponding acid halide derivative.

### b. Compound of formula I wherein R₃ is COZ (IA) can also be formed by the reversed process:

A reaction as the catalytic cycle using a zerovalent transition metal M⁰, such as Pd, or Ni with ability to undergo an oxidation addition to Z-Y, wherein Z is defined Cl, Br, OH or ORₚ where Rₚ is C₁-C₆ alkyl and Y is a leaving group such as SO₃CF₃ and halide, treatment with carbon monoxide followed by addition of a compound of formula III, wherein X, R, R₁, R₂ and R₄ are as defined under formula I. 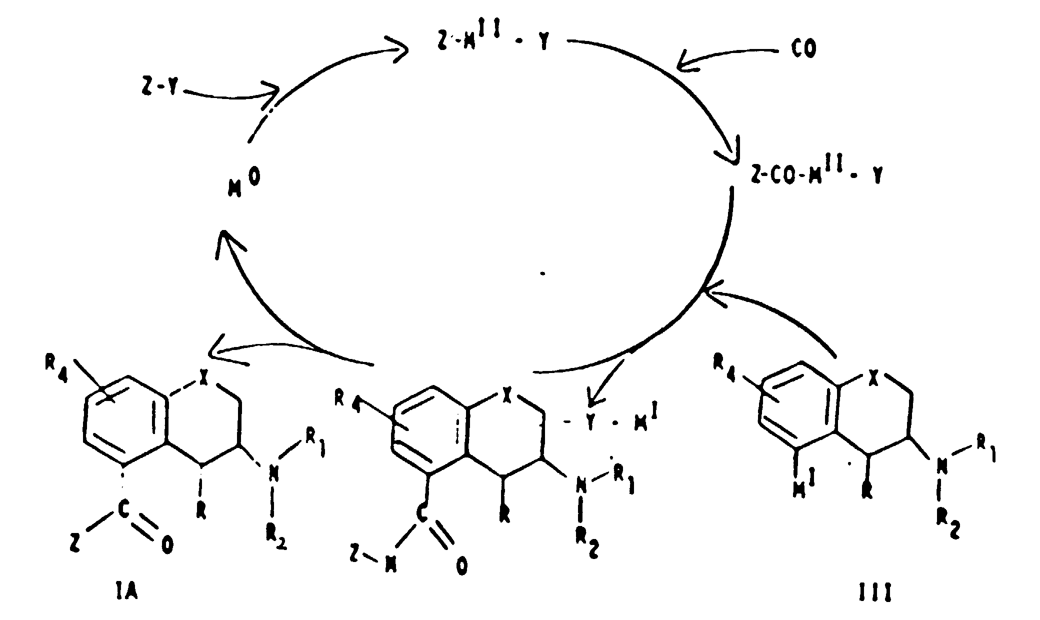 The Z-CO-M^{II}-Y can also be formed from Z-COCl directly. The reaction conditions and reagant are the same as described in method a. above. Hydrolyze of suitable carboxylic acid ester forms the free acid, which can be converted to its acid halide derivative.

### c. Converting a compound of formula II

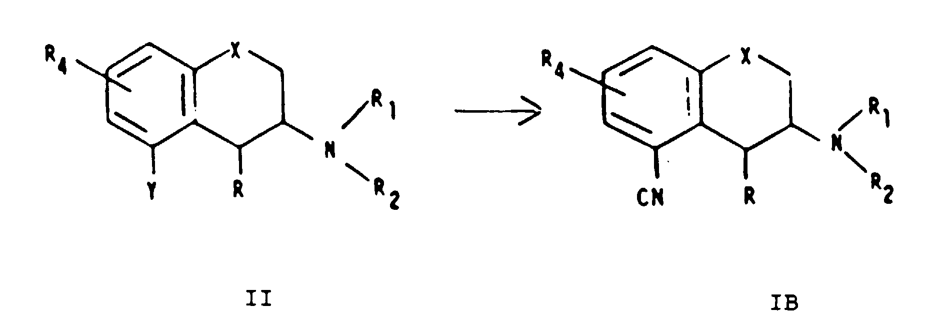

wherein X, R, R₁, R₂ and R₄ are as defined above and Y is a leaving group such as Cl, Br or SO₃CF₃ by treatment with a cyanide reagant such as cupper cyanide (CuCN) to obtain a compound of formula I wherein R₃ is CN. The reaction with cyanide reagant is performed in an inert organic solvent such as dimethylformamide, hexamethylenphosphotriamide etc. at a temperature between 20° to 200°C preferrably between 50° to 150°C and at normal temperature.

### d. Amination of a compound of formula IA

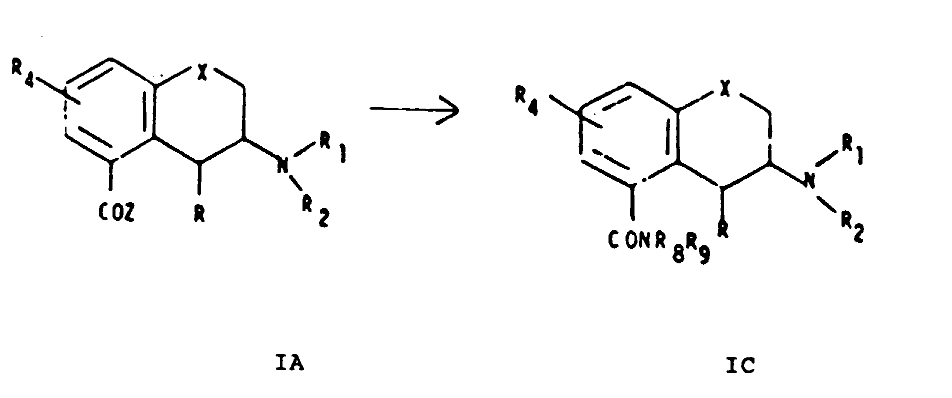

wherein X, R, R₁, R₂ and R₄ are as defined above and Z is Cl, Br, OH or ORₚ where Rₚ is C₁-C₆ alkyl.

If the compound of formula IA is a carboxylic acid ester it must first be hydrolyzed to form the free acid. The free acid is then transformed into the amide IC via its acid chloride derivative by reaction of the corresponding amine NR₈R₉, where R₈ and R₉ are as defined under formula I, in a nonpolar aprotic solvent e.g. toluene, benzen at reflux temperature between 0 to 100°C.

### e. Substitution of a 5-bromo-chroman/thiochroman

derivative by treatment with an appropriate stannic trialkyl reagant in presence of a zerovalent metal preferrably palladium (Pd°) to obtain a compound of formula I wherein R₃ is aryl, in presence of carbonmonoxide (CO) is formed a compound of formula I wherein R₃ is COR₇ wherein R₇ is C₁-C₆ alkyl, C₂-C₆ alkylen or aryl.

The substitution may be performed by one of the following ways:

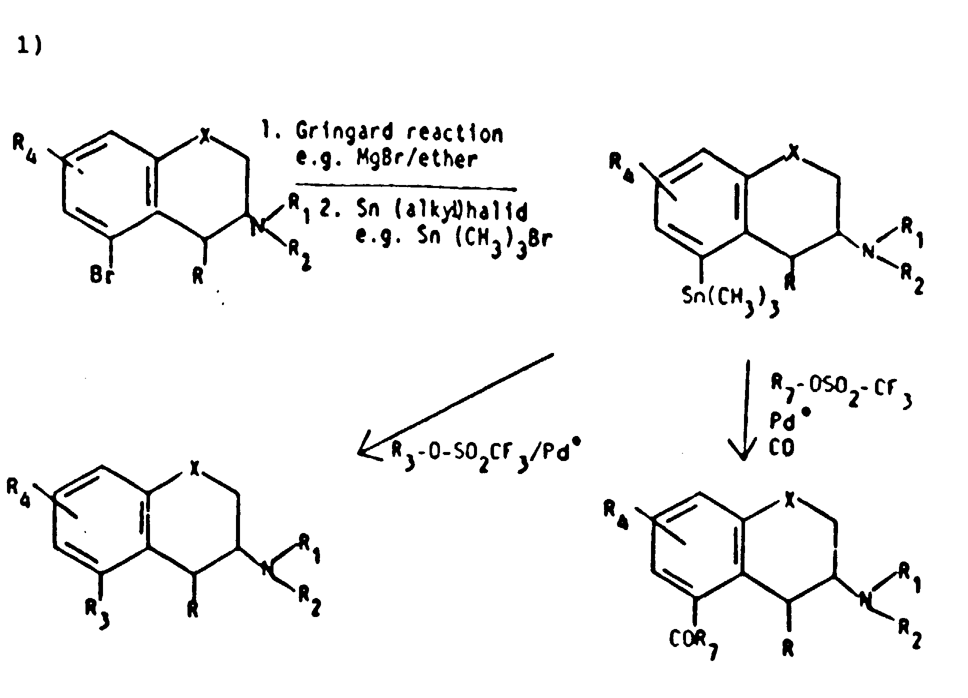

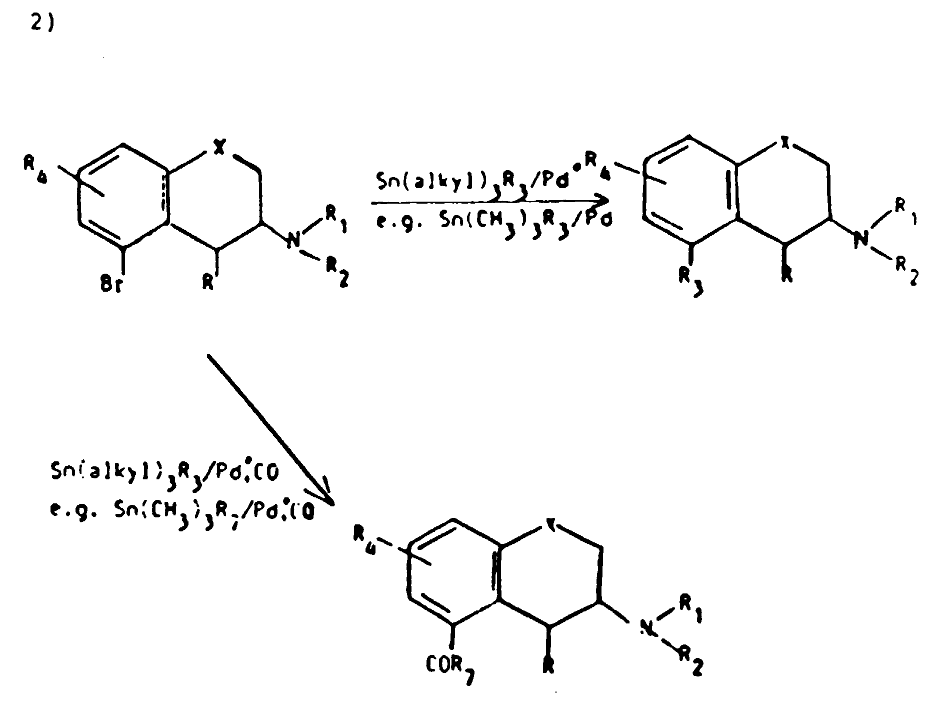

### f. Converting the 5-carboxy chroman/thiochroman derivative of formula I

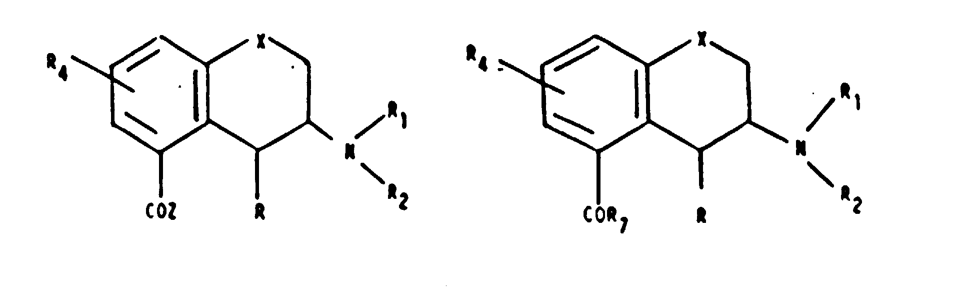

where X, R, R₁, R₂ and R₄ are defined as above and Z is Cl, Br by using R₇Li wherein R₇ is alkyl, alkenyl or aryl as a cuprate reagant to obtain corresponding 5-ketochroman/thiochroman derivative. Suitable R₇Li used is alkyllithium e.g. CH₃Li, alkenyllithium e.g. CH₂CHLi or aryllithium e.g. phenyl-Li. The reaction is performed in an inert organic solvent preferrably a nonpolar aprotic solvent such as ethers e.g. diethyl ether, tetrahydrofuran at a temperature between -50° - +50°C.

### g. Hydrolysis of a compound of formula I, wherein R₃ is CN (IB)

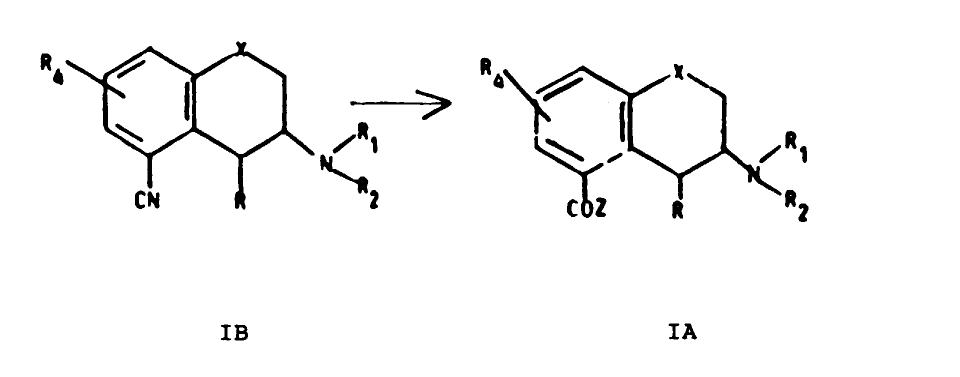

wherein X, R, R₁, R₂ and R₄ are as defined above optionally followed by treatment with a thionyl halide e.g. thionylchloride, thionylbromide to obtain a compound of formula I wherein R₃ is COZ where Z is OH, Cl or Br.

### h. Substitution of a compound of formula I, wherein R₃ is CN (IB)

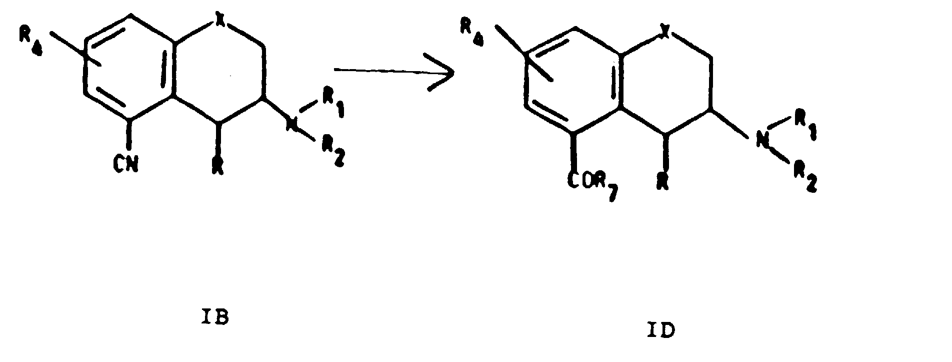

wherein X, R, R₁, R₂ and R₄ are defined as above by treatment with an appropriate organometallic reagant preferentially an organolithium such as R₇Li or a Gringard reagent such as R₇Mg halide in an inert organic solvent preferentially a nonpolar aprotic solvent such as bensen, ethers e.g. diethylether, tetrahydrofuran followed by hydrolysis of the intermediate complex to obtain a compound of formula I wherein R₃ is COR₇ where R₇ is C₁-C₆ alkyl, C₂-C₆ alkenyl or aryl.

### i. Converting a compound of the formula (II)

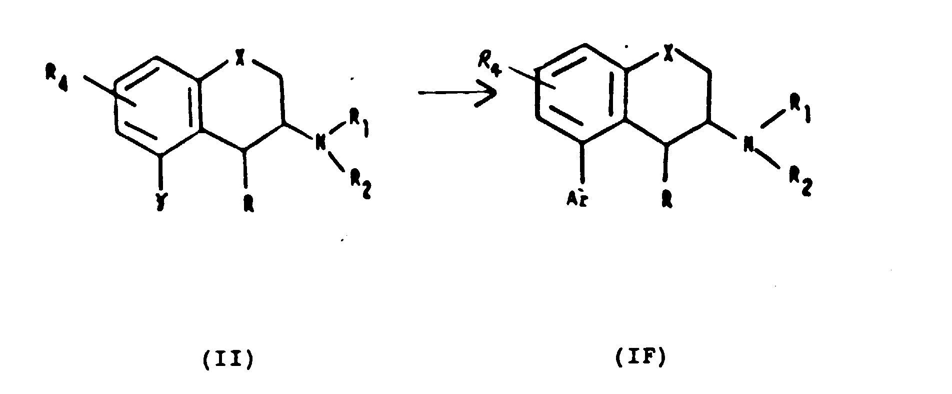

wherein Y is a leaving group such as trifluoromethanesulphonate (Tf), phosphonate, halide such as Br or I and R, R₁ and R₂ are defined as above by substitution of the group Y to 5- or 6-membered aryl (Ar) which may contain 1 or 2 heteroatoms selected from N, O, or S being either substituted or fused at two adjacent carbon atoms to an aryl ring as defined above to formation of a compound of formula IF.

The compound (II) may be converted to (IF) by reaction with a transition metal, such as Pd or Ni with ability to form ligand complex and undergo oxidative addition. A suitable aryl-substituent can be introduced via a suitable trialkylarylstannane or aryl-boric acid reagents.

Further reagents are an amine such as triethylamine and lithiumsalt e.g. lithium chloride. The reaction is preferentially carried out in a polar aprotic solvent such as dimethylformamide, dioxane, acetonitril or dimethylsulfoxide at a temperature between +40 to +120°C.

The following method describes one way of obtaining the intermediate of formula IB 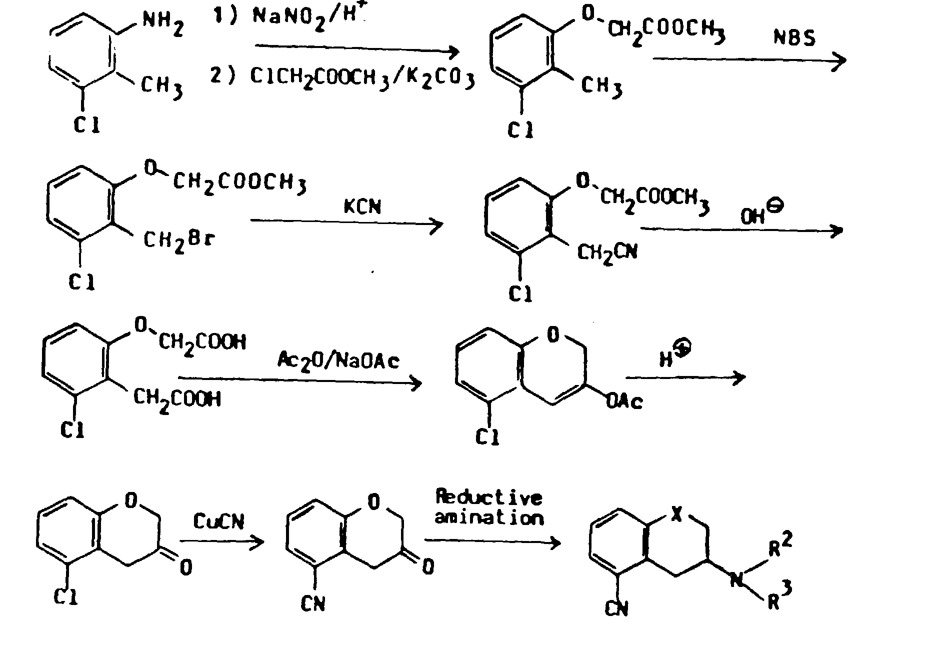 wherein R₁, R² and R⁴ are defined as in formula I

### Pharmaceutical preparations

According to the present invention the compounds of the formula I will normally be administered orally, rectally or by injection, in the form of pharmaceutical preparations comprising the active ingredient either as a free base or a pharmaceutically acceptable non-toxic acid addition salt, e.g. the hydrochloride, hydrobromide, lactate, acetate, phosphate, sulphate, sulphamate, citrate, tartrate, oxalate and the like in a pharmaceutically acceptable dosage form. The dosage form may be a solid, semisolid or liquid preparation. Usually the active substance will constitute between 0.1 and 99% by weight of the preparation, more specifically between 0.5 and 20% by weight for preparations intended for injection and between 0.2 and 50% by weight for preparations suitable for oral administration.

To produce pharmaceutical preparations containing a compound of the formula I in the form of dosage units for oral application, the selected compound may be mixed with a solid excipient, e.g. lactose, saccharose, sorbitol, mannitol, starches such as potato starch, corn starch or amylopectin, cellulose derivatives, a binder such as gelatine or polyvinylpyrrolidone, and a lubricant such as magnesium stearate, calcium stearate, polyethylene glycol, waxes, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet can be coated with a polymer known to the man skilled in the art, dissolved in a readily volatile organic solvent or mixture of organic solvents. Dyestuffs may be added to these coatings in order to readily distinguish between tablets containing different active substances or different amounts of the active compounds.

For the preparation of soft gelatine capsules, the active substance may be admixed with e.g. a vegetable oil or polyethylene glycol. Hard gelatine capsules may contain granules of the active substance using either the abovementioned excipients for tablets e.g. lactose, saccharose, sorbitol, mannitol, starches (e.g. potato starch, corn starch or amylopectin), cellulose derivatives or gelatine. Also liquids or semisolids of the drug can be filled into hard gelatine capsules.

Dosage units for rectal application can be solutions or suspensions or can be prepared in the form of suppositories comprising the active substance in admixture with a neutral fatty base, or gelatine rectal capsules comprising the active substance in admixture with vegetable oil or paraffin oil.

Liquid preparations for oral application may be in the form of syrups or suspensions, for example solutions containing from about 0.2% to about 20% by weight of the active substance herein described, the balance being sugar and mixture of ethanol, water, glycerol and propylene glycol. Optionally such liquid preparations may contain colouring agents, flavouring agents, saccharine and carboxymethyl-cellulose as a thickening agent or other excipients known to the man in the art.

Solutions for parenteral applications by injection can be prepared in an aqueous solution of a water-soluble pharmaceutically acceptable salt of the active substance, preferably in a concentration of from about 0.5% to about 10% by weight. These solutions may also contain stabilizing agents and/or buffering agents and may conveniently be provided in various dosage unit ampoules.

Suitable daily doses of the compounds of the invention in therapeutical treatment of humans are about 0.01-100 mg/kg bodyweight at peroral administration and 0.001-100 mg/kg bodyweight at parenteral administration.

### Working examples

The following examples will further illustrate the invention.

### Example 1

### 3-Dipropylamino-5-trifluoromethanesulfonylchroman

3-Dipropylamino-5-hydroxychroman (Thorberg et al. Acta Pharm. Suec. 24(1987)) (1.4 g, 4.0 mmol) and N,N-dimethylamino-pyridine (0.1 g, 0.75 mmol) were dissolved in 50 mL methylene dichloride (CH₂Cl₂) and cooled to -30°C. 2,4,6-Collidine (0.75 mL, 5.7 mmol) was added followed by trifluoromethane sulfonic anhydride (1.0 mL, 6.0 mmol). The solution was stirred at -20°C for 3 hours and then allowed to reach ambient temperature. The solution was washed with aqueous NaHCO₃, dried with Na₂SO₄ and evaporated to dryness. The pale yellow oil was finally purified by flash chromatography (silica gel) by elution with ethyl acetate/hexane 1:9. Yield: 55%, Mp125-127°C (oxalate).

### Example 2

### 3-Dipropylamino-5-methyloxycarbonylchroman

3-Dipropylamino-5-trifluoromethanesulfonylchroman (Example 1; 4.43 g, 11.6 mmol) was dissolved in 80 mL dimethylformamide/methanol 6:2 and the solution was degassed (10 mm Hg, 20°C, 15 min). PdOAc₂ (76 mg, 0.34 mmol), 1,3-bis-diphenyl-phosphinopropane (141 mg, 0.34 mmol) and triethylamine (3.5 mL, 25 mmol) were then added. The mixture was heated to 70°C under CO atmosphere and stirred for 5 hours. The solution was cooled, diluted with toluene (200 mL), washed with aqueous NaHCO₃, dried with Na₂SO₄ and evaporated to dryness. The oil was purified by flash chromatography (silica gel) by elution with ethyl acetate/hexane 1:8.

### Yield: 76%, Mp 150-152°C (HCl-salt).

### Example 3

### 3-Dipropylamino-5-carbamoylchroman

3-Dipropylamino-5-methyloxychroman (Example 2; 400 mg, 1.37 mmol) was dissolved in 10 ml methanol and NaOH (60 mg, 1.5 mmol) in 2 mL H₂O was added. The mixture was refluxed for 5 hours, cooled, filtered through Celite " and evaporated to dryness. The residue was refluxed in SOCl₂ (5 mL, 68 mmol) for 30 minutes. The excess SOCl₂ was then removed in vacuo to give 3-dipropylamino-5-chloroformylchroman*HCL as a gum. The pale brown gum was dissolved in CH₂Cl₂ (50 mL), and a stream of NH₃ (g) was introduced during 2 minutes. The solution was washed with aqueous NaHCO₃, dried with Na₂SO₄ and evaporated to dryness. The oil was purified by flash chromatography (silica gel) by elution with ethyl acetate/hexane 1:4. Yield 80%, ¹³C-NMR: 172.0 154.9 136.5 126.9 120.4 119.1 118.6 67.8 53.0 52.6 26.1 22.4 21.9 14.1 11.7.

### Example 4

### 3-dipropylamino-5-N,N-dimethylcarbamoylchroman

The title compound was prepared analogous to the procedure used in Example 3 starting from 3-dipropylamino-5-methyloxycarbonylchroman and substituting dimethylamine (g) in place of NH₃ (g). ¹³C-NMR: 189.3 170.3 149.9 137.4 126.7 126.1 124.9 65.8 64.7 48.2 47.7 30.7 26.0 15.1 10.9.

### Example 5

### 3-Dipropylamino-5-N,N-diisopropylcarbamoylchroman

The title compound was prepared analogous to the procedure used in Example 3 starting from 3-dipropylamino-5-methyloxychroman. Mp 228-230°C (HCl-salt).

### Example 6

### 3-Dipropylamino-5-N-methylcarbamoylchroman

The title compound was prepared analogous to the procedure used in Example 3 starting from 3-dipropylamino-5-methyloxycarbonylchroman and substituting methylamine (g) in place of NH₃ (g). Mp 95-97°C (oxalate).

### Example 7

### 3-Dipropylamino-5-acetylchroman

3-Dipropylamino-5-chloroformylchroman*HCl (4.42 g, 13.4 mmol), prepared from 3-dipropylamino-5-methyloxycarbonylchroman (Example 2) analogous to the procedure used in Example 3, in dry tetrahydrofuran (20 ml), was added to a pre-formed solution of lithium dimethylcuprate; prepared from MeLi and CuI, in 200 mL tetrahydrofuran at -78°C. The solution was stirred for 15 minutes at -78°C and was then allowed to reach room temperature during 10 minutes. Then, 30 mL H₂O was slowly added. The organic phase was decanted, dried with Na₂SO₄ and evaporated to dryness. The residue was purified by flash chromatography (silica gel) by elution with ethyl acetate/hexane 1:8. The title compound was crystallized as salt from ethyl acetate. Mp 106-108°C (oxalate).

### Example 8

### 3-Dipropylamino-5-cyclopropylcarbonylchroman

The title compound was prepared analogous to the procedure used in Example 7 substituting lithium dicyclopropylcuprate (J. Org. Chem., 41 (22), 1976) in place of lithium dimethylcuprate. Mp 100-102°C (oxalate).

### Example 9

### 3-Dipropylamino-5-tertbutylcarbonylchroman

The title compound was prepared analogous to the procedure used in Example 7 substituting lithium di-tertbutylcuprate (from tertbutyllithium and CuBr*Me₂S) in place of lithium dimethylcuprate. Mp 118-120°C (oxalate).

### Example 10

### 3-Dipropylamino-5-isopropylcarbonylchroman

The title compond was prepared analogous to the procedure used in Example 7 substituting magnesium diisopropylcuprate (from isopropylmagnesium chloride and CuBr*Me₂S) in place of lithium dimethylcuprate. Mp 60-62°C (oxalate).

### Example 11

### 3-Dipropylamino-5-(4-fluorophenylcarbonyl)chroman

The title compound was prepared analogous to the procedure in Example 7, substituting magnesiumdi(4-fluorophenyl)cuprate (from 4-fluorophenyl magnesium bromide and CuI) in place of lithium dimethylcuprate. Mp 98.3-98.4°C (oxalate).

### Example 12

### 3-Dipropylamino-5-(2-thienylcarbonyl)chroman

The title compound was prepared analogous to the procedure used in Example 7 substituting lithium di(2-thienyl)cuprate (from 2-thienyllithium and CuI) in place of lithium dimethylcuprate. Mp 87-88.5 (oxalate).

### Example 13

### 3-Dipropylamino-5-isopropenylchroman

Methyltriphenylphosphoniumbromide (0.62 g, 1.74 mmol) was dissolved in dry ethyl ether (20 ml) under nitrogen at ambient temperature and n-BuLi (0.7 ml, 2.5 M, 1.74 mmol) was added and the solution was stirred for 4 hours. 3-Dipropylamino-5-acetylchroman (Example 7; 0.40 g, 1.45 mmol) was dissolved in dry diethyl ether (2.0 ml) and this solution was added to the previously formed Wittig-reagent. The mixture was stirred at ambient temperature overnight. The solution was diluted with toluene and washed with water. Drying of the organic phase with Na₂SO₄ and evaporation to dryness gave a solid, which was finally purified by flash chromatography by elution with ethyl acetate/hexane 1:4. The collected fractions were evaporated and gave the title compound as a colourless oil. ¹³C-NMR: 11.82 21.94 24.28 26.69 52.79 53.64 67.70 115.03 115.13 118.73 120.07 126.83 144.88 145.27 154.03.

### Example 14

### 3-Dipropylamino-5-aminochroman

3-Dipropylamino-5-methyloxycarbonylchroman (Example 2; 1.0 g, 3.4 mmol) was dissolved in methanol (20 ml). Sodium hydroxide (0.16 g, 4.1 mmol) in water (1.0 ml) was added and the solution was refluxed with nitrogen overnight. The solution was evaporated to dryness, toluene (20 ml) was added and again the solution was evaporated to dryness. The residue was dissolved in toluene 20 ml, diphenylphosphoryl azide (1.87 g, 6.8 mmol) was added and the solution was refluxed for 2 hours. Methanol (2.0 ml) was added and reflux was continued for 4 hours. The solution was cooled, washed with water and extracted with dilute HCl (aq.). The acidic water phase was neutralized NaOH (aq.) and extracted with toluene. The toluene-phase was dried with sodium sulphate and evaporated to dryness. The residue was dissolved in ethanol containing 10% NaOH (20 ml) and the solution was refluxed overnight. The solution was cooled and diluted with toluene. Washing with water, drying of the organic phase and evaporation to dryness afforded the title compound as an oil, which was converted to a dihydrochloride salt. Mp 173-174°C.

### Example 15

### 3-Dipropylamino-5-nitrochroman

3-Dipropylamino-5-aminochroman (Example 14; 0.050 g, 0.20 mmol) was dissolved in a mixture of trifluoracetic acid (0.080 ml, 1.0 mmol) in water (5 ml). The clear solution was cooled to 0-4°C. Sodium nitrite (0.017 g, 2.5 mmol) in water (1.0 ml), was added dropwise with good stirring. The solution was stirred for 15 minutes and neutralized with calcium carbonate. A solution of sodium nitrite (0.50 g, 7.2 mmol) in water (1.0 ml) was added followed by a mixture of copper sulfate (0.10 g, 0.62 mmol) and copper (1) oxide in water (1.0 ml). The solution was stirred at 0°C for 20 minutes and then at ambient temperature for 2 hours. The solution was extracted with diethyl ether. The organic phase was dried with sodium sulfate and evaporated to dryness. The residue was purified by flash chromatography on silica gel by elution with ethyl acetate/hexane 1:9 to give the title compound. Mp 150-151°C (hydrochloride).

### Example 16

### 3-Dipropylamino-5-azidochroman

3-Dipropylamino-5-aminochroman (Example 14; 0.050 g, 0.20 mmol) was diazotized according to the procedure of Example 15. After stirring for 15 minutes, sodium azide (0.026 g, 0.4 mmol) in water (1.0 ml) was added. After stirring at 5°C overninght the solution was worked-up and purified according to the procedure of Example 15 to give the title compound. Mp 167-168°C (oxalate).

### Example 17

### 3-Dipropylamino-5-(pyrrol-1-yl)chroman

3-Dipropylamino-5-aminochroman (Example 14; 0.60 g, 2.42 mmol) was dissolved in acetic acid (10 ml) and 2,5-dimethoxytetrahydrofuran (0.40 g, 3.0 mmol) was added. The solution was refluxed for 1 hour. The solution was neutralized with NaOH (aq.) and extracted with toluene. The organic phase was dried with sodium sulfate and evaporated to dryness. The residue was purified by flash chromatography on silica gel by elution with ethyl acetate/hexane 1:9 to give the title compound. ¹³C-NMR: 111.75 21.89 24.81 52.69 53.15 67.94 108.93 115.67 118.22 118.44 121.87 127.22 141.47 155.27

### Example 18

### 3-(Methyl(3-phenylpropyl)amino)-5-hydroxychroman

3-Amino-5-methoxychroman (Thorberg et al. Acta Pharm. Suec. 24(1987)) (2.0 g, 9.28 mmol) was dissolved in methanol (50 ml) and pH was adjusted to 6.0 with acetic acid. The solution was cooled to 0°C and sodium cyanoborohydrid (0.87 g, 13.8 mmol) was added together with 3-phenylpropanal (1.22 ml, 9.28 mmol), the cooling was withdrawn and the solution was stirred at ambient temperature for 4 hours. Paraformaldehyde (0.42 g, 14 mmol) and sodium cyanoborohydride (0.87 g, 9.28 mmol) was added and stirring was continued overnight at ambient temperature. The solution was diluted with toluene and washed with water. Drying with sodium sulfate and evaporation to dryness gives an oil. The oil was purified by flash chromatography on silica gel by elution with ethylacete/hexane 1:4. The collected fractions were evaporated to give an oil. The oil was treated with HBr (47% aq.) at 120°C for 1 hour. The solution was cooled and neutralised with sodium hydroxide and extracted with toluene. The organic phase was dried and evaporated to give the title compound as an oil. ¹³C-NMR: 22.502 29.09 33.47 38.19 53.66 67.75 102.04 109.20 110.46 125.78 127.05 128.36 142.20 155.29 158.28.

### Example 19

### 3-(methyl(3-phenylpropyl))amino-5-methyloxycarbonylchroman

3-(methyl(3-phenylpropyl))amino-5-hydroxychroman (Example 18; 1.0 g, 3.37 mmol) was dissolved in CH₂Cl₂ (20 ml) at - 20°C. Pyridine (0.32 ml, 4 mmol), trifluoromethanesulfonic anhydride (0.65 ml, 5.9 mmol) and dimethylaminopyridine (DMAP), (0.041 g, 0.59 mmol) was added at -20°C under nitrogen. The solution was stirred for 3 hours at -20°C. Cooling was withdrawn and the solution was diluted with toluene, washed with sodium hydrogen carbonate (aq.), dried with sodium sulfate, filtered through silica gel and evaporated to dryness. The remaining oil was dissolved in 13 ml degassed methanol/DMF 3:10. Palladium acetate (0.056 g, 0.25 mmol), 1,3-bis(diphenylphosphino)propane (0.103 g, 0.25 mmol) and triethyl amine (0.76 ml, 5 mmol) was added and the solution was flushed with CO(g) under vigorous stirring. The pressure in the reaction vessel was raised to 20.2 KPa(e) with the aid of a CO(g)-cylinder fitted with a regulator. Stirring was continued overnight at 75°C. The pressure and temperature was normalized and the solution was diluted with toluene and washed with water. The organic phase was dried and evaporated to dryness. The remaining oil was purified by flash chromatography on silica gel by elution with ethyl acetate/hexane 1:4. The collected fractions were evaporated to give the title compound as a colourless oil. ¹³C-NMR: 26.88 29.00 33.20 37.85 51.64 53.37 55.44 67.24 120.60 123.06 123.40 125.59 126.47 128.17 128.24 130.36 142.01 154.93 167.29.

### Example 20

### 3-(methyl(3-phenylpropyl))amino-5-N-methylcarbamoyl chroman

3-(methyl(3-phenylpropyl))amino-5-methyloxycarbonylchroman (Example 19; 0.32 g, 0.94 mmol) was dissolved in methanol (10 ml). NaOH (0.08 g, 2 mmol) in 1 ml water was added and the solution was refluxed overnight under nitrogen. The solution was evaporated to dryness and co-evaporated with toluene (10 ml) to dryness again. The remaining solid was refluxed in SOCl₂ for 30 minutes and evaporated to dryness. The pale brown gum was dissolved in tetrahydrofuran (THF) 20 ml and treated with methyl amine (g) for 1 minute under vigorous stirring. The solution was diluted with toluene and washed with sodium hydrogencarbonate (aq.). Drying and evaporation gave a gum, which was finally purified by flash chromatography on silica gel by elution with ethyl acetate/hexane 1:2. The collected fractions were evaporated to give the title compound as a colourless gum. Crystallization from ethyl acetate as oxalate gave white needles. Mp 150-151°C. (oxalate).

### Example 21

### 3-Dipropylamino-5-trifluoromethanesulfonylthiochroman

3-Dipropylamino-5-hydroxybenzothiopyran (EP 0222 996; 420 mg, 1.58 mmol) and collidine (0.27 g, 0.29 mL) were dissolved in 15 mL of CH₂Cl₂ and cooled to -30°C. Trifluoromethanesulfonic anhydride (0.54 g, 0.32 mL) was added dropwise and allowed to reach ambient temperature, and after 20 minutes diluted with methylendichloride. The solution was washed with saturated NaHCO₃, dried with Na₂SO₄ and evaporated in vacuo.

Chromatography on silica by elution with CHCl₃ gave 0.62 g of the title compound as the base. Yield: 98%. Mp. 37-8°C; ¹³C NMR (200 MHz-CDCl₃) PPM 148.3, 136.7, 128.4, 127.2, 126.3, 122.0, 117.1, 115.2, 55.6, 52.5, 28.0, 26.6,-22.6,11.8.

### Example 22

### 3-Dipropylamino-5-methyloxycarbonylthiocroman

3-Dipropylamino-5-hydroxybenzothiopyran (EP 0222 996; 620 mg, 1.6 mmol) was dissolved in 11 mL of dimethylformamide/methanol (6:2) and the solution was degassed (10 mm, 22°C, 15 min). Pd(OAc)₂ (11mg), 1,3-bis-diphenyl-phosphinopropane (19 mg), and triethylamine (0.48 mL, 0.35 g) were added to the reaction mixture.

The mixture was heated to 70°C under carbonmonoxide atmosphere and stirred for 5 hours.

The solution was cooled, diluted with 30 mL of toluene, washed with saturated NaHCO₃, dried with Na₂SO₄, and evaporated in vacuo.

Chromatography on silica by elution using a gradient CHCl₃ -> 10% EtOAc/CHCl₃ gave 310 mg of the title compound (base) as a slightly yellow oil; Yield: 64%. ¹³C NMR (200 MHz-CDCl₃) PPM 168.2, 136.6, 134.8, 131.6, 130.1, 126.5, 125.7, 56.7, 52.5, 52.1, 30.4, 28.0, 22.3, 11.9.

### Example 23

### 3-Dipropylamino-5-acetylthiochroman

3-Dipropylamino-5-methyloxycarbonylthiochroman (Example 22; 310 mg, 1.01 mmol) was dissolved in 8 mL methanol and 60 mg of sodium hydroxide in 2 mL water was added. After 5 hours reflux the mixture was cooled and evaporated in vacuo. The residue was dissolved in thionylchloride (5 ml) and refluxed for 1 hour. The excess thionylchloride was evaporated in vacuo to obtain a gum.

The residual gum was dissolved in a minimal amount of tetrahydrofuran and added dropwise to a cooled (-78°C) solution of lithium dimethyl cuprate (2.02 mmol) in 20 mL of tetrahydrofuran.

The reaction mixture was stirred for 15 minutes at -78°C, then allowed to reach ambient temperature and after 10 minutes the reaction was quenched with 0.9 mL of water. The reaction was filtered through celite" and evaporated to dryness.

The residue were dissolved in ether, washed with saturated NaHCO₃, treated with brine, dried with Na₂SO₄, and evaporated in vacuo to afford the crude base as an oil.

The crude residue was chromatographed on silica by elution using a gradient of CHCl₃ -> 5% EtOAc/CHCl₃.

The hydrochloride salt was obtained by dissolving the pure base in ether and dropping an excess of an ethereal HCl solution. Recrystallization in trichloromethane/diethylether gave 92 mg of the title compound as a white solid; Yield: 27%. Mp 141-2°C; ¹³C NMR (200 MHz-CDCl₃) PPM 201.9, 138.4, 135.9, 131.7, 131.2, 127.2, 127.0, 59.9, 54.1, 51.8, 29.9, 27.9, 26.1, 18.6, 18.2, 11.6.

### Example 24

### 5-Allyl-3-(dipropylamino)thiochroman

To a solution of 3-(dipropylamino)-5-trifluoromethane-sulfonylthiochroman (Example 21; 1.28 g, 3.22 mmol), tetrakis(triphenylphosphine)palladium(0) (76 mg, 0.064 mmol) and a few crystals of 2,6-di-t-butyl-4-methylphenol in 10 mL anhydrous toluene was 1.17 g (1.1 mL, 3.53 mmol) tributylallyltin added neat. The resulting solution was refluxed for 4 hours then pyridine (1 mL) was added to the cooled solution followed by 2.1 ml of a hydrogen fluoride-pyridine complex (Stille J.K. et al. JOC 52(1987) 422).

After stirring for 1 hour at room temperature, the reaction mixture was diluted with 50 ml dietyl ether and treated, successively, with 50 mL 1 M NaOH solution, H₂O (x2), washed with a saturated NaCl solution and dried (NaSO₄). After filtering and removal of the solvent in vacuo, the crude was obtained as a dark oil.

Chromatography on silica by elution using a gradient hexane -> 5% EtOAc/hexane gave 0.85 g of the title compound (base) as a slightly yellow oil. Yield: 91%. ¹³C NMR: (200 MHz-CDCl₃) PPM 139.0, 136.5, 134.0, 133.0, 126.1, 125.9, 125.0, 116.0, 57.0, 52.6, 37.7, 29.5, 27.7, 22.5, 11.9. A portion of the base was taken out and made into the hydrochloride salt by dissolving the pure base in ether and dropping an excess of an ethereal HCl solution. Recrystallizing (AcCN-Et20-hexane) gave a white solid. Mp 164-5°C.

### Example 25

### 3-(Dipropylamine)-5-propylthiochroman hydrochloride

To a stirred suspension of potassium azodicarboxylate (0.76 g, 3.9 mmol) (made fresh from diethyl azocarboxylate and potassium hydroxide) and 5-allyl-3-(N,N-dipropylamino)-thiochroman (Example 24; 0.4 g, 1.4 mmol) in 10 mL anhydrous methanol was added a solution of glacial acetic acid/methanol (1:4) until the yellow color (from the potassium salt) disappeared.

After 30 min stirring at room temperature more potassium azodicarboxylate (200 mg) was added and again decomposed as before. This process was continued until analysis (GC) showed no starting material remaining.

Upon completion, (2 hours and 4 additions of the potassium salt) the solvent was removed in vacuo. To the remains, a 2 M NaOH solution was added which was extracted (x2) with diethyl ether and the combined organic portions were treated with a saturated NaCl solution, and dried (Na₂SO₄). The crude base was obtained as a light colored oil upon the removal of the solvent in vacuo.

Chromatography on silica by elution using a gradient hexane → 5% EtOAc/hexane gave the title compound (base) as a clear oil. The hydrochloride salt was made by dissolvning the pure base in ether and dropping and an excess of an ethereal HCl

solution. Recrystallizing (chloroform-Et₂O) gave 0.30 g of a white solid. Yield: 66%. Mp 150-151°C. ¹³C NMR: (on base, 200 MHz-CDCl₃) PPM 141.6 133.7 132.8, 125.8, 125.6, 124.5, 57.1, 52.6, 35.3, 29.5, 27.6, 23.6, 22.4, 14.3, 11.9.

### Pharmacology

### Pharmacological treatment of depression in man

Evidence is available that in depressed patients the neurotransmission in the central nervous system (CNS) may be disturbed. These disturbances appear to involve the neurotransmitters noradrenaline (NA) and 5-hydroxytryptamine (5-HT). The drugs most frequently used in the treatment of depression are considered to act by improving the neurotransmission of either or both of these physiological agonists. Available data suggest that the enhancement of 5-HT neurotransmission will primarily improve the depressed mood and anxiety, whereas the enhancement of noradrenaline neurotransmission will rather improve the retardation symptoms occurring in depressed patients. In recent years many efforts have been made to develop new drugs with high selectivity for the improvement of the 5-HT neurotransmission in the CNS.

The mechanism of action for the drugs generally used today in the therapy of mental depression is indirect, i.e. they act by blocking the reuptake of the neurotransmitters (NA and/or 5-HT) released from nerve terminals in the CNS, thus increasing the concentration of these transmitters in the synaptic cleft and hence restoring an adequate neurotransmission.

A fundamentally different way to improve the neurotransmission in the central 5-HT-neurons would be to use a direct 5-HT-receptor agonist. In order to minimize side effects, a high selectivity for this kind of receptors would then be preferable.

Antagonisms of the inhibitory autoreceptors located on the cellbodies of 5-HT-neurons would be another fundamentally different way to improve the 5-HT neurotransmission.

Surprisingly, we have found that a group of compounds of the formula I have selective, direct stimulating or inhibitory effect on a subgroup of central 5-HT receptors. Another observation is that some of those compounds have a particularly good oral bioavailability. In order to evaluate the 5-HT-receptor stimulating effect and selectivity, the affinity for various receptors in rat brain were measured in vitro using receptor assays (Ki nM).

### In vitro test: Receptor binding assay

5HT_{1A} binding assay. Cerebral cortex + hippocampus from each rat was dissected and homogenized in 15 ml ice-cold 50 mM Tris-HCl buffer 4.0 mM CaCl₂ and 5.7 mM ascorbic acid, pH 7.5 with an Ultra-Turrax (Janke & Kunkel, Staufen, FRG) for ten s. After centrifugation for 12.5 min at 17,000 rpm (39,800 x g in a Beckman centrifuge with a chilled JA-17 rotor (Beckman, Palo Alto, CA, USA), the pellets were resuspended in the same buffer and homogenization and centrifugation repeated. To each pellet 5 ml ice-cold 0.32 M sucrose were added and homogenized for 5 sec. These samples were kept frozen at -70°C. When used they were diluted with the buffer to 8 mg tissue/ml and homogenized for 10 sec. The tissue homogenates were incubated for ten minutes at 37°C and then supplied with 10 µM pargyline followed by reincubation for 10 minutes.

The binding assay followed that described by Peroutka, J. Neurochem. 47, 529-540, (1986). The incubation mixture (2 ml) contained ³H-8-OH-DPAT (0.25 to 8 nM), 5 mg/ml tissue homogenate in 50 mM Tris-HCl buffer containing 4.0 mM CaCl₂ and 5.7 mM ascorbic acid, pH 7.5. Six different concentrations of ³H-8-OH-DPAT were analyzed. Binding experiments were started by the addition of tissue homogenate and followed by incubation at 37°C for 10 minutes. The incubation mixtures were filtered through Whatman GF/B glass filters with Brandel Cell Harvester (Gaithersburg, MD, USA). The filters were washed twice with 5 ml ice-cold 50mM Tris-HCl buffer, pH 7.5, and counted with 5 ml Ready-solv HP (Beckman) in a Beckman LS 3801 scintillation counter. Nonspecific binding was measured by the addition of 10 µM 5-HT to the reaction mixture. The binding data was processed by non-linear least squares computer analysis (Munson and Rodbard, Anal. Biochem. 107, 220-239, (1980).

The test results are expressed as Kᵢ and are given in nM. For instance, 3-dipropylamino-5-acetylchroman has Kᵢ 1,0 (nM), 3-dipropylamino-5-carbamoylchroman has Kᵢ 3,1 (nM), 3-dipropylamino-5-N-methylcarbamoylchroman has Kᵢ 3,3 (nM) and 3-dipropylamino-5-(2-thienylcarbonylchroman has Kᵢ 1,7 (nM).

## Claims (Claims for the following Contracting State(s): AT, BE, CH, DE, DK, FR, GB, IT, LI, LU, NL, SE)

1. A compound of the formula 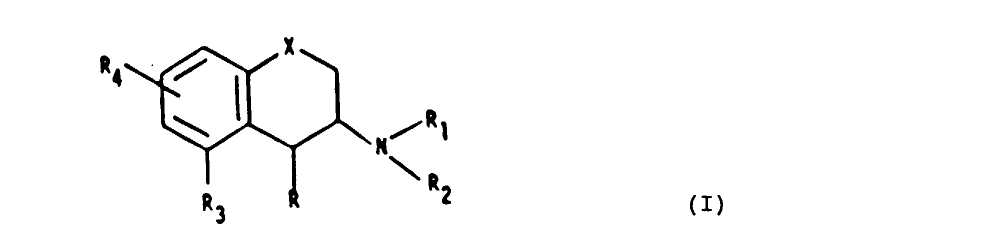 wherein
X is O or 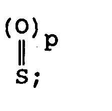
p is an integer 0, 1 or 2;
R is hydrogen, fluoro or C₁-C₆ alkyl;
R₁ is hydrogen, C₁-C₆ alkyl or C₂-C₆ alkenyl;
R₂ is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy; or
R₃ is CN, SO₃CF₃, N₃, NR₅R₆, COR₇, 5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy;
R₄ is hydrogen or halogen;
R₅ is hydrogen, C₁-C₆ alkyl or C₂-C₆ alkenyl;
R₆ is C₁-C₆ alkyl or C₂-C₆ alkenyl; or
R₅ and R₆ may together form a 5- or 6-membered ring which may contain 1 or 2 heteroatoms selected from N, O or S;
R₇ is hydrogen, hydroxy, chloro, bromo, C₁-C₆ alkyl, C₂-C₆ alkenyl, C₁-C₄ alkoxy, NR₈R₉ or 5- or 6-membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy;
R₈ and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy or may together form a 5- or 6-membered ring containing 1 or 2 heteroatoms selected from N, O or S;
an enantiomer or a salt thereof.

2. A compound according to claim 1 wherein X is O.

3. A compound according to claim 1 wherein X is 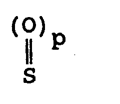 and p is 0, 1 or 2.

4. A compound according to any of claim 1, 2, or 3 wherein R₃ is NR₅R₆, COR₇, 5- or 6- membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substitutents independently selected from halogen, CN CF₃, C₁-C₆ alkyl, C₂-C₆ alkenyl or C₁-C₄ alkoxy; R₇ is hydrogen, C₁-C₆ alkyl, C₂-C₆ alkenyl, C₁-C₄ alkoxy, NR₈R₉ or 5- or 6- membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy; X, p, R, R₁, R₂, R₄, R₅, R₆, R₈ and R₉ are defined as in claim 1, an enantiomer or a pharmaceutically acceptable salt thereof for use in theraphy.

5. A compound according to any of claims 1-4, wherein R₁ and R₂ are the same or different and selected from hydrogen, n-propyl, i-propyl and cyclopropyl.

6. A compound according to any of claim 1-5, wherein R₃ is COR₇.

7. A compound according to claim 6 wherein R₇ is C₁-C₄ alkyl, phenyl, furanyl or thienyl optionally substituted with halogen, NR₈ R₉ wherein R₈ and R₉ are each independently hydrogen or C₁-C₄ alkyl, or C₁-C₄ alkoxy.

8. A compound according to claim 6 wherein R and R₄ are hydrogen, R₁ and R₂ are n-propyl and R₇ is methyl, ethyl, n-propyl, i-propyl, cyclopropyl, n-butyl, i-butyl, t-butyl, cyclobutyl, thienyl, furanyl, phenyl, N- methylamino, methoxy or fluorophenyl.

9. A compound according to any of claims 1-5 wherein R₃ is phenyl, furanyl, thienyl or fluorophenyl.

10. A compound according to any of claims 1-7 and 9 wherein R₄ is halogen in 8 position.

11. A compound according to claim 1 which is 3-dipropylamino-5-acetylchroman, an enantiomer or a salt thereof.

12. A compound according to claim 1 which is 3-dipropylamino-5-carbamoylchroman, an enantiomer or a salt thereof.

13. A compound according to claim 1 which is 3-dipropylamino-5-N-methylcarbamoylchroman, an enantiomer or a salt thereof.

14. A compound according to claim 1 which is 3-dipropylamino-5-(2-thienylcarbonyl)-chroman, an enantiomer or a salt thereof.

15. A compound according to claim 1 wherein R₃ is CN, COOH, COCl, COBr, N₃ or SO₃CF₃, and X, R, R₁, R₂ and R₄, are as defined in claim 1, an enantiomer or a salt thereof.

16. A compound according to claim 15 wherein R₁ and R₂ are the same or different and selected from hydrogen, n-propyl, i-propyl and cyclopropyl.

17. A compound according to claim 16 wherein R and R₄ are hydrogen, R₁ and R₂ are n-propyl and R₃ is SO₃CF₃.

18. A pharmaceutical preparation containing as active ingredient a compound according to formula I 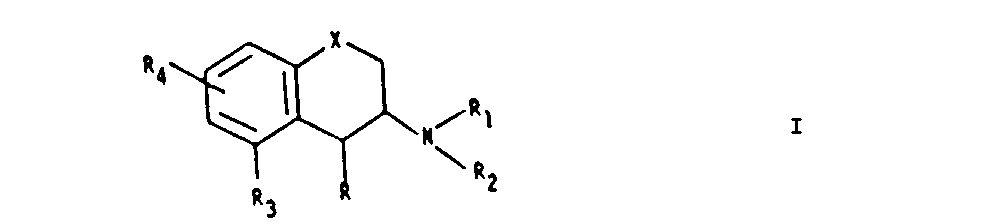 wherein
X is O or 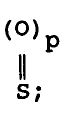
p is an integer 0, 1 or 2;
R is hydrogen, fluoro or C₁-C₆ alkyl;
R₁ is hydrogen, C₁-C₆ alkyl or C₂-C₆ alkenyl;
R₂ is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy; or
R₃ is NR₅R₆, COR₇, 5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy;
R₄ is hydrogen or halogen;
R₅ is hydrogen, C₁-C₆ alkyl or C₂-C₆ alkenyl;
R₆ is C₁-C₆ alkyl or C₂-C₆ alkenyl; or
R₅ and R₆ may together form a 5- or 6- membered ring which may contain 1 or 2 heteroatoms selected from N, O or S;
R₇ is hydrogen, C₁-C₆ alkyl, C₂-C₆ alkenyl, C₁-C₄ alkoxy, NR₈ R₉ or 5- or 6- membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy;
R₈ and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy or may together form a 5- or 6-membered ring containing 1 or 2 heteroatoms selected from N, O or S;
an enantiomer or a pharmaceutically acceptable salt thereof.

19. A pharmaceutical preparation according to claim 18 wherein X, R, R₁, R₂, R₃, R₄, R₇, R₈ and R₉ are defined as in any of claims 2-3, 5-14.

20. A compound according to any of claims 4-14 for use in the treatment of disorders in the centralnervous system, especially 5-hydroxy tryptamine mediated disorders.

21. A compound according to claim 20 for use in the treatment of depression, anxiety, anorexia, senile dementia, migraine, Alzheimer's disiese, hypertension, termoregulator and sexual disturbances, pain and disturbances in the cardiovascular system.

22. Use of a compound according to any of claims 4-14 for the manufacture of a medicament for treatment of disorders in the central nervous system, especially 5-hydroxy tryptamine mediated disorders.

23. Use according to claim 22 for the manufacture of a medicament for treatment of depression, anxiety, anorexia, senile dementia, migraine, Alzheimer's disease, termoregulator and sexual disturbances, pain and disturbances in the cardiovascular system.

24. A process for the preparation of a compound of the formula I according to any of claims 1-17, by
a) converting a compound of formula II 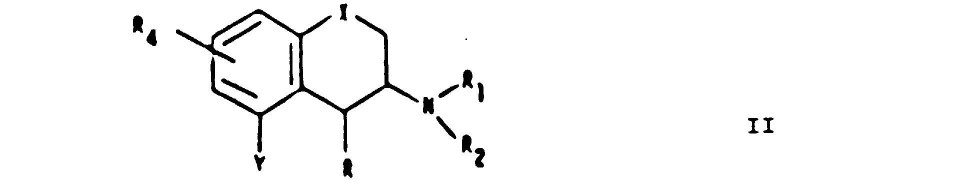 wherein Y is a leaving group and X, R, R₁, R₂ and R₄ are as defined under formula I by a catalytic cycle using a zerovalent transition metal (M°), which undergoes an oxidative addition to the aryl-Y-bonds, treatment with carbon monoxide followed by Z-H, where Z is Cl, Br, OH, ORₚ, where Rₚ is C₁-C₆ alkyl, and the initially formed carbonylated o-aryl-metal-Y complex, to formation of a compound of formula I wherein R₃ is COZ (IA), or
b) reaction by a catalytic cycle using a zerovalent transition metal (M°) which undergoes an oxidative addition to Z-Y, wherein Z is defined Cl, Br, OH, ORₚ, where Rₚ is C₁-C₆ alkyl and Y is a leaving group, treatment with carbon monoxide, followed by addition of the compound of the formula III 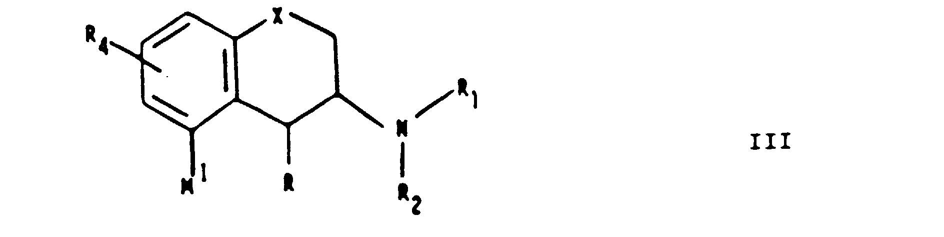 wherein X, R, R₁, R₂ and R₄ are as defined under formula I and M^{I} is a transition metal to formation of a compound of formula I wherein R₃ is COZ (IA), or
c) converting a compound of formula II 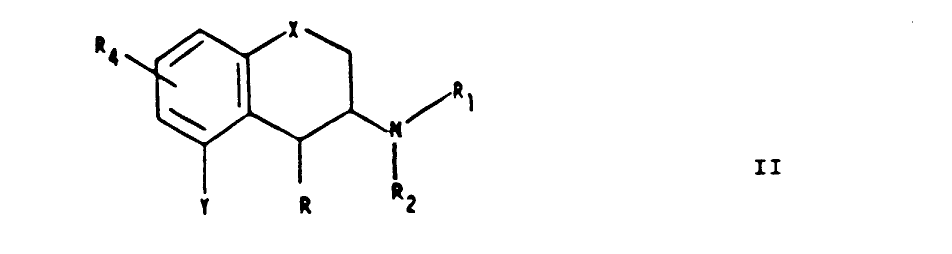 wherein X, R, R₁, R₂ and R₄ are as defined under formula I and Y is a leaving group such as SO₃CF₃, halide by treatment with a cyanide reagent, to formation of a compound of formula I wherein R₃ is CN (IB).
d) amination of a compound of formula IA 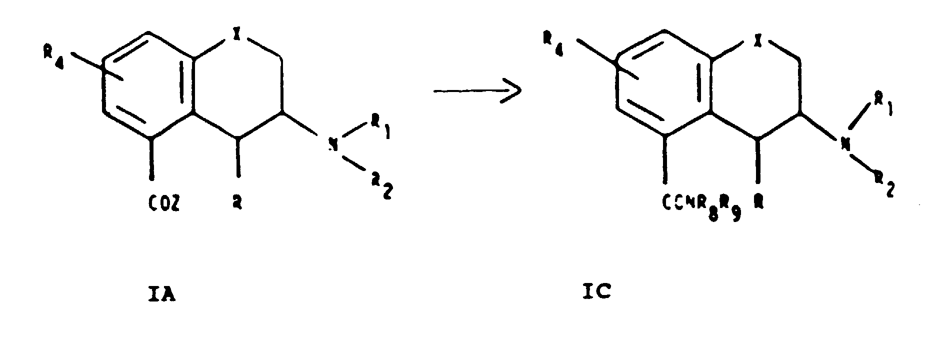 wherein X, R, R₁, R₂, and R₄, are as defined under formula I and Z is Cl, OH or ORₚ, where Rₚ is C₁-C₆ alkyl by reaction with NHR₈R₉ to give the corresponding amide of formula I, where R₃ is CONR₈R₉ (IC), or
e) substitution of a 5-bromochroman/thiochroman derivative 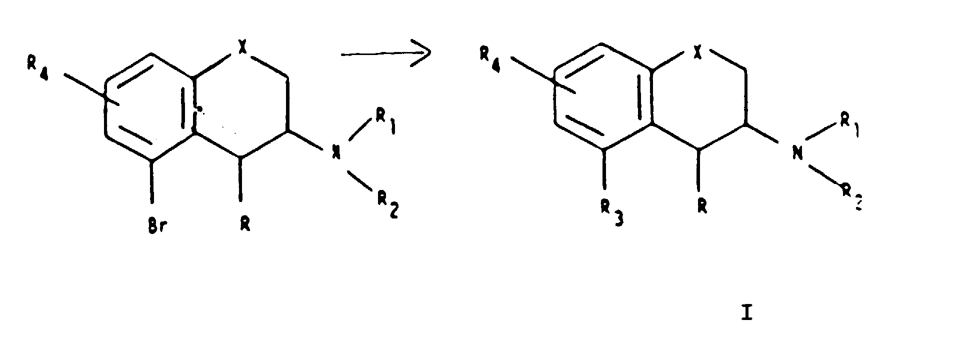 wherein X, R, R₁, R₂ and R₄ are as defined under formula I by treatment with a stannictrialkyl reagent in presence of a zerovalent metal e.g. Pd° to obtain a compound of formula I wherein R₃ is aryl or in presence of carbonmonoxide formed a compound where R₃ is COR₇, where R₇ is C₁-C₆ alkyl, C₂-C₆ alkenyl or aryl, or
f) converting the 5-carboxychroman/thiochroman derivative of formula IA 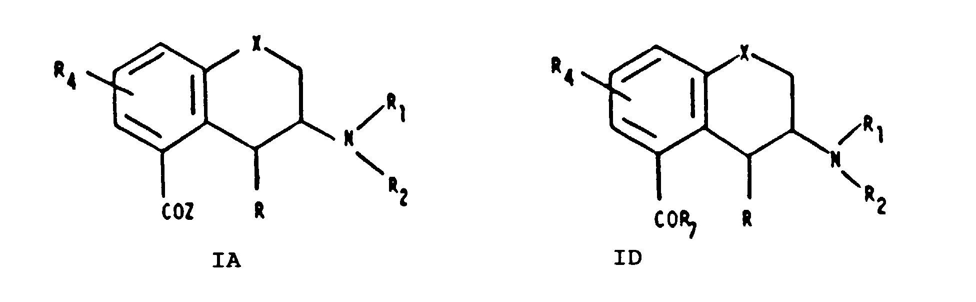 wherein X, R, R₁, R₂ and R₄ are defined as in formula I and Z is Cl, Br by using R₇Li to obtain corresponding compound of formula I when R₃ is COR₇ (ID) where R₇ is defined as C₁-C₆ alkyl, C₂-C₆ alkenyl or aryl, or
g) hydrolysis of a compound of formula I wherein R₃ is CN (IB) 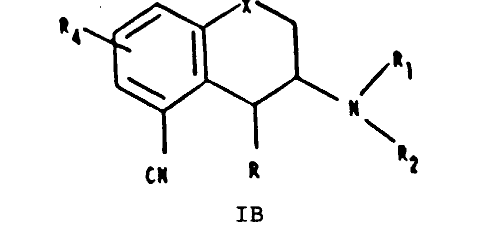 wherein X, R, R₁, R₂ and R₄ are as defined under formula I optionally followed by treatment with thionylhalide to obtain a compound of formula I wherein R₃ is COZ, where Z is OH, Cl or Br (IA), or
h) substitution of a compound of formula I wherein R₃ is CN 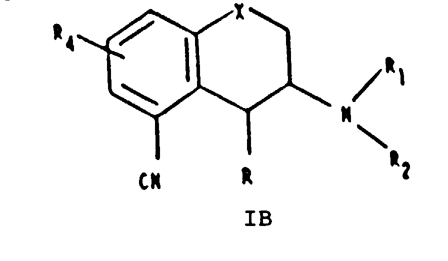 wherein X, R, R₁, R₂ and R₄ are as defined under formula I by treatment with an organometallic reagent followed by hydrolysis to obtain a compound of formula I wherein R₃ is COR₇, where R₇ is C₁-C₆ alkyl, C₂-C₆ alkenyl or aryl (ID), or
i) converting a compound of formula II 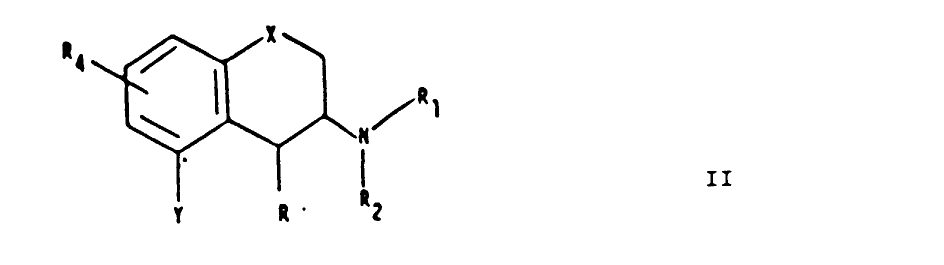 wherein Y is a leaving group and X, R, R₁, R₂ and R₄ are as defined under formula I by a reaction with a transition metal to form a ligand complex, which undergoes an oxidative addition by treatment with a trialkylaryl stannane or aryl-boric acid reagents, to formation of a compound of formula I wherein R₃ is a 5-6-membered aryl which may contain 1 or 2 heteroatoms selected from N, O or S being either substituted or fused at two adjacent carbon atoms;
whereupon optionally a base obtained is converted to a acid addition salt or a salt obtained is converted to the base or to a different, acid addition salt, or optionally an isomeric mixture obatined is separated to a pure enantiomer.

25. A compound according to any of claims 11-14 for use in theraphy.

## Claims (Claims for the following Contracting State(s): ES)

1. A process for the preparation of a compound of the formula 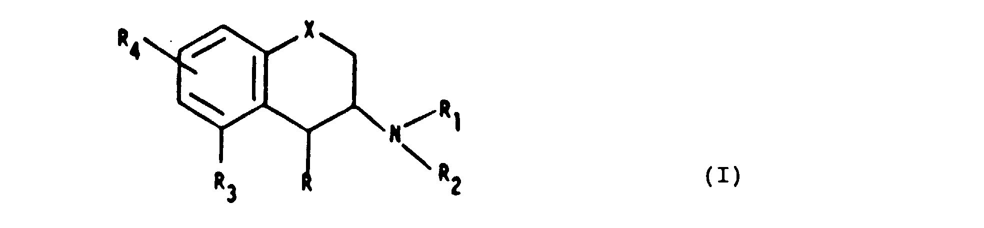 wherein
X is O or 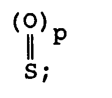
p is an integer 0, 1 or 2;
R is hydrogen, fluoro or C₁-C₆ alkyl;
R₁ is hydrogen, C₁-C₆ alkyl or C₂-C₆ alkenyl;
R₂ is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy; or
R₃ is CN, SO₃CF₃, N₃, NR₅R₆, COR₇, 5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy;
R₄ is hydrogen or halogen;
R₅ is hydrogen, C₁-C₆ alkyl or C₂-C₆ alkenyl;
R₆ is C₁-C₆ alkyl or C₂-C₆ alkenyl; or
R₅ and R₆ may together form a 5- or 6-membered ring which may contain 1 or 2 heteroatoms selected from N, O or S;
R₇ is hydrogen, hydroxy, chloro, bromo, C₁-C₆ alkyl, C₂-C₆ alkenyl, C₁-C₄ alkoxy, NR₈R₉ or 5- or 6-membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy;
R₈ and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy or may together form a 5- or 6-membered ring containing 1 or 2 heteroatoms selected from N, O or S;
an enantiomer or a salt thereof, by
a) converting a compound of formula II 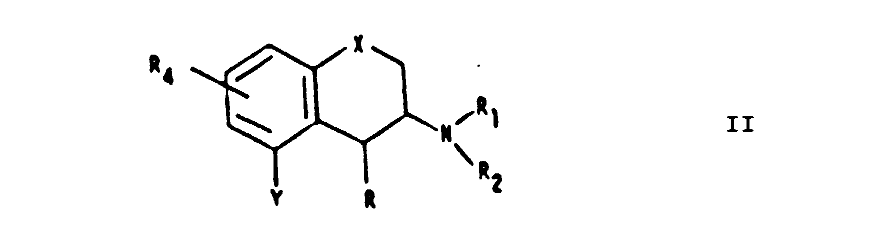 wherein Y is a leaving group and X, R, R₁, R₂ and R₄ are as defined under formula I by a catalytic cycle using a zerovalent transition metal (M°), which undergoes an oxidative addition to the aryl-Y-bonds, treatment with carbon monoxide followed by Z-H, where Z is Cl, Br, OH, ORₚ, where Rₚ is C₁-C₆ alkyl, and the initially formed carbonylated o-aryl-metal-Y complex, to formation of a compound of formula I wherein R₃ is COZ (IA), or
b) reaction by a catalytic cycle using a zerovalent transition metal (M°) which undergoes an oxidative addition to Z-Y, wherein Z is defined Cl, Br, OH, ORₚ, where Rₚ is C₁-C₆ alkyl and Y is a leaving group, treatment with carbon monoxide, followed by addition of the compound of the formula III 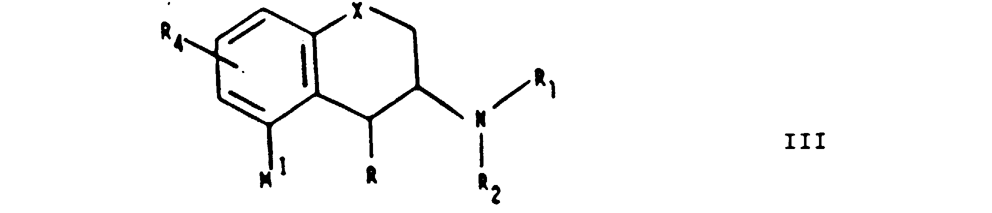 wherein X, R, R₁, R₂ and R₄ are as defined under formula I and M^{I} is a transition metal to formation of a compound of formula I wherein R₃ is COZ (IA), or
c) converting a compound of formula II 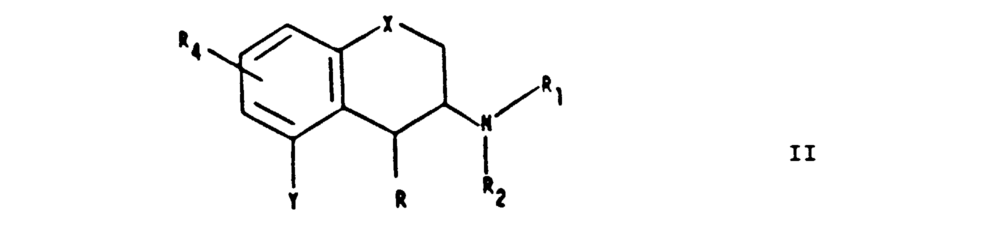 wherein X, R, R₁, R₂ and R₄ are as defined under formula I and Y is a leaving group such as SO₃CF₃, halide by treatment with a cyanide reagent, to formation of a compound of formula I wherein R₃ is CN (IB).
d) amination of a compound of formula IA 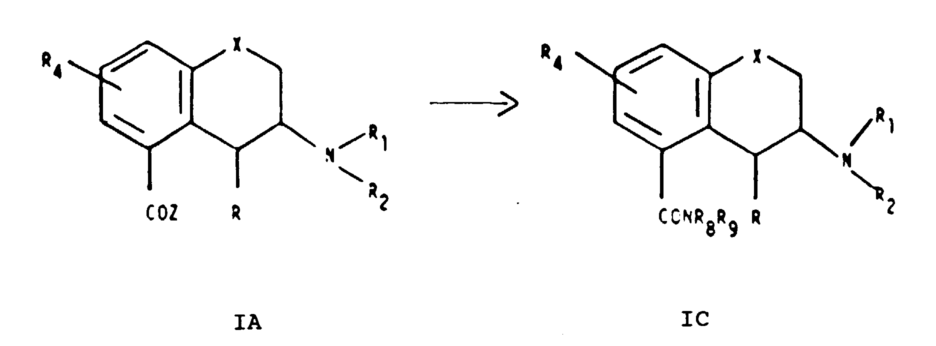 wherein X, R, R₁, R₂, and R₄, are as defined under formula I and Z is Cl, OH or ORₚ, where Rₚ is C₁-C₆ alkyl by reaction with NHR₈R₉ to give the corresponding amide of formula I, where R₃ is CONR₈R₉ (IC), or
e) substitution of a 5-bromochroman/thiochroman derivative 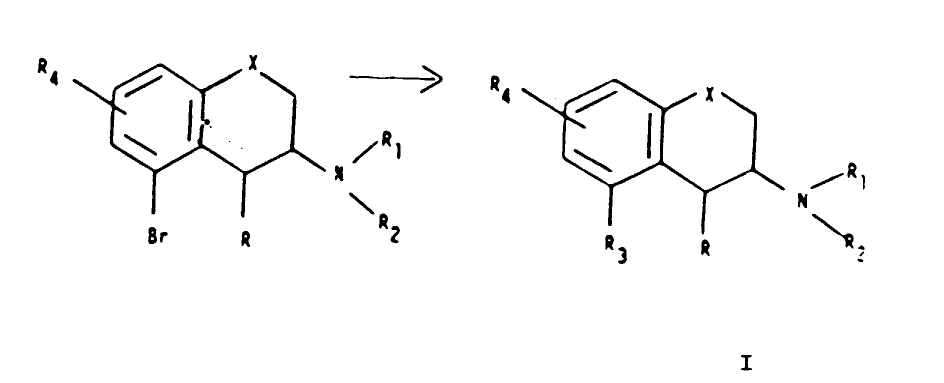 wherein X, R, R₁, R₂ and R₄ are as defined under formula I by treatment with a stannictrialkyl reagent in presence of a zerovalent metal e.g. Pd° to obtain a compound of formula I wherein R₃ is aryl or in presence of carbonmonoxide formed a compound where R₃ is COR₇, where R₇ is C₁-C₆ alkyl, C₂-C₆ alkenyl or aryl, or
f) converting the 5-carboxychroman/thiochroman derivative of formula IA 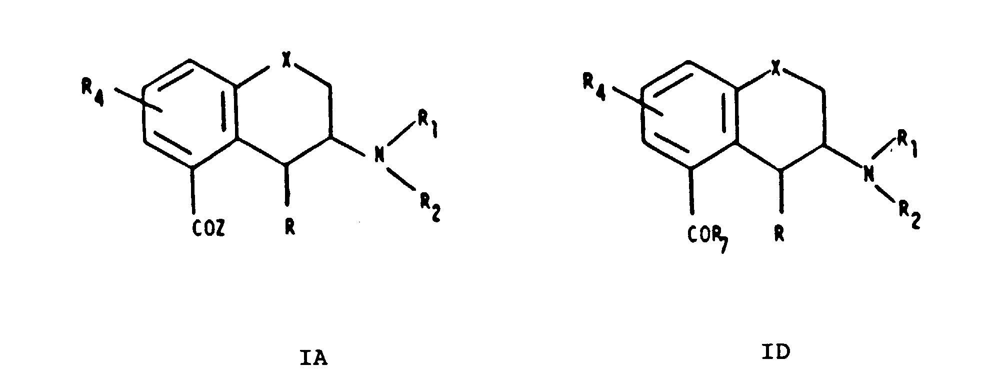 wherein X, R, R₁, R₂ and R₄ are defined as in formula I and Z is Cl, Br by using R₇Li to obtain corresponding compound of formula I when R₃ is COR₇ (ID) where R₇ is defined as C₁-C₆ alkyl, C₂-C₆ alkenyl or aryl, or
g) hydrolysis of a compound of formula I wherein R₃ is CN (IB) 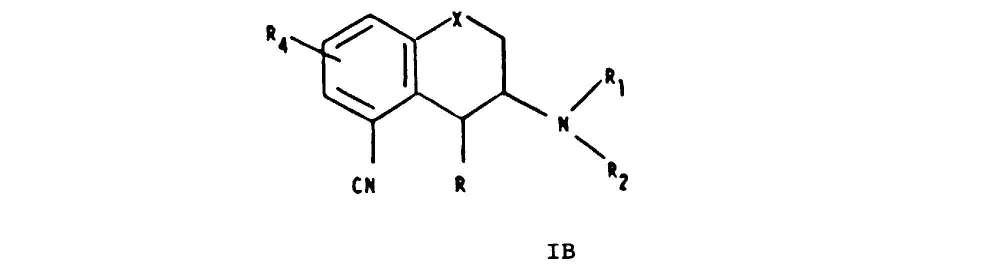 wherein X, R, R₁, R₂ and R₄ are as defined under formula I optionally followed by treatment with thionylhalide to obtain a compound of formula I wherein R₃ is COZ, where Z is OH, Cl or Br (IA), or
h) substitution of a compound of formula I wherein R₃ is CN 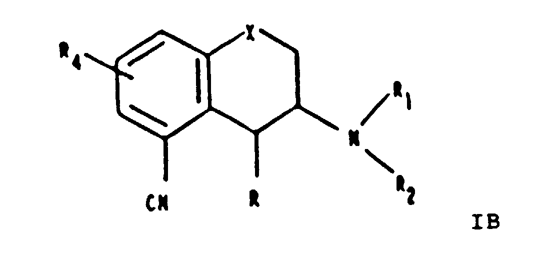 wherein X, R, R₁, R₂ and R₄ are as defined under formula I by treatment with an organometallic reagent followed by hydrolysis to obtain a compound of formula I wherein R₃ is COR₇, where R₇ is C₁-C₆ alkyl, C₂-C₆ alkenyl or aryl (ID), or
i) converting a compound of formula II 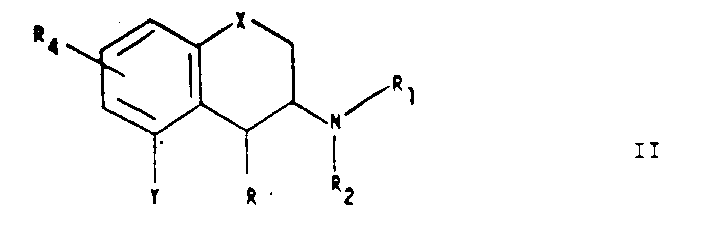 wherein Y is a leaving group and X, R, R₁, R₂ and R₄ are as defined under formula I by a reaction with a transition metal to form a ligand complex, which undergoes an oxidative addition by treatment with a trialkylaryl stannane or aryl-boric acid reagents, to formation of a compound of formula I wherein R₃ is a 5-6-membered aryl which may contain 1 or 2 heteroatoms selected from N, O or S being either substituted or fused at two adjacent carbon atoms;
whereupon optionally a base obtained is converted to a acid addition salt or a salt obtained is converted to the base or to a different, acid addition salt, or optionally an isomeric mixture obatined is separated to a pure enantiomer.

2. A process according to claim 1 for the preparation of a compound according to claim 1 wherein X is O.

3. A process according to claim 1 for the preparation of a compound according to claim 1 wherein X is 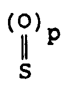 and p is 0, 1 or 2.

4. A process according to claim 1 for the preparation of a compound according to any of claims 1, 2, or 3 wherein R₃ is NR₅R₆, COR₇, 5- or 6- membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substitutents independently selected from halogen, CN CF₃, C₁-C₆ alkyl, C₂-C₆ alkenyl or C₁-C₄ alkoxy; R₇ is hydrogen, C₁-C₆ alkyl, C₂-C₆ alkenyl, C₁-C₄ alkoxy, NR₈ R₉ or 5- or 6- membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy; X, p, R, R₁, R₂, R₄, R₅, R₆, R₈ and R₉ are defined as in claim 1, an enantiomer or a pharmaceutically acceptable salt thereof for use in theraphy.

5. A process according to claim 1 for the preparation of a compound according to any of claims 1-4, wherein R₁ and R₂ are the same or different and selected from hydrogen, n-propyl, i-propyl and cyclopropyl.

6. A process according to claim 1 for the preparation of a compound according to any of claim 1-5, wherein R₃ is COR₇.

7. A process according to claim 1 for the preparation of a compound according to claim 6 wherein R₇ is C₁-C₄ alkyl, phenyl, furanyl or thienyl optionally substituted with halogen, NR₈ R₉ wherein R₈ and R₉ are each independently hydrogen or C₁-C₄ alkyl, or C₁-C₄ alkoxy.

8. A process according to claim 1 for the preparation of a compound according to claim 6 wherein R and R₄ are hydrogen, R₁ and R₂ are n-propyl and R₇ is methyl, ethyl, n-propyl, i-propyl, cyclopropyl, n-butyl, i-butyl, t-butyl, cyclobutyl, thienyl, furanyl, phenyl, N- methylamino, methoxy or fluorophenyl.

9. A process according to claim 1 for the preparation of a compound according to any of claims 1-5 wherein R₃ is phenyl, furanyl, thienyl or fluorophenyl.

10. A process according to claim 1 for the preparation of a compound according to any of claims 1-7 and 9 wherein R₄ is halogen in 8 position.

11. A process according to claim 1 for the preparation of 3-dipropylamino-5-acetylchroman, an enantiomer or a salt thereof.

12. A process according to claim 1 for the preparation of 3-dipropylamino-5-carbamoylchroman, an enantiomer or a salt thereof.

13. A process according to claim 1 for the preparation of 3-dipropylamino-5-N-methylcarbamoylchroman, an enantiomer or a salt thereof.

14. A process according to claim 1 for the preparation of 3-dipropylamino-5-(2-thienylcarbonyl)chroman, an enantiomer or a salt thereof.

15. A process according to claim 1 for the preparation of a compound according to claim 1 wherein R₃ is CN, COOH, COCl, COBr, N₃ or SO₃CF₃, and X, R, R₁, R₂ and R₄, are as defined in claim 1, an enantiomer or a salt thereof.

16. A process according to claim 1 for the preparation of a compound according to claim 15 wherein R₁ and R₂ are the same or different and selected from hydrogen, n-propyl, i-propyl and cyclopropyl.

17. A process according to claim 1 for the preparation of a compound according to claim 16 wherein R and R₄ are hydrogen, R₁ and R₂ are n-propyl and R₃ is SO₃CF₃.

18. Use of a compound according to any of claims 4-14 for the manufacture of a medicament for treatment of disorders in the central nervous system, especially 5-hydroxy tryptamine mediated disorders.

19. Use according to claim 18 for the manufacture of a medicament for treatment of depression, anxiety, anorexia, senile dementia, migraine, Alzheimer's disease, termoregulator and sexual disturbances, pain and disturbances in the cardiovascular system.

## Claims (Claims for the following Contracting State(s): GR)

1. A process for the preparation of a compound of the formula 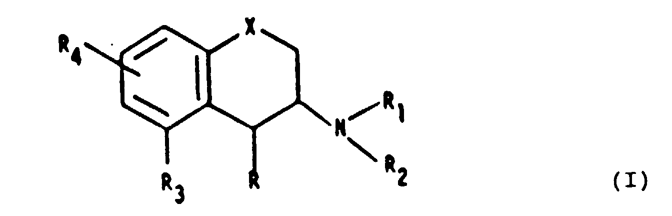 wherein
X is O or 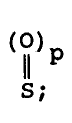
p is an integer 0, 1 or 2;
R is hydrogen, fluoro or C₁-C₆ alkyl;
R₁ is hydrogen, C₁-C₆ alkyl or C₂-C₆ alkenyl;
R₂ is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy; or
R₃ is CN, SO₃CF₃, N₃, NR₅R₆, COR₇, 5- or 6-membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substituents independently selected from halogen, CN, CF₃, C₁-C₆ alkyl, C₂-C₆ alkenyl or C₁-C₄ alkoxy;
R₄ is hydrogen or halogen;
R₅ is hydrogen, C₁-C₆ alkyl or C₂-C₆ alkenyl;
R₆ is C₁-C₆ alkyl or C₂-C₆ alkenyl; or
R₅ and R₆ may together form a 5- or 6-membered ring which may contain 1 or 2 heteroatoms selected from N, O or S;
R₇ is hydrogen, hydroxy, chloro, bromo, C₁-C₆ alkyl, C₂-C₆ alkenyl, C₁-C₄ alkoxy, NR₈R₉ or 5- or 6-membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy;
R₈ and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy or may together form a 5- or 6-membered ring containing 1 or 2 heteroatoms selected from N, O or S;
an enantiomer or a salt thereof, by
a) converting a compound of formula II 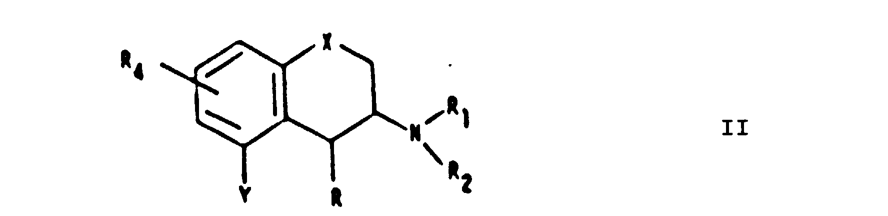 wherein Y is a leaving group and X, R, R₁, R₂ and R₄ are as defined under formula I by a catalytic cycle using a zerovalent transition metal (M°), which undergoes an oxidative addition to the aryl-Y-bonds, treatment with carbon monoxide followed by Z-H, where Z is Cl, Br, OH, ORₚ, where Rₚ is C₁-C₆ alkyl, and the initially formed carbonylated o-aryl-metal-Y complex, to formation of a compound of formula I wherein R₃ is COZ (IA), or
b) reaction by a catalytic cycle using a zerovalent transition metal (M°) which undergoes an oxidative addition to Z-Y, wherein Z is defined Cl, Br, OH, ORₚ, where Rₚ is C₁-C₆ alkyl and Y is a leaving group, treatment with carbon monoxide, followed by addition of the compound of the formula III 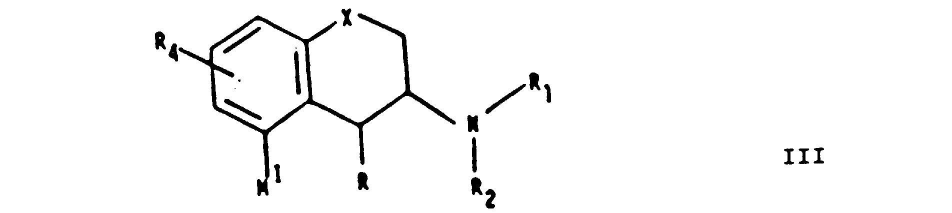 wherein X, R, R₁, R₂ and R₄ are as defined under formula I and M^{I} is a transition metal to formation of a compound of formula I wherein R₃ is COZ (IA), or
c) converting a compound of formula II 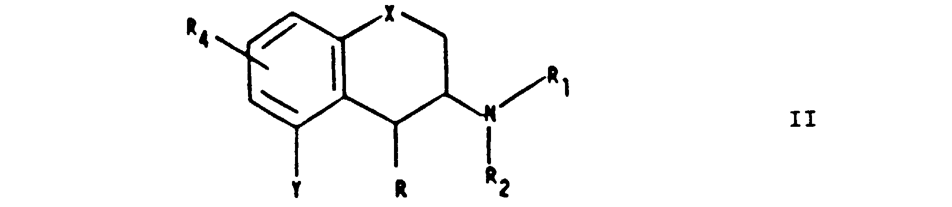 wherein X, R, R₁, R₂ and R₄ are as defined under formula I and Y is a leaving group such as SO₃CF₃, halide by treatment with a cyanide reagent, to formation of a compound of formula I wherein R₃ is CN (IB).
d) amination of a compound of formula IA 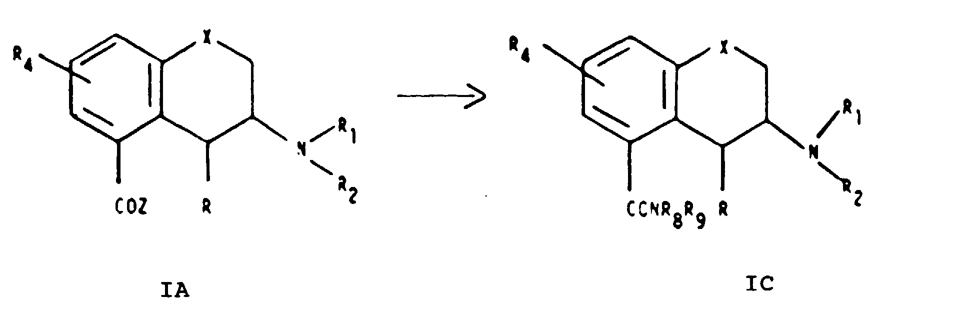 wherein X, R, R₁, R₂, and R₄, are as defined under formula I and Z is Cl, OH or ORₚ, where Rₚ is C₁-C₆ alkyl by reaction with NHR₈R₉ to give the corresponding amide of formula I, where R₃ is CONR₈R₉ (IC), or
e) substitution of a 5-bromochroman/thiochroman derivative 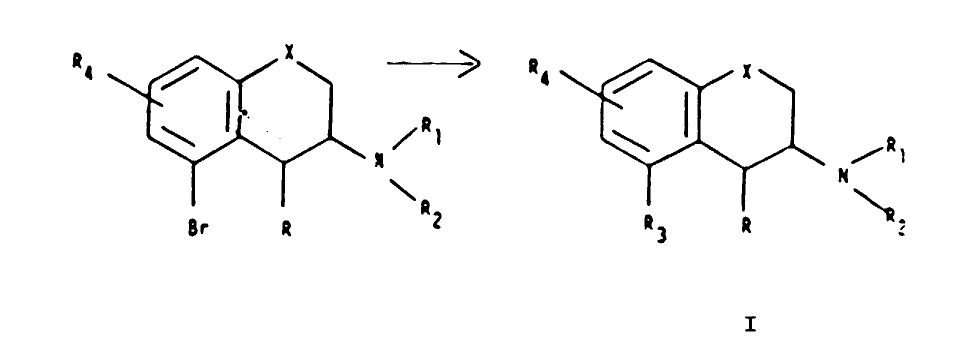 wherein X, R, R₁, R₂ and R₄ are as defined under formula I by treatment with a stannictrialkyl reagent in presence of a zerovalent metal e.g. Pd° to obtain a compound of formula I wherein R₃ is aryl or in presence of carbonmonoxide formed a compound where R₃ is COR₇, where R₇ is C₁-C₆ alkyl, C₂-C₆ alkenyl or aryl, or
f) converting the 5-carboxychroman/thiochroman derivative of formula IA 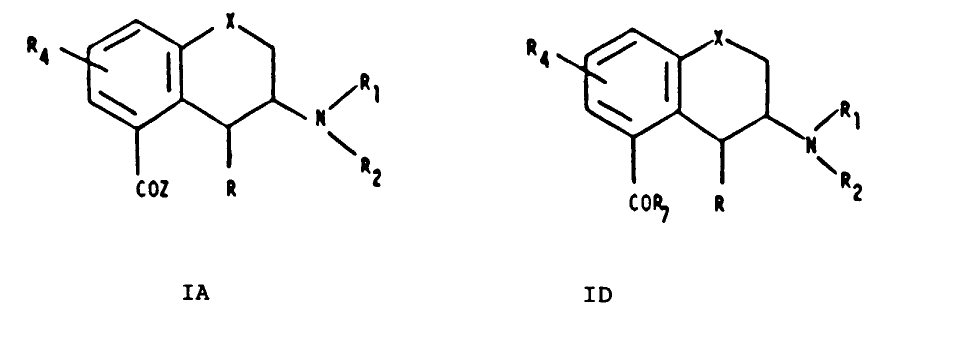 wherein X, R, R₁, R₂ and R₄ are defined as in formula I and Z is Cl, Br by using R₇Li to obtain corresponding compound of formula I when R₃ is COR₇ (ID) where R₇ is defined as C₁-C₆ alkyl, C₂-C₆ alkenyl or aryl, or
g) hydrolysis of a compound of formula I wherein R₃ is CN (IB) 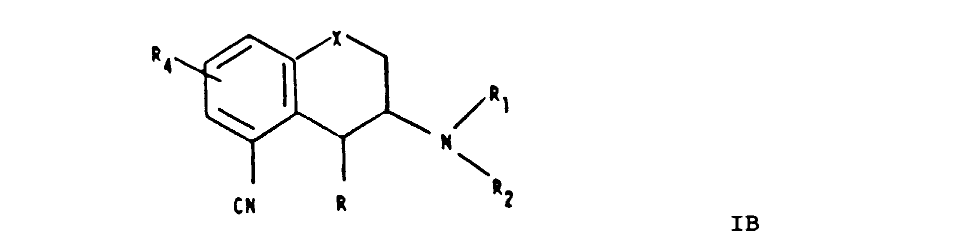 wherein X, R, R₁, R₂ and R₄ are as defined under formula I optionally followed by treatment with thionylhalide to obtain a compound of formula I wherein R₃ is COZ, where Z is OH, Cl or Br (IA), or
h) substitution of a compound of formula I wherein R₃ is CN 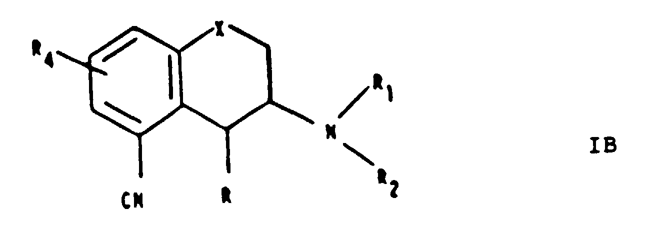 wherein X, R, R₁, R₂ and R₄ are as defined under formula I by treatment with an organometallic reagent followed by hydrolysis to obtain a compound of formula I wherein R₃ is COR₇, where R₇ is C₁-C₆ alkyl, C₂-C₆ alkenyl or aryl (ID), or
i) converting a compound of formula II 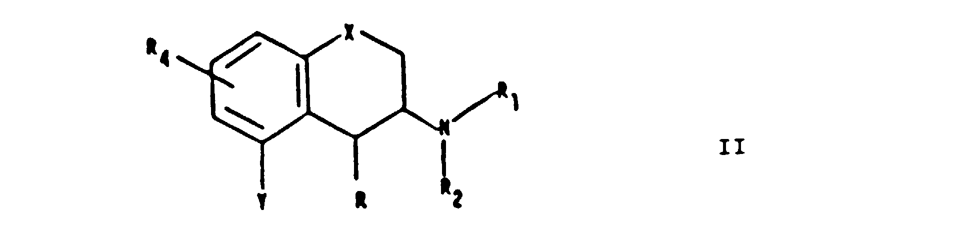 wherein Y is a leaving group and X, R, R₁, R₂ and R₄ are as defined under formula I by a reaction with a transition metal to form a ligand complex, which undergoes an oxidative addition by treatment with a trialkylaryl stannane or aryl-boric acid reagents, to formation of a compound of formula I wherein R₃ is a 5-6-membered aryl which may contain 1 or 2 heteroatoms selected from N, O or S being either substituted or fused at two adjacent carbon atoms,
whereupon optionally a base obtained is converted to a acid addition salt or a salt obtained is converted to the base or to a different, acid addition salt, or optionally an isomeric mixture obatined is separated to a pure enantiomer.

2. A process according to claim 1 for the preparation of a compound according to claim 1 wherein X is O.

3. A process according to claim 1 for the preparation of a compound according to claim 1 wherein X is 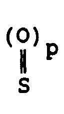 and p is 0, 1 or 2.

4. A process according to claim 1 for the preparation of a compound according to any of claims 1, 2, or 3 wherein R₃ is NR₅ R₆, COR₇, 5- or 6- membered aryl which may contain 1 or 2 heteroatoms selected from N, O or S and being either (i) optionally substituted by one or more substituents independently selected from halogen, CN, CF₃, C₁-C₆ alkyl, C₂-C₆ alkenyl or C₁-C₄ alkoxy or either (ii) fused at two adjacent carbon atoms to an aryl ring, said aryl ring being optionally substituted by one or more substitutents independently selected from halogen, CN CF₃, C₁-C₆ alkyl, C₂-C₆ alkenyl or C₁-C₄ alkoxy; R₇ is hydrogen, C₁-C₆ alkyl, C₂-C₆ alkenyl, C₁-C₄ alkoxy, NR₈ R₉ or 5- or 6-membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy;
X, p, R, R₁, R₂, R₄, R₅, R₆, R₈ and R₉ are defined as in claim 1, an enantiomer or a pharmaceutically acceptable salt thereof for use in theraphy.

5. A process according to claim 1 for the preparation of a compound according to any of claims 1-4, wherein R₁ and R₂ are the same or different and selected from hydrogen, n-propyl, i-propyl and cyclopropyl.

6. A process according to claim 1 for the preparation of a compound according to any of claim 1-5, wherein R₃ is COR₇.

7. A process according to claim 1 for the preparation of a compound according to claim 6 wherein R₇ is C₁-C₄ alkyl, phenyl, furanyl or thienyl optionally substituted with halogen, NR₈ R₉ wherein R₈ and R₉ are each independently hydrogen or C₁-C₄ alkyl, or C₁-C₄ alkoxy.

8. A process according to claim 1 for the preparation of a compound according to claim 6 wherein R and R₄ are hydrogen, R₁ and R₂ are n-propyl and R₇ is methyl, ethyl, n-propyl, i-propyl, cyclopropyl, n-butyl, i-butyl, t-butyl, cyclobutyl, thienyl, furanyl, phenyl, N- methylamino, methoxy or fluorophenyl.

9. A process according to claim 1 for the preparation of a compound according to any of claims 1-5 wherein R₃ is phenyl, furanyl, thienyl or fluorophenyl.

10. A process according to claim 1 for the preparation of a compound according to any of claims 1-7 and 9 wherein R₄ is halogen in 8 position.

11. A process according to claim 1 for the preparation of 3-dipropylamino-5-acetylchroman, an enantiomer or a salt thereof.

12. A process according to claim 1 for the preparation of 3-dipropylamino-5-carbamoylchroman, an enantiomer or a salt thereof.

13. A process according to claim 1 for the preparation of 3-dipropylamino-5-N-methylcarbamoylchroman, an enantiomer or a salt thereof.

14. A process according to claim 1 for the preparation of 3-dipropylamino-5-(2-thienylcarbonyl)chroman, an enantiomer or a salt thereof.

15. Use of a compound according to any of claims 4-14 for the manufacture of a medicament for treatment of disorders in the central nervous system, especially 5-hydroxy tryptamine mediated disorders.

16. Use according to claim 15 for the manufacture of a medicament for treatment of depression, anxiety, anorexia, senile dementia, migraine, Alzheimer's disease, termoregulator and sexual disturbances, pain and disturbances in the cardiovascular system.

17. A compound of the formula 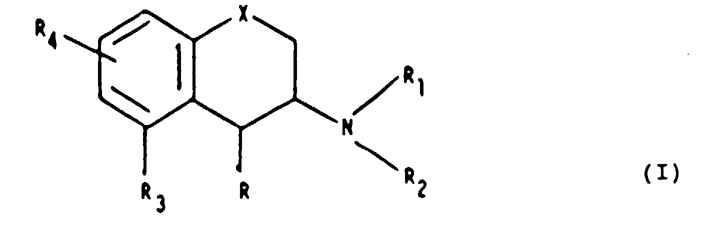 wherein
X is O or 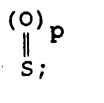
p is an integer 0, 1 or 2;
R is hydrogen, fluoro or C₁-C₆ alkyl;
R₁ is hydrogen, C₁-C₆ alkyl or C₂-C₆ alkenyl;
R₂ is hydrogen, C₁-C₆ alkyl, C₂-C₆ alkenyl, C₁-C₄ alkylaryl where aryl may contain 1 or 2 heteroatoms selected from N, O or S optionally substituted by halogen, CN, CF₃, C₁-C₆ alkyl, C₂-C₆ alkenyl or C₁-C₄ alkoxy; or
R₃ is CN, COOH, COCl, COBr, N₃ or SO₃CF₃;
R₄ is hydrogen or halogen;
R₅ is hydrogen, C₁-C₆ alkyl or C₂-C₆ alkenyl;
R₆ is C₁-C₆ alkyl or C₂-C₆ alkenyl; or
R₅ and R₆ may together form a 5- or 6-membered ring which may contain 1 or 2 heteroatoms selected from N, O or S;
R₇ is hydrogen, hydroxy, chloro, bromo, C₁-C₆ alkyl, C₂-C₆ alkenyl, C₁-C₄ alkoxy, NR₈R₉ or 5- or 6-membered aryl which may contain 1 or 2 heteroatoms selected from N, O or S optionally substituted by one or more of halogen, CN, CF₃, C₁-C₆ alkyl, C₂-C₆ alkenyl, or C₁-C₄ alkoxy;
R₈ and R₉ are each independently hydrogen, C₁-C₆ alkyl, C₂-C₆ alkenyl, 5- or 6- membered aryl which may contain 1 or 2 heteroatoms selected from N, O or S optionally substituted by halogen, CN, CF₃, C₁-C₆ alkyl, C₂-C₆ alkenyl, C₁-C₄ alkoxy or may together form a 5- or 6- membered ring containing 1 or 2 heteroatoms selected from N, O or S;
an enantiomer or a salt thereof.

18. A compound according to claim 17 wherein R₁ and R₂ are the same or different and selected from hydrogen, n-propyl, i-propyl and cyclopropyl.

19. A compound according to claim 18 wherein R and R₄ are hydrogen, R₁ and R₂ are n-propyl and R₃ is SO₃CF₃.

## Patentansprüche (Patentansprüche für folgende(n) Vertragsstaat(en): AT, BE, CH, DE, DK, FR, GB, IT, LI, LU, NL, SE)

1. Verbindung der Formel 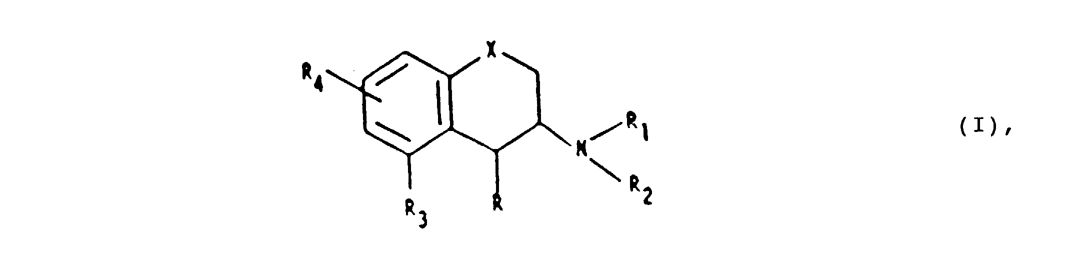 worin X die Bedeutung O oder 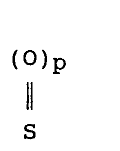 hat; p eine ganze Zahl Null, 1 oder 2 ist; R Wasserstoff, Fluor oder C₁-C₆-Alkyl bedeutet; R₁ Wasserstoff, C₁-C₆-Alkyl oder C₂-C₆-Alkenyl darstellt; R₂ bedeutet: Wasserstoff, C₁-C₆-Alkyl, C₂-C₆-Alkenyl, C₁-C₄-Alkylaryl, wobei Aryl 1 oder 2 Heteroatome, ausgewählt aus N, O oder S, gegebenenfalls substituiert durch Halogen, CN, CF₃, C₁-C₆-Alkyl, C₂-C₆-Alkenyl oder C₁-C₄-Alkoxy, enthalten kann; oder R₃ darstellt: CN, SO₃CF₃, N₃, NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₄ Wasserstoff oder Halogen ist; R₅ Wasserstoff, C₁-C₆-Alkyl oder C₂-C₆-Alkenyl bedeutet; R₆ C₁-C₆-Alkyl oder C₂-C₆-Alkenyl darstellt; oder R₅ und R₆ zusammen einen 5- oder 6-gliedrigen Ring bilden können, der 1 oder 2 Heteroatome, ausgewählt aus N, O oder S, enthalten kann; R₇ bedeutet: Wasserstoff, Hydroxy, Chlor, Brom, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; R₈ und R₉ jeweils unabhängig darstellen: Wasserstoff, C₁-C₆-Alkyl, C₂-C₆-Alkenyl, 5- oder 6-gliedriges Aryl, das 1 oder 2 Heteroatome, ausgewählt aus N, O oder S, gegebenenfalls substituiert durch Halogen, CN, CF₃, C₁-C₆-Alkyl, C₂-C₆-Alkenyl, C₁-C₄-Alkoxy, enthalten kann, oder zusammen einen 5- oder 6-gliedrigen Ring, der 1 oder 2 Heteroatome, ausgewählt aus N, O oder S, enthält, bilden können; ein Enantiomer oder ein Salz hievon.

2. Verbindung nach Anspruch 1, worin X die Bedeutung O hat.

3. Verbindung nach Anspruch 1, worin X die Bedeutung 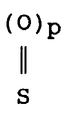 hat, und p Null, 1 oder 2 ist.

4. Verbindung nach einem der Ansprüche 1, 2 oder 3, worin R₃ bedeutet: NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₇ darstellt: Wasserstoff, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; X, p, R, R₁, R₂, R₄, R₅, R₆, R₈ und R₉ wie in Anspruch 1 definiert sind; ein Enantiomer oder ein pharmazeutisch annehmbares Salz hievon zur Verwendung in der Therapie.

5. Verbindung nach einem der Ansprüche 1 bis 4, worin R₁ und R₂ gleich oder verschieden und aus Wasserstoff, n-Propyl, i-Propyl und Cyclopropyl ausgewählt sind.

6. Verbindung nach einem der Ansprüche 1 bis 5, worin R₃ die Bedeutung COR₇ hat.

7. Verbindung nach Anspruch 6, worin R₇ C₁-C₄-Alkyl, Phenyl, Furanyl oder Thienyl, gegebenenfalls substituiert mit Halogen, NR₈R₉ darstellt, wobei R₈ und R₉ jeweils unabhängig Wasserstoff oder C₁-C₄-Alkyl oder C₁-C₄-Alkoxy bedeuten.

8. Verbindung nach Anspruch 6, worin R und R₄ Wasserstoff sind, R₁ und R₂ n-Propyl bedeuten, und R₇ Methyl, Ethyl, n-Propyl, i-Propyl, Cyclopropyl, n-Butyl, i-Butyl, tert.Butyl, Cyclobutyl, Thienyl, Furanyl, Phenyl, N-Methylamino, Methoxy oder Fluorphenyl darstellt.

9. Verbindung nach einem der Ansprüche 1 bis 5, worin R₃ Phenyl, Furanyl, Thienyl oder Fluorphenyl bedeutet.

10. Verbindung nach einem der Ansprüche 1 bis 7 und 9, worin R₄ Halogen in Stellung 8 darstellt.

11. Verbindung nach Anspruch 1, welche 3-Dipropylamino-5-acetylchroman, ein Enantiomer oder ein Salz hievon ist.

12. Verbindung nach Anspruch 1, welche 3-Dipropylamino-5-carbamoylchroman, ein Enantiomer oder ein Salz hievon ist.

13. Verbindung nach Anspruch 1, welche 3-Dipropylamino-5-N-methylcarbamoylchroman, ein Enantiomer oder ein Salz hievon ist.

14. Verbindung nach Anspruch 1, welche 3-Dipropylamino-5-(2-thienylcarbonyl)-chroman, ein Enantiomer oder ein Salz hievon ist.

15. Verbindung nach Anspruch 1, worin R₃ CN, COOH, COCl, COBr, N₃ oder SO₃CF₃ bedeutet, und X, R, R₁, R₂ und R₄ wie in Anspruch 1 definiert sind, ein Enantiomer oder ein Salz hievon.

16. Verbindung nach Anspruch 15, worin R₁ und R₂ gleich oder verschieden und aus Wasserstoff, n-Propyl, i-Propyl und Cyclopropyl ausgewählt sind.

17. Verbindung nach Anspruch 16, worin R und R₄ Wasserstoff sind, R₁ und R₂ n-Propyl bedeuten, und R₃ SO₃CF₃ darstellt.

18. Pharmazeutische Zubereitung, welche als aktiven Bestandteil enthält: eine Verbindung der Formel (I) 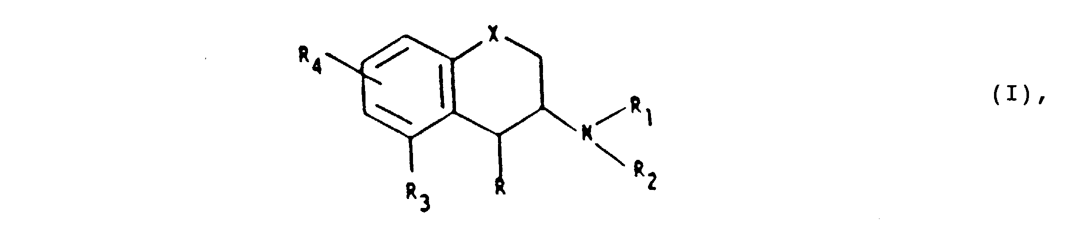 worin X die Bedeutung O oder 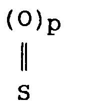 hat, p eine ganze Zahl Null, 1 oder 2 ist; R Wasserstoff, Fluor oder C₁-C₆-Alkyl bedeutet; R₁ Wasserstoff, C₁-C₆-Alkyl oder C₂-C₆-Alkenyl darstellt; R₂ bedeutet: Wasserstoff, C₁-C₆-Alkyl, C₂-C₆-Alkenyl, C₁-C₄-Alkylaryl, wobei Aryl 1 oder 2 Heteroatome, ausgewählt aus N, O oder S, gegebenenfalls substituiert durch Halogen, CN, CF₃, C₁-C₆-Alkyl, C₂-C₆-Alkenyl oder C₁-C₄-Alkoxy, enthalten kann; oder R₃ darstellt: NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₄ Wasserstoff oder Halogen ist; R₅ Wasserstoff, C₁-C₆-Alkyl oder C₂-C₆-Alkenyl bedeutet; R₆ C₁-C₆-Alkyl oder C₂-C₆-Alkenyl darstellt; oder R₅ und R₆ zusammen einen 5- oder 6-gliedrigen Ring bilden können, der 1 oder 2 Heteroatome, ausgewählt aus N, O oder S, enthalten kann; R₇ bedeutet: Wasserstoff, C₁-C₆-Alkyl, C₂-C₆-Alkenyl, C₁-C₄-Alkoxy, NR₈R₉ oder 5-oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; R₈ und R₉ jeweils unabhängig darstellen: Wasserstoff, C₁-C₆-Alkyl, C₂-C₆-Alkenyl, 5- oder 6-gliedriges Aryl, das 1 oder 2 Heteroatome, ausgewählt aus N, O oder S, gegebenenfalls substituiert durch Halogen, CN, CF₃, C₁-C₆-Alkyl, C₂-C₆-Alkenyl, C₁-C₄-Alkoxy, enthalten kann, oder zusammen einen 5- oder 6-gliedrigen Ring, der 1 oder 2 Heteroatome, ausgewählt aus N, O oder S, enthält, bilden können; ein Enantiomer oder ein pharmazeutisch annehmbares Salz hievon.

19. Pharmazeutische Zubereitung nach Anspruch 18, worin X, R, R₁, R₂, R₃, R₄, R₇, R₈ und R₉ wie in einem der Ansprüche 2, 3 und 5 bis 14 definiert sind.

20. Verbindung nach einem der Ansprüche 4 bis 14 zur Verwendung bei der Behandlung von Störungen des Zentralnervensystems, insbesondere 5-Hydroxytryptamin-vermittelten Störungen.

21. Verbindung nach Anspruch 20 zur Verwendung bei der Behandlung von Depressionen, Anxietas, Anorexie, Altersdemenz, Migräne, Alzheimer-Krankheit, Hypertension, thermoregulatorischen und sexuellen Störungen, Schmerzen und Störungen des kardiovaskulären Systems.

22. Verwendung einer Verbindung nach einem der Ansprüche 4 bis 14 bei der Herstellung eines Medikaments zur Behandlung von Störungen des Zentralnervensystems, insbesondere 5-Hydroxytryptamin-vermittelten Störungen.

23. Verwendung nach Anspruch 22 bei der Herstellung eines Medikaments zur Behandlung von Depressionen, Anxietas, Anorexie, Altersdemenz, Migräne, Alzheimer-Krankheit, thermoregulatorischen und sexuellen Störungen, Schmerzen und Störungen des kardiovaskulären Systems.

24. Verfahren zur Herstellung einer Verbindung der Formel (I) nach einem der Ansprüche 1 bis 17, durch
a) Überführen einer Verbindung der Formel (II) 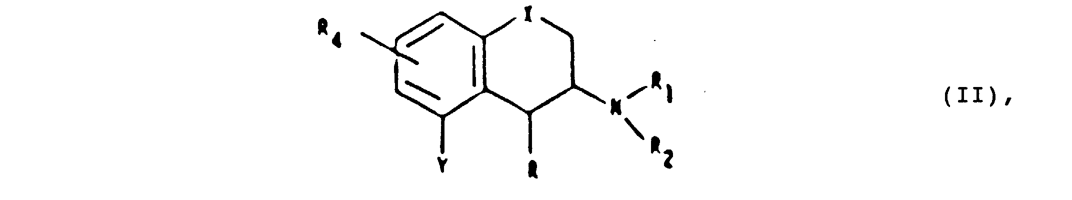 worin Y eine Abgangsgruppe ist, und X, R, R₁, R₂ und R₄ wie gemäß Formel (I) definiert sind, durch einen katalytischen Zyklus unter Verwendung eines nullwertigen Übergangsmetalls (M⁰), das eine oxidative Addition an die Aryl-Y-Bindungen eingeht, Behandlung mit Kohlenmonoxid, gefolgt von Z-H, wobei Z bedeutet: Cl, Br, OH, ORₚ, worin Rₚ C₁-C₆-Alkyl darstellt, und den anfänglich gebildeten carbonylierten o-Aryl-Metall-Y-Komplex zur Bildung einer Verbindung der Formel (I), worin R₃ COZ bedeutet, (IA), oder
b) Umsetzen durch einen katalytischen Zyklus unter Verwendung eines nullwertigen Übergangsmetalls (M⁰), das eine oxidative Addition an Z-Y eingeht, wobei Z definiert ist als Cl, Br, OH, ORₚ, worin Rp C₁-C₆-Alkyl darstellt, und Y eine Abgangsgruppe ist, Behandlung mit Kohlenmonoxid, gefolgt vom Zusatz der Verbindung der Formel (III) 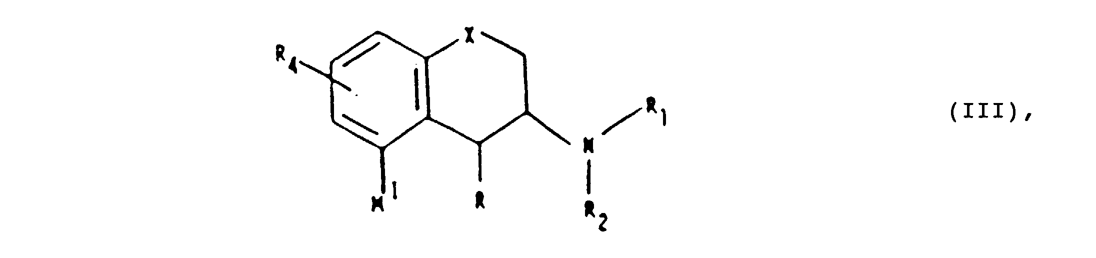 worin X, R, R₁, R₂ und R₄ wie gemäß Formel (I) definiert sind, und M^{I} ein Übergangsmetall bedeutet, zur Bildung einer Verbindung der Formel (I), worin R₃ COZ bedeutet, (IA), oder
c) Überführen einer Verbindung der Formel (II) 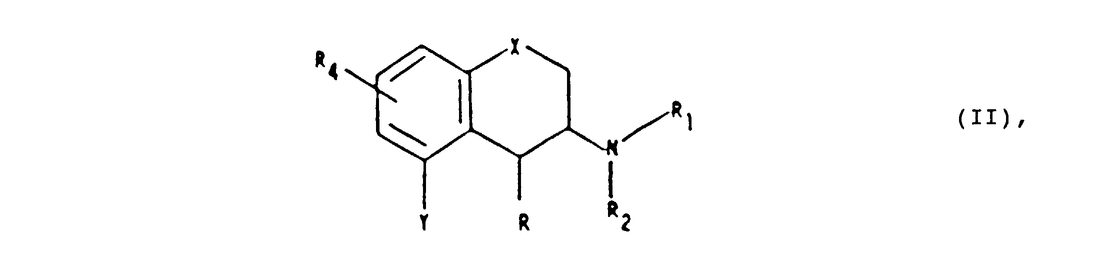 worin X, R, R₁, R₂ und R₄ wie gemäß Formel (I) definiert sind, und Y eine Abgangsgruppe, wie SO₃CF₃, Halogenid, ist, durch Behandlung mit einem Cyanid-Reagens zur Bildung einer Verbindung der Formel (I), worin R₃ CN bedeutet, (IB),
d) Aminieren einer Verbindung der Formel (IA) 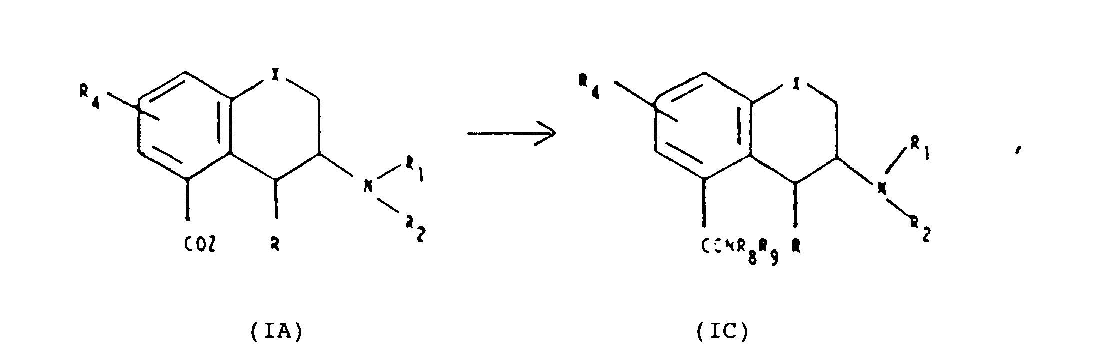 worin X, R, R₁, R₂ und R₄ wie gemäß Formel (I) definiert sind, und Z bedeutet: Cl, OH oder ORₚ, wobei Rₚ C₁-C₆-Alkyl darstellt, durch Umsetzen mit NHR₈R₉, wobei das entsprechende Amid der Formel (I) erhalten wird, worin R₃ CONR₈R₉ bedeutet, (IC), oder
e) Substitution eines 5-Bromchroman/Thiochroman-Derivats 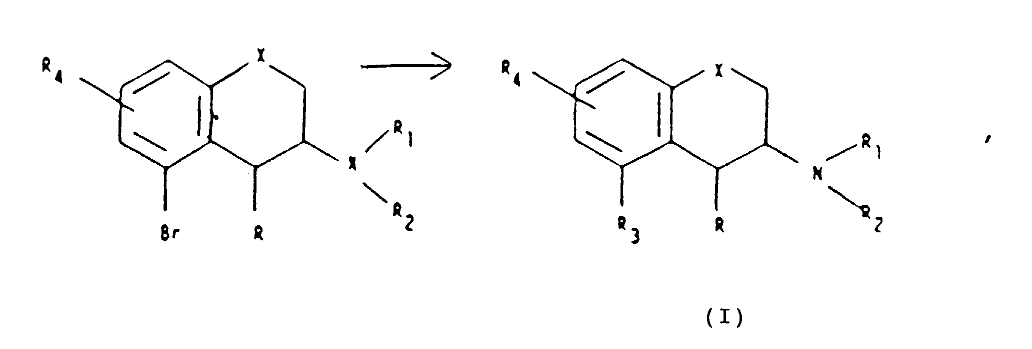 worin X, R, R₁, R₂ und R₄ wie gemäß Formel (I) definiert sind, durch Behandlung mit einem Zinn(IV)-trialkyl-Reagens in Anwesenheit eines nullwertigen Metalls, z.B. Pd⁰, wobei eine Verbindung der Formel (I) erhalten wird, worin R₃ Aryl bedeutet, oder in Anwesenheit von Kohlenmonoxid zur Bildung einer Verbindung, worin R₃ COR₇ bedeutet, wobei R₇ C₁-C₆-Alkyl, C₂-C₆-Alkenyl oder Aryl darstellt, oder
f) Überführen des 5-Carboxychroman/Thiochroman-Derivats der Formel (IA) 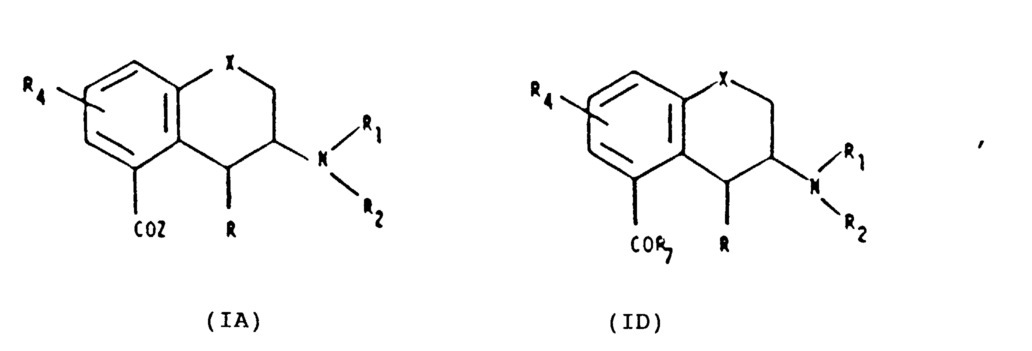 worin X, R, R₁, R₂ und R₄ wie gemäß Formel (I) definiert sind, und Z Cl, Br bedeutet, unter Verwendung von R₇Li, wobei eine entsprechende Verbindung der Formel (I) erhalten wird, worin R₃ COR₇ darstellt, (ID), wobei R₇ definiert ist als C₁-C₆-Alkyl, C₂-C₆-Alkenyl oder Aryl, oder
g) Hydrolyse einer Verbindung der Formel (I), worin R₃ CN bedeutet, (IB), 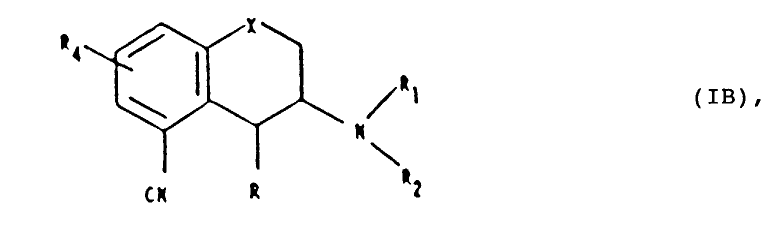 worin X, R, R₁, R₂ und R₄ wie gemäß Formel (I) definiert sind, gegebenenfalls gefolgt von einer Behandlung mit Thionylhalogenid, wobei eine Verbindung der Formel (I) erhalten wird, worin R₃ COZ darstellt, wobei Z OH, Cl oder Br ist, (IA), oder
h) Substitution einer Verbindung der Formel (I), worin R₃ die Bedeutung CN hat, 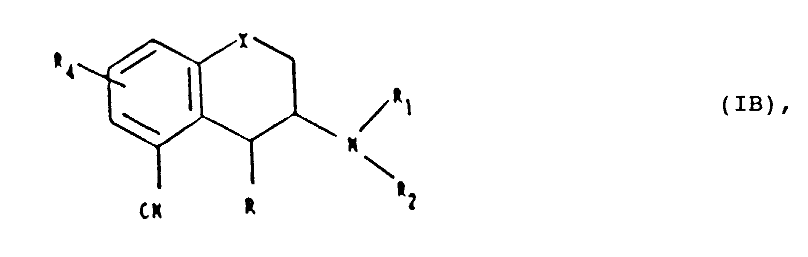 worin X, R, R₁, R₂ und R₄ wie gemäß Formel (I) definiert sind, durch Behandlung mit einem Organometall-Reagens, gefolgt von Hydrolyse, wobei eine Verbindung der Formel (I) erhalten wird, worin R₃ COR₇ darstellt, wobei R₇ C₁-C₆-Alkyl, C₂-C₆-Alkenyl oder Aryl darstellt, (ID), oder
i) Überführen einer Verbindung der Formel (II) 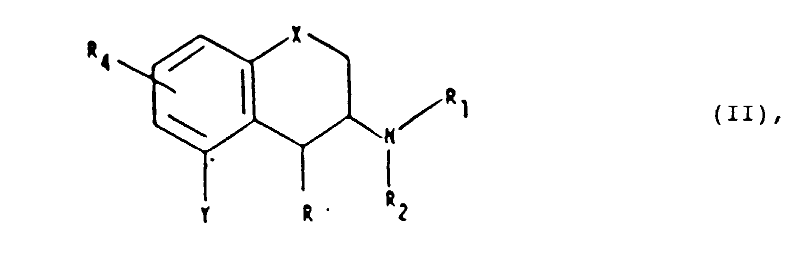 worin Y eine Abgangsgruppe ist, und X, R, R₁, R₂ und R₄ wie gemäß Formel (I) definiert sind, durch Umsetzen mit einem Übergangsmetall, wobei ein Liganden-Komplex gebildet wird, der eine oxidative Addition durch Behandlung mit einem Trialkylstannan oder Aryl-Borsäure-Reagentien eingeht, wobei eine Verbindung der Formel (I) gebildet wird, worin R₃ ein 5- bis 6-gliedriges Aryl bedeutet, das 1 oder 2 Heteroatome, ausgewählt aus N, O oder S, entweder substituiert oder an zwei benachbarten Kohlenstoffatomen kondensiert, enthalten kann;
worauf gegebenenfalls eine erhaltene Base in ein Säureadditionssalz übergeführt wird, oder ein erhaltenes Salz in die Base oder ein anderes Säureadditionssalz übergeführt wird, oder gegebenenfalls eine erhaltene isomere Mischung in ein reines Enantiomer getrennt wird.

25. Verbindung nach einem der Ansprüche 11 bis 14 zur Verwendung in der Therapie.

## Patentansprüche (Patentansprüche für folgende(n) Vertragsstaat(en): ES)

1. Verfahren zur Herstellung einer Verbindung der Formel 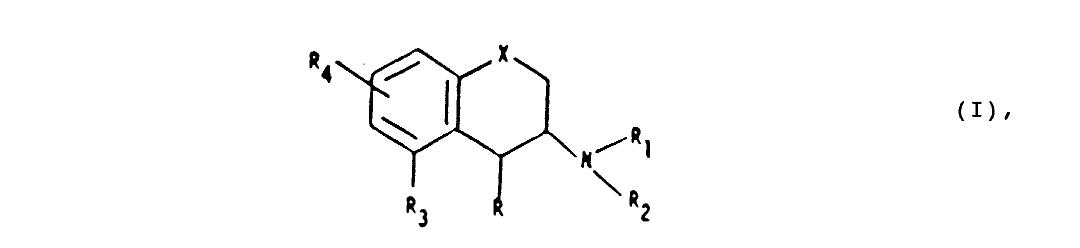 worin X die Bedeutung O oder 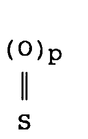 hat, p eine ganze Zahl Null, 1 oder 2 ist; R Wasserstoff, Fluor oder C₁-C₆-Alkyl bedeutet; R₁ Wasserstoff, C₁-C₆-Alkyl oder C₂-C₆-Alkenyl darstellt; R₂ bedeutet: Wasserstoff, C₁-C₆-Alkyl, C₂-C₆-Alkenyl, C₁-C₄-Alkylaryl, wobei Aryl 1 oder 2 Heteroatome, ausgewählt aus N, O oder S, gegebenenfalls substituiert durch Halogen, CN, CF₃, C₁-C₆-Alkyl, C₂-C₆-Alkenyl oder C₁-C₄-Alkoxy, enthalten kann; oder R₃ darstellt: CN, SO₃CF₃, N₃, NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₄ Wasserstoff oder Halogen ist; R₅ Wasserstoff, C₁-C₆-Alkyl oder C₂-C₆-Alkenyl bedeutet; R₆ C₁-C₆-Alkyl oder C₂-C₆-Alkenyl darstellt; oder R₅ und R₆ zusammen einen 5- oder 6-gliedrigen Ring bilden können, der 1 oder 2 Heteroatome, ausgewählt aus N, O oder S, enthalten kann; R₇ bedeutet: Wasserstoff, Hydroxy, Chlor, Brom, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; R₈ und R₉ jeweils unabhängig darstellen: Wasserstoff, C₁-C₆-Alkyl, C₂-C₆-Alkenyl, 5- oder 6-gliedriges Aryl, das 1 oder 2 Heteroatome, ausgewählt aus N, O oder S, gegebenenfalls substituiert durch Halogen, CN, CF₃, C₁-C₆-Alkyl, C₂-C₆-Alkenyl, C₁-C₄-Alkoxy, enthalten kann, oder zusammen einen 5- oder 6-gliedrigen Ring, der 1 oder 2 Heteroatome, ausgewählt aus N, O oder S, enthält, bilden können; eines Enantiomers oder eines Salzes hievon, durch
a) Überführen einer Verbindung der Formel (II) 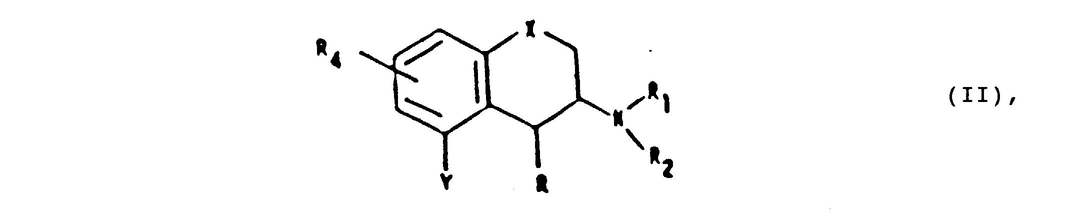 worin Y eine Abgangsgruppe ist, und X, R, R₁, R₂ und R₄ wie gemäß Formel (I) definiert sind, durch einen katalytischen Zyklus unter Verwendung eines nullwertigen Übergangsmetalls (M⁰), das eine oxidative Addition an die Aryl-Y-Bindungen eingeht, Behandlung mit Kohlenmonoxid, gefolgt von Z-H, wobei Z bedeutet: Cl, Br, OH, ORₚ, worin Rₚ C₁-C₆-Alkyl darstellt, und den anfänglich gebildeten carbonylierten o-Aryl-Metall-Y-Komplex zur Bildung einer Verbindung der Formel (I), worin R₃ COZ bedeutet, (IA), oder
b) Umsetzen durch einen katalytischen Zyklus unter Verwendung eines nullwertigen Übergangsmetalls (M⁰), das eine oxidative Addition an Z-Y eingeht, wobei Z definiert ist als Cl, Br, OH, ORₚ, worin Rₚ C₁-C₆-Alkyl darstellt, und Y eine Abgangsgruppe ist, Behandlung mit Kohlenmonoxid, gefolgt vom Zusatz der Verbindung der Formel (III) 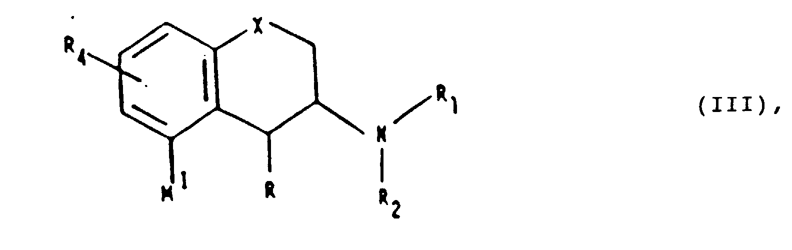 worin X, R, R₁, R₂ und R₄ wie gemäß Formel (I) definiert sind, und M^{I} ein Übergangsmetall bedeutet, zur Bildung einer Verbindung der Formel (I), worin R₃ COZ bedeutet, (IA), oder
c) Überführen einer Verbindung der Formel (II) 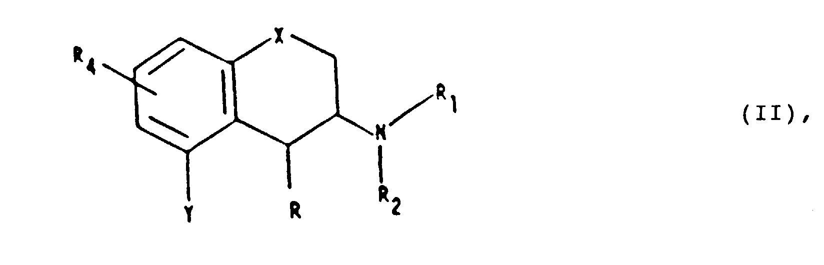 worin X, R, R₁, R₂ und R₄ wie gemäß Formel (I) definiert sind, und Y eine Abgangsgruppe, wie SO₃CF₃, Halogenid, ist, durch Behandlung mit einem Cyanid-Reagens zur Bildung einer Verbindung der Formel (I), worin R₃ CN bedeutet, (IB),
d) Aminieren einer Verbindung der Formel (IA) 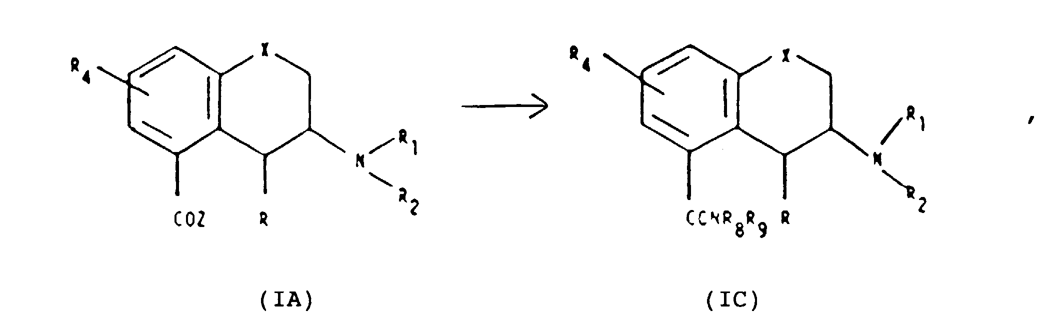 worin X, R, R₁, R₂ und R₄ wie gemäß Formel (I) definiert sind, und Z bedeutet: Cl, OH oder ORₚ, wobei Rₚ C₁-C₆-Alkyl darstellt, durch Umsetzen mit NHR₈R₉, wobei das entsprechende Amid der Formel (I) erhalten wird, worin R₃ CONR₈R₉ bedeutet, (IC), oder
e) Substitution eines 5-Bromchroman/Thiochroman-Derivats 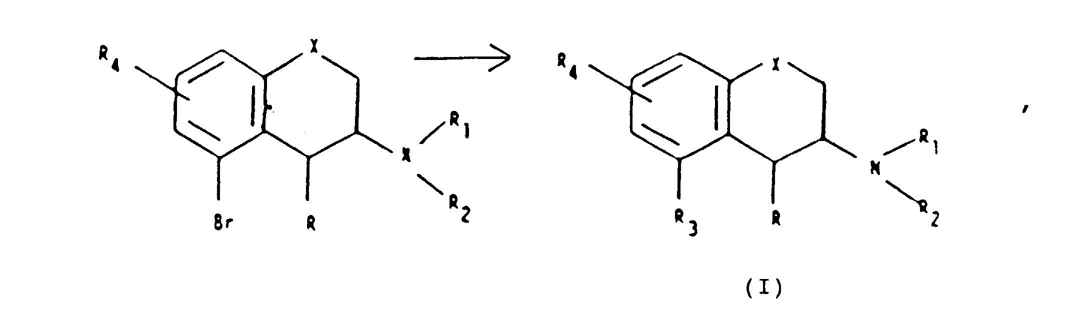 worin X, R, R₁, R₂ und R₄ wie gemäß Formel (I) definiert sind, durch Behandlung mit einem Zinn(IV)-trialkyl-Reagens in Anwesenheit eines nullwertigen Metalls, z.B. Pd⁰, wobei eine Verbindung der Formel (I) erhalten wird, worin R₃ Aryl bedeutet, oder in Anwesenheit von Kohlenmonoxid zur Bildung einer Verbindung, worin R₃ COR₇ bedeutet, wobei R₇ C₁-C₆-Alkyl, C₂-C₆-Alkenyl oder Aryl darstellt, oder
f) Überführen des 5-Carboxychroman/Thiochroman-Derivats der Formel (IA) 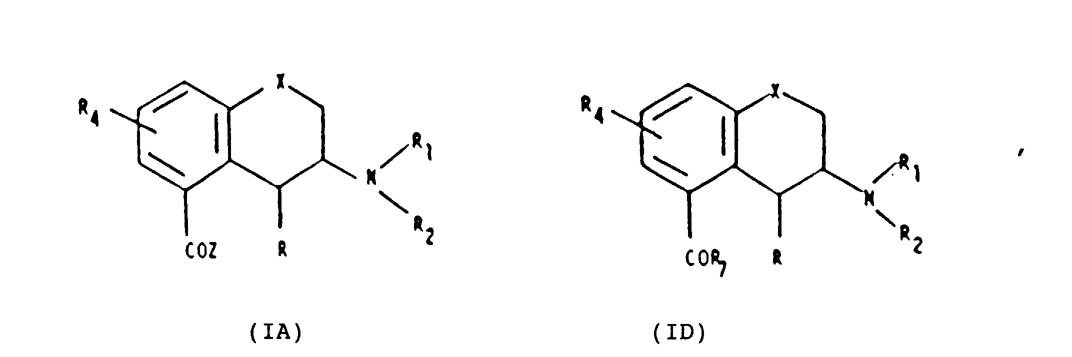 worin X, R, R₁, R₂ und R₄ wie gemäß Formel (I) definiert sind, und Z Cl, Br bedeutet, unter Verwendung von R₇Li, wobei eine entsprechende Verbindung der Formel (I) erhalten wird, worin R₃ COR₇ darstellt, (ID), wobei R₇ definiert ist als C₁-C₆-Alkyl, C₂-C₆-Alkenyl oder Aryl, oder
g) Hydrolyse einer Verbindung der Formel (I), worin R₃ CN bedeutet, (IB), 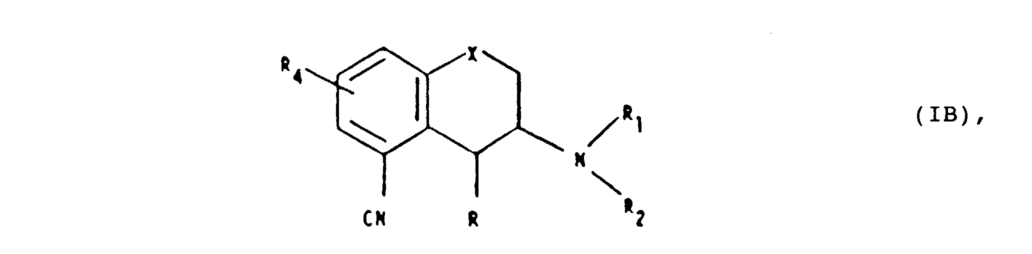 worin X, R, R₁, R₂ und R₄ wie gemäß Formel (I) definiert sind, gegebenenfalls gefolgt von einer Behandlung mit Thionylhalogenid, wobei eine Verbindung der Formel (I) erhalten wird, worin R₃ COZ darstellt, wobei Z OH, Cl oder Br ist, (IA), oder
h) Substitution einer Verbindung der Formel (I), worin R₃ die Bedeutung CN hat, 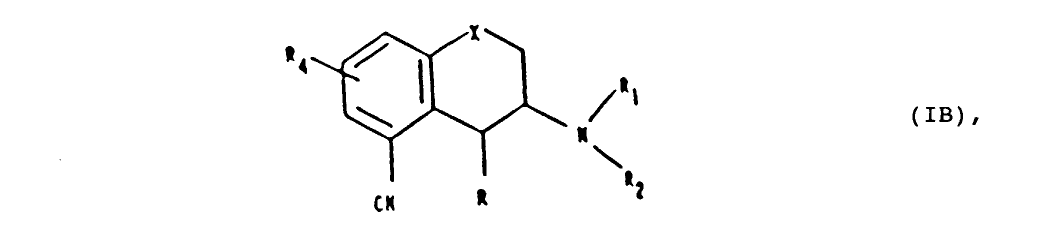 worin X, R, R₁, R₂ und R₄ wie gemäß Formel (I) definiert sind, durch Behandlung mit einem Organometall-Reagens, gefolgt von Hydrolyse, wobei eine Verbindung der Formel (I) erhalten wird, worin R₃ COR₇ darstellt, wobei R₇ C₁-C₆-Alkyl, C₂-C₆-Alkenyl oder Aryl darstellt, (ID), oder
i) Überführen einer Verbindung der Formel (II) 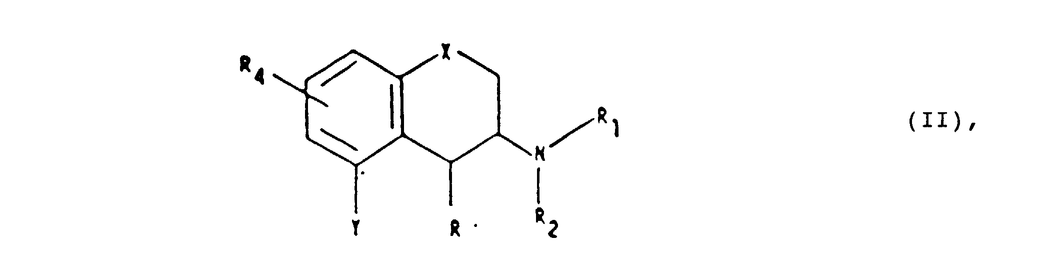 worin Y eine Abgangsgruppe ist, und X, R, R₁, R₂ und R₄ wie gemäß Formel (I) definiert sind, durch Umsetzen mit einem Übergangsmetall, wobei ein Liganden-Komplex gebildet wird, der eine oxidative Addition durch Behandlung mit einem Trialkylstannan oder Aryl-Borsäure-Reagentien eingeht, wobei eine Verbindung der Formel (I) gebildet wird, worin ein R₃ 5- bis 6-gliedriges Aryl bedeutet, das 1 oder 2 Heteroatome, ausgewählt aus N, O oder S, entweder substituiert oder an zwei benachbarten Kohlenstoffatomen kondensiert, enthalten kann;
worauf gegebenenfalls eine erhaltene Base in ein Säureadditionssalz übergeführt wird, oder ein erhaltenes Salz in die Base oder ein anderes Säureadditionssalz übergeführt wird, oder gegebenenfalls eine erhaltene isomere Mischung in ein reines Enantiomer getrennt wird.

2. Verfahren nach Anspruch 1 zur Herstellung einer Verbindung nach Anspruch 1, worin X die Bedeutung O hat.

3. Verfahren nach Anspruch 1 zur Herstellung einer Verbindung nach Anspruch 1, worin X die Bedeutung 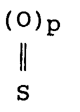 hat, und p Null, 1 oder 2 ist.

4. Verfahren nach Anspruch 1 zur Herstellung einer Verbindung nach einem der Ansprüche 1, 2 oder 3, worin R₃ bedeutet: NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₇ darstellt: Wasserstoff, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; X, p, R, R₁, R₂, R₄, R₅, R₆, R₈ und R₉ wie in Anspruch 1 definiert sind; eines Enantiomers oder eines pharmazeutisch annehmbaren Salzes hievon zur Verwendung in der Therapie.

5. Verfahren nach Anspruch 1 zur Herstellung einer Verbindung nach einem der Ansprüche 1 bis 4, worin R₁ und R₂ gleich oder verschieden und aus Wasserstoff, n-Propyl, i-Propyl und Cyclopropyl ausgewählt sind.

6. Verfahren nach Anspruch 1 zur Herstellung einer Verbindung nach einem der Ansprüche 1 bis 5, worin R₃ die Bedeutung COR₇ hat.

7. Verfahren nach Anspruch 1 zur Herstellung einer Verbindung nach Anspruch 6, worin R₇ C₁-C₄-Alkyl, Phenyl, Furanyl oder Thienyl, gegebenenfalls substituiert mit Halogen, NR₈R₉ darstellt, wobei R₈ und R₉ jeweils unabhängig Wasserstoff oder C₁-C₄-Alkyl oder C₁-C₄-Alkoxy bedeuten.

8. Verfahren nach Anspruch 1 zur Herstellung einer Verbindung nach Anspruch 6, worin R und R₄ Wasserstoff sind, R₁ und R₂ n-Propyl bedeuten, und R₇ Methyl, Ethyl, n-Propyl, i-Propyl, Cyclopropyl, n-Butyl, i-Butyl, tert.Butyl, Cyclobutyl, Thienyl, Furanyl, Phenyl, N-Methylamino, Methoxy oder Fluorphenyl darstellt.

9. Verfahren nach Anspruch 1 zur Herstellung einer Verbindung nach einem der Ansprüche 1 bis 5, worin R₃ Phenyl, Furanyl, Thienyl oder Fluorphenyl bedeutet.

10. Verfahren nach Anspruch 1 zur Herstellung einer Verbindung nach einem der Ansprüche 1 bis 7 und 9, worin R₄ Halogen in Stellung 8 darstellt.

11. Verfahren nach Anspruch 1 zur Herstellung von 3-Dipropylamino-5-acetylchroman, eines Enantiomers oder eines Salzes hievon.

12. Verfahren nach Anspruch 1 zur Herstellung von 3-Dipropylamino-5-carbamoylchroman, eines Enantiomers oder eines Salzes hievon.

13. Verfahren nach Anspruch 1 zur Herstellung von 3-Dipropylamino-5-N-methylcarbamoylchroman, eines Enantiomers oder eines Salzes hievon.

14. Verfahren nach Anspruch 1 zur Herstellung von 3-Dipropylamino-5-(2-thienylcarbonyl)-chroman, eines Enantiomers oder eines Salzes hievon.

15. Verfahren nach Anspruch 1 zur Herstellung einer Verbindung nach Anspruch 1, worin R₃ CN, COOH, COCl, COBr, N₃ oder SO₃CF₃ bedeutet, und X, R, R₁, R₂ und R₄ wie in Anspruch 1 definiert sind, eines Enantiomers oder eines Salzes hievon.

16. Verfahren nach Anspruch 1 zur Herstellung einer Verbindung nach Anspruch 15, worin R₁ und R₂ gleich oder verschieden und aus Wasserstoff, n-Propyl, i-Propyl und Cyclopropyl ausgewählt sind.

17. Verfahren nach Anspruch 1 zur Herstellung einer Verbindung nach Anspruch 16, worin R und R₄ Wasserstoff sind, R₁ und R₂ n-Propyl bedeuten, und R₃ SO₃CF₃ darstellt.

18. Verwendung einer Verbindung nach einem der Ansprüche 4 bis 14 bei der Herstellung eines Medikaments zur Behandlung von Störungen des Zentralnervensystems, insbesondere 5-Hydroxytryptamin-vermittelten Störungen.

19. Verwendung nach Anspruch 18 bei der Herstellung eines Medikaments zur Behandlung von Depressionen, Anxietas, Anorexie, Altersdemenz, Migräne, Alzheimer-Krankheit, thermoregulatorischen und sexuellen Störungen, Schmerzen und Störungen des kardiovaskulären Systems.

## Patentansprüche (Patentansprüche für folgende(n) Vertragsstaat(en): GR)

1. Verfahren zur Herstellung einer Verbindung der Formel 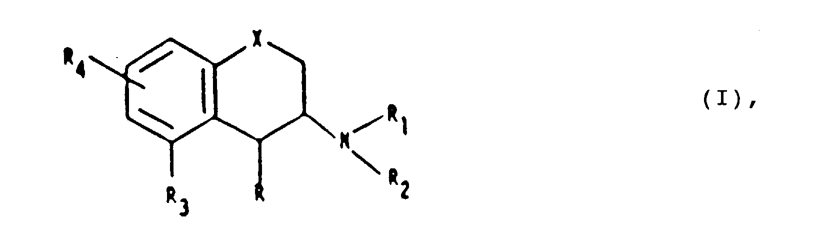 worin X die Bedeutung O oder 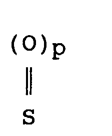 hat, p eine ganze Zahl Null, 1 oder 2 ist; R Wasserstoff, Fluor oder C₁-C₆-Alkyl bedeutet; R₁ Wasserstoff, C₁-C₆-Alkyl oder C₂-C₆-Alkenyl darstellt; R₂ bedeutet: Wasserstoff, C₁-C₆-Alkyl, C₂-C₆-Alkenyl, C₁-C₄-Alkylaryl, wobei Aryl 1 oder 2 Heteroatome, ausgewählt aus N, O oder S, gegebenenfalls substituiert durch Halogen, CN, CF₃, C₁-C₆-Alkyl, C₂-C₆-Alkenyl oder C₁-C₄-Alkoxy, enthalten kann; oder R₃ darstellt: CN, SO₃CF₃, N₃, NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₄ Wasserstoff oder Halogen ist; R₅ Wasserstoff, C₁-C₆-Alkyl oder C₂-C₆-Alkenyl bedeutet; R₆ C₁-C₆-Alkyl oder C₂-C₆-Alkenyl darstellt; oder R₅ und R₆ zusammen einen 5- oder 6-gliedrigen Ring bilden können, der 1 oder 2 Heteroatome, ausgewählt aus N, O oder S, enthalten kann; R₇ bedeutet: Wasserstoff, Hydroxy, Chlor, Brom, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; R₈ und R₉ jeweils unabhängig darstellen: Wasserstoff, C₁-C₆-Alkyl, C₂-C₆-Alkenyl, 5- oder 6-gliedriges Aryl, das 1 oder 2 Heteroatome, ausgewählt aus N, O oder S, gegebenenfalls substituiert durch Halogen, CN, CF₃, C₁-C₆-Alkyl, C₂-C₆-Alkenyl, C₁-C₄-Alkoxy, enthalten kann, oder zusammen einen 5- oder 6-gliedrigen Ring, der 1 oder 2 Heteroatome, ausgewählt aus N, O oder S, enthält, bilden können; eines Enantiomers oder eines Salzes hievon, durch
a) Überführen einer Verbindung der Formel (II) 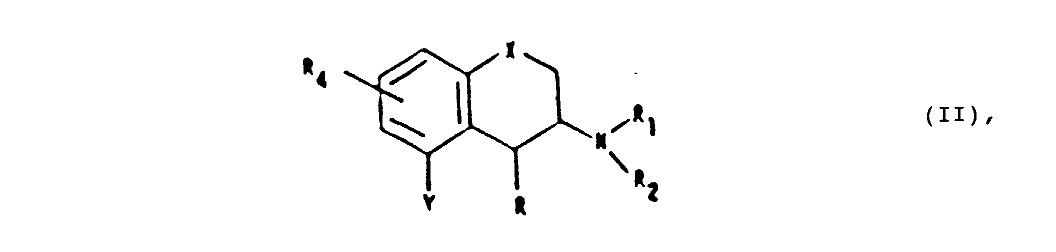 worin Y eine Abgangsgruppe ist, und X, R, R₁, R₂ und R₄ wie gemäß Formel (I) definiert sind, durch einen katalytischen Zyklus unter Verwendung eines nullwertigen Übergangsmetalls (M⁰), das eine oxidative Addition an die Aryl-Y-Bindungen eingeht, Behandlung mit Kohlenmonoxid, gefolgt von Z-H, wobei Z bedeutet: Cl, Br, OH, ORₚ, worin Rₚ C₁-C₆-Alkyl darstellt, und den anfänglich gebildeten carbonylierten o-Aryl-Metall-Y-Komplex zur Bildung einer Verbindung der Formel (I), worin R₃ COZ bedeutet, (IA), oder
b) Umsetzen durch einen katalytischen Zyklus unter Verwendung eines nullwertigen Übergangsmetalls (M⁰), das eine oxidative Addition an Z-Y eingeht, wobei Z definiert ist als Cl, Br, OH, ORₚ, worin Rₚ C₁-C₆-Alkyl darstellt, und Y eine Abgangsgruppe ist, Behandlung mit Kohlenmonoxid, gefolgt vom Zusatz der Verbindung der Formel (III) 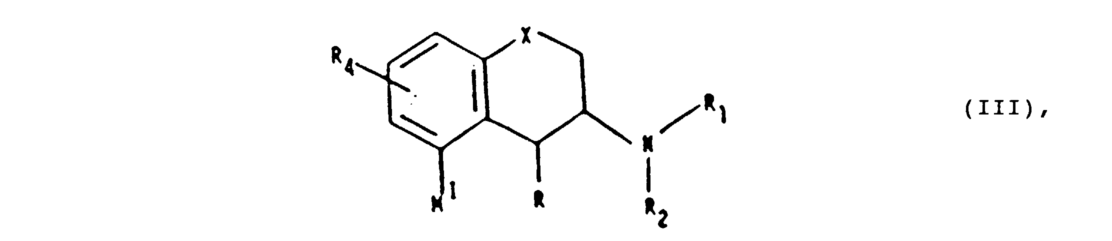 worin X, R, R₁, R₂ und R₄ wie gemäß Formel (I) definiert sind, und M^{I} ein Übergangsmetall bedeutet, zur Bildung einer Verbindung der Formel (I), worin R₃ COZ bedeutet, (IA), oder
c) Überführen einer Verbindung der Formel (II) 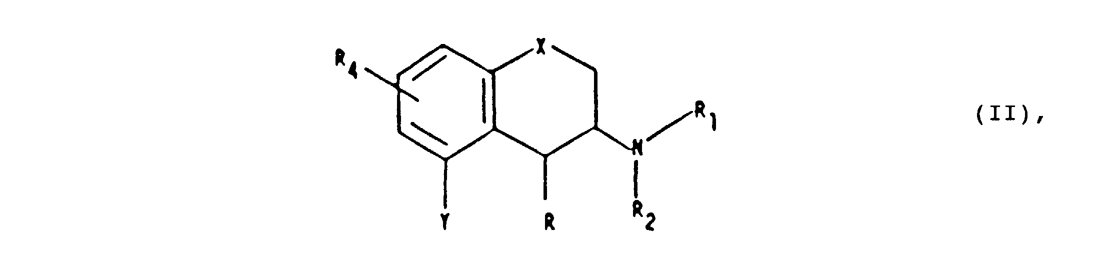 worin X, R, R₁, R₂ und R₄ wie gemäß Formel (I) definiert sind, und Y eine Abgangsgruppe, wie SO₃CF₃, Halogenid, ist, durch Behandlung mit einem Cyanid-Reagens zur Bildung einer Verbindung der Formel (I), worin R₃ CN bedeutet, (IB),
d) Aminieren einer Verbindung der Formel (IA) 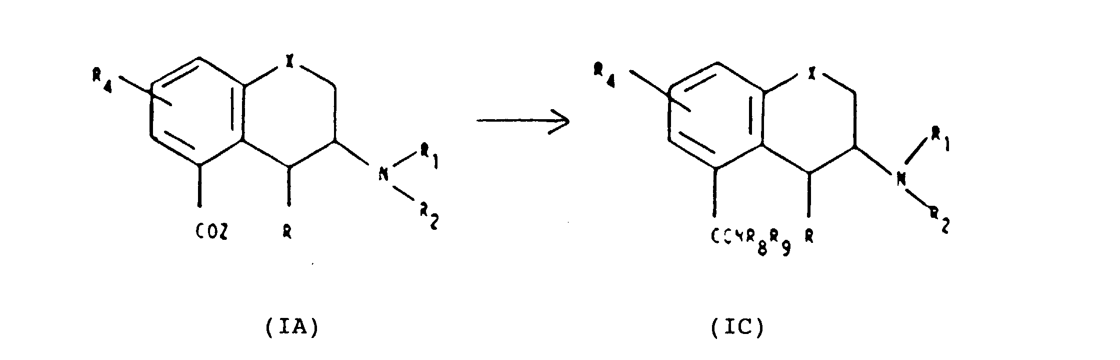 worin X, R, R₁, R₂ und R₄ wie gemäß Formel (I) definiert sind, und Z bedeutet: Cl, OH oder ORₚ, wobei Rₚ C₁-C₆-Alkyl darstellt, durch Umsetzen mit NHR₈R₉, wobei das entsprechende Amid der Formel (I) erhalten wird, worin R₃ CONR₈R₉ bedeutet, (IC), oder
e) Substitution eines 5-Bromchroman/Thiochroman-Derivats 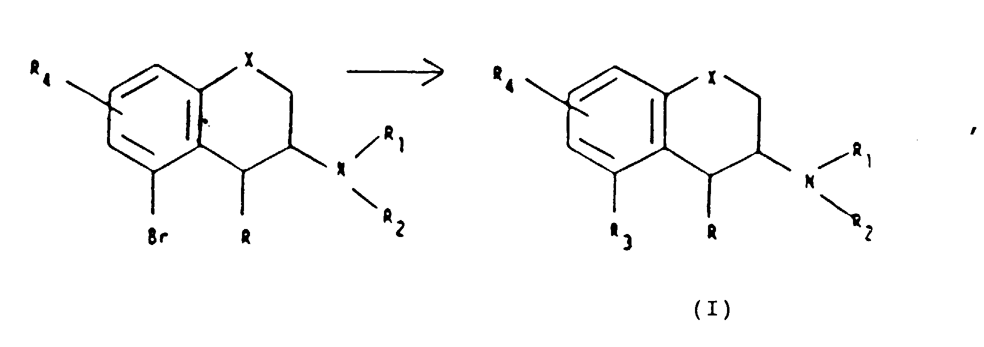 worin X, R, R₁, R₂ und R₄ wie gemäß Formel (I) definiert sind, durch Behandlung mit einem Zinn(IV)-trialkyl-Reagens in Anwesenheit eines nullwertigen Metalls, z.B. Pd⁰, wobei eine Verbindung der Formel (I) erhalten wird, worin R₃ Aryl bedeutet, oder in Anwesenheit von Kohlenmonoxid zur Bildung einer Verbindung, worin R₃ COR₇ bedeutet, wobei R₇ C₁-C₆-Alkyl, C₂-C₆-Alkenyl oder Aryl darstellt, oder
f) Überführen des 5-Carboxychroman/Thiochroman-Derivats der Formel (IA) 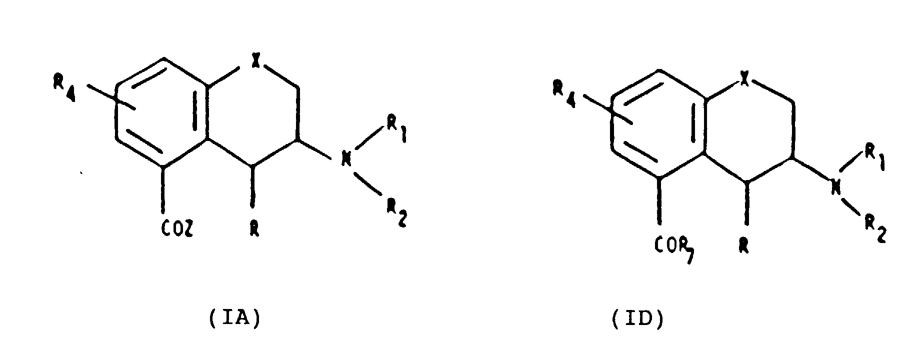 worin X, R, R₁, R₂ und R₄ wie gemäß Formel (I) definiert sind, und Z Cl, Br bedeutet, unter Verwendung von R₇Li, wobei eine entsprechende Verbindung der Formel (I) erhalten wird, worin R₃ COR₇ darstellt, (ID), wobei R₇ definiert ist als C₁-C₆-Alkyl, C₂-C₆-Alkenyl oder Aryl, oder
g) Hydrolyse einer Verbindung der Formel (I), worin R₃ CN bedeutet, (IB), 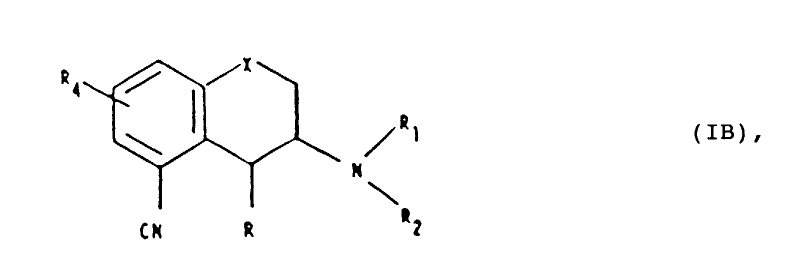 worin X, R, R₁, R₂ und R₄ wie gemäß Formel (I) definiert sind, gegebenenfalls gefolgt von einer Behandlung mit Thionylhalogenid, wobei eine Verbindung der Formel (I) erhalten wird, worin R₃ COZ darstellt, wobei Z OH, Cl oder Br ist, (IA), oder
h) Substitution einer Verbindung der Formel (I), worin R₃ die Bedeutung CN hat, 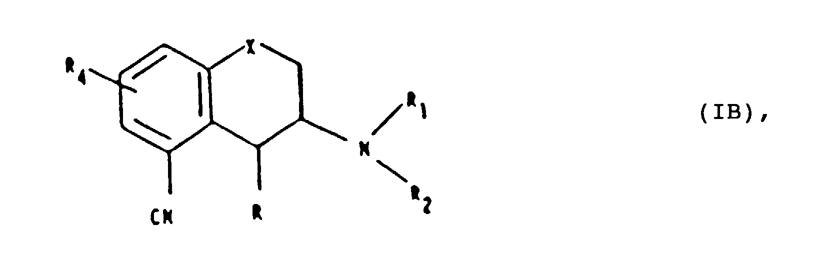 worin X, R, R₁, R₂ und R₄ wie gemäß Formel (I) definiert sind, durch Behandlung mit einem Organometall-Reagens, gefolgt von Hydrolyse, wobei eine Verbindung der Formel (I) erhalten wird, worin R₃ COR₇ darstellt, wobei R₇ C₁-C₆-Alkyl, C₂-C₆-Alkenyl oder Aryl darstellt, (ID), oder
i) Überführen einer Verbindung der Formel (II) 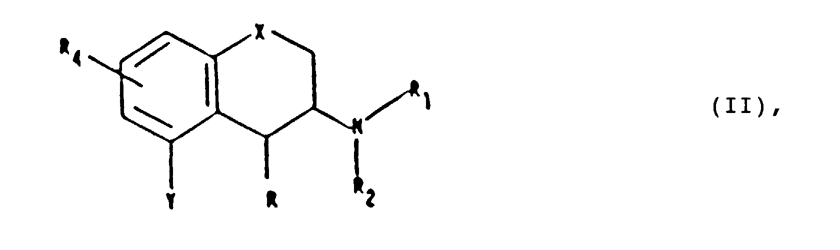 worin Y eine Abgangsgruppe ist, und X, R, R₁, R₂ und R₄ wie gemäß Formel (I) definiert sind, durch Umsetzen mit einem Übergangsmetall, wobei ein Liganden-Komplex gebildet wird, der eine oxidative Addition durch Behandlung mit einem Trialkylstannan oder Aryl-Borsäure-Reagentien eingeht, wobei eine Verbindung der Formel (I) gebildet wird, worin ein R₃ 5- bis 6-gliedriges Aryl bedeutet, das 1 oder 2 Heteroatome, ausgewählt aus N, O oder S, entweder substituiert oder an zwei benachbarten Kohlenstoffatomen kondensiert, enthalten kann;
worauf gegebenenfalls eine erhaltene Base in ein Säureadditionssalz übergeführt wird, oder ein erhaltenes Salz in die Base oder ein anderes Säureadditionssalz übergeführt wird, oder gegebenenfalls eine erhaltene isomere Mischung in ein reines Enantiomer getrennt wird.

2. Verfahren nach Anspruch 1 zur Herstellung einer Verbindung nach Anspruch 1, worin X die Bedeutung O hat.

3. Verfahren nach Anspruch 1 zur Herstellung einer Verbindung nach Anspruch 1, worin X die Bedeutung 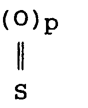 hat, und p Null, 1 oder 2 ist.

4. Verfahren nach Anspruch 1 zur Herstellung einer Verbindung nach einem der Ansprüche 1, 2 oder 3, worin R₃ bedeutet: NR₅R₆, COR₇, 5- oder 6-gliedriges Aryl, das 1 oder 2 Heteroatome, ausgewählt aus N, O oder S, enthalten kann, und entweder (i) gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy, oder auch (ii) an zwei benachbarten Kohlenstoffatomen an einen Aryl-Ring kondensiert ist, welcher Aryl-Ring gegebenenfalls substituiert ist durch einen oder mehrere Substituenten, unabhängig ausgewählt aus Halogen, CN, CF₃, C₁-C₆-Alkyl, C₂-C₆-Alkenyl oder C₁-C₄-Alkoxy; R₇ darstellt: Wasserstoff, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; X, p, R, R₁, R₂, R₄, R₅, R₆, R₈ und R₉ wie in Anspruch 1 definiert sind; eines Enantiomers oder eines pharmazeutisch annehmbaren Salzes hievon zur Verwendung in der Therapie.

5. Verfahren nach Anspruch 1 zur Herstellung einer Verbindung nach einem der Ansprüche 1 bis 4, worin R₁ und R₂ gleich oder verschieden und aus Wasserstoff, n-Propyl, i-Propyl und Cyclopropyl ausgewählt sind.

6. Verfahren nach Anspruch 1 zur Herstellung einer Verbindung nach einem der Ansprüche 1 bis 5, worin R₃ die Bedeutung COR₇ hat.

7. Verfahren nach Anspruch 1 zur Herstellung einer Verbindung nach Anspruch 6, worin R₇ C₁-C₄-Alkyl, Phenyl, Furanyl oder Thienyl, gegebenenfalls substituiert mit Halogen, NR₈R₉ darstellt, wobei R₈ und R₉ jeweils unabhängig Wasserstoff oder C₁-C₄-Alkyl oder C₁-C₄-Alkoxy bedeuten.

8. Verfahren nach Anspruch 1 zur Herstellung einer Verbindung nach Anspruch 6, worin R und R₄ Wasserstoff sind, R₁ und R₂ n-Propyl bedeuten, und R₇ Methyl, Ethyl, n-Propyl, i-Propyl, Cyclopropyl, n-Butyl, i-Butyl, tert.Butyl, Cyclobutyl, Thienyl, Furanyl, Phenyl, N-Methylamino, Methoxy oder Fluorphenyl darstellt.

9. Verfahren nach Anspruch 1 zur Herstellung einer Verbindung nach einem der Ansprüche 1 bis 5, worin R₃ Phenyl, Furanyl, Thienyl oder Fluorphenyl bedeutet.

10. Verfahren nach Anspruch 1 zur Herstellung einer Verbindung nach einem der Ansprüche 1 bis 7 und 9, worin R₄ Halogen in Stellung 8 darstellt.

11. Verfahren nach Anspruch 1 zur Herstellung von 3-Dipropylamino-5-acetylchroman, eines Enantiomers oder eines Salzes hievon.

12. Verfahren nach Anspruch 1 zur Herstellung von 3-Dipropylamino-5-carbamoylchroman, eines Enantiomers oder eines Salzes hievon.

13. Verfahren nach Anspruch 1 zur Herstellung von 3-Dipropylamino-5-N-methylcarbamoylchroman, eines Enantiomers oder eines Salzes hievon.

14. Verfahren nach Anspruch 1 zur Herstellung von 3-Dipropylamino-5-(2-thienylcarbonyl)-chroman, eines Enantiomers oder eines Salzes hievon.

15. Verwendung einer Verbindung nach einem der Ansprüche 4 bis 14 bei der Herstellung eines Medikaments zur Behandlung von Störungen des Zentralnervensystems, insbesondere 5-Hydroxytryptamin-vermittelten Störungen.

16. Verwendung nach Anspruch 15 bei der Herstellung eines Medikaments zur Behandlung von Depressionen, Anxietas, Anorexie, Altersdemenz, Migräne, Alzheimer-Krankheit, thermoregulatorischen und sexuellen Störungen, Schmerzen und Störungen des kardiovaskulären Systems.

17. Verbindung der Formel 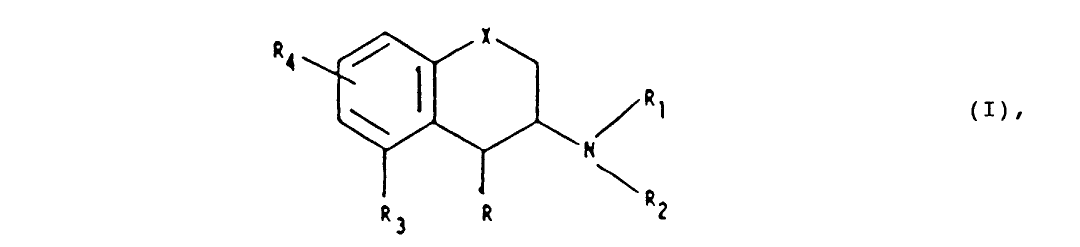 worin X die Bedeutung O oder 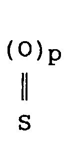 hat, p eine ganze Zahl Null, 1 oder 2 ist; R Wasserstoff, Fluor oder C₁-C₆-Alkyl bedeutet; R₁ Wasserstoff, C₁-C₆-Alkyl oder C₂-C₆-Alkenyl darstellt; R₂ bedeutet: Wasserstoff, C₁-C₆-Alkyl, C₂-C₆-Alkenyl, C₁-C₄-Alkylaryl, wobei Aryl 1 oder 2 Heteroatome, ausgewählt aus N, O oder S, gegebenenfalls substituiert durch Halogen, CN, CF₃, C₁-C₆-Alkyl, C₂-C₆-Alkenyl oder C₁-C₄-Alkoxy, enthalten kann; oder R₃ darstellt: CN, COOH, COCl, COBr, N₃ oder SO₃CF₃; R₄ Wasserstoff oder Halogen ist; R₅ Wasserstoff, C₁-C₆-Alkyl oder C₂-C₆-Alkenyl bedeutet; R₆ C₁-C₆-Alkyl oder C₂-C₆-Alkenyl darstellt; oder R₅ und R₆ zusammen einen 5- oder 6-gliedrigen Ring bilden können, der 1 oder 2 Heteroatome, ausgewählt aus N, O oder S, enthalten kann; R₇ bedeutet: Wasserstoff, Hydroxy, Chlor, Brom, C₁-C₆-Alkyl, C₂-C₆-Alkenyl, C₁-C₄-Alkoxy, NR₈R₉ oder 5- oder 6-gliedriges Aryl, das 1 oder 2 Heteroatome, ausgewählt aus N, O oder S, gegebenenfalls substituiert durch eines oder mehrere von Halogen, CN, CF₃, C₁-C₆-Alkyl, C₂-C₆-Alkenyl oder C₁-C₄-Alkoxy, enthalten kann; R₈ und R₉ jeweils unabhängig darstellen: Wasserstoff, C₁-C₆-Alkyl, C₂-C₆-Alkenyl, 5- oder 6-gliedriges Aryl, das 1 oder 2 Heteroatome, ausgewählt aus N, O oder S, gegebenenfalls substituiert durch Halogen, CN, CF₃, C₁-C₆-Alkyl, C₂-C₆-Alkenyl, C₁-C₄-Alkoxy, enthalten kann, oder zusammen einen 5- oder 6-gliedrigen Ring, der 1 oder 2 Heteroatome, ausgewählt aus N, O oder S, enthält, bilden können; ein Enantiomer oder ein Salz hievon.

18. Verbindung nach Anspruch 17, worin R₁ und R₂ gleich oder verschieden und aus Wasserstoff, n-Propyl, i-Propyl und Cyclopropyl ausgewählt sind.

19. Verbindung nach Anspruch 18, worin R und R₄ Wasserstoff sind, R₁ und R₂ n-Propyl bedeuten, und R₃ SO₃CF₃ darstellt.

## Revendications (Revendications pour l'(les) Etat(s) contractant(s) suivant(s): AT, BE, CH, DE, DK, FR, GB, IT, LI, LU, NL, SE)

1. Composé de formule 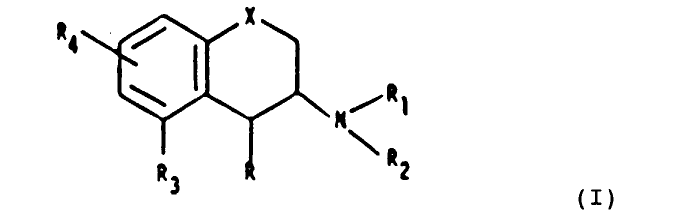 dans laquelle
X est O ou 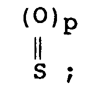
p est un nombre entier égal à 0, 1 ou 2;
R est un atome d'hydrogène, de fluor ou un groupe alkyle en C₁-C₆;
R₁ est un atome d'hydrogène, un groupe alkyle en C₁-C₆ ou alcényle en C₂-C₆;
R₂ est un atome d'hydrogène, un groupe alkyle en C₁-C₆, alcényle en C₂-C₆, alkylaryle en C₁-C₄, où le groupe aryle peut renfermer 1 ou 2 hétéroatomes choisis parmi N, O ou S, éventuellement substitué par un halogène,un groupe CN, CF₃, alkyle en C₁-C₆, alcényle en C₂-C₆ ou alcoxy en C₁-C₄; ou
R₃ est CN, SO₃CF₃, N₃, NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄, soit (ii) fusionné au niveau de deux atomes de carbone adjacents à un cycle aryle, ledit cycle aryle étant éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄;
R₄ est un atome d'hydrogène ou d'halogène;
R₅ est un atome d'hydrogène, un groupe alkyle en C₁-C₆ ou alcényle en C₂-C₆;
R₆ est un groupe alkyle en C₁-C₆ ou alcényle en C₂-C₆; ou
R₅ et R₆ peuvent former ensemble un cycle à 5 ou 6 chaînons qui peut renfermer 1 ou 2 hétéroatomes choisis parmi N, O ou S;
R₇ est un atome d'hydrogène, un hydroxy, un atome de chlore, de brome, un groupe alkyle en C₁-C₆, alcényle en C₂-C₆, alcoxy en C₁-C₄, NR₈R₉ ou un groupe aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
R₈ et R₉ représentent chacun indépendamment un atome d'hydrogène, un groupe alkyle en C₁-C₆, alcényle en C₂-C₆, aryle à 5 ou 6 chaînons qui peut renfermer 1 ou 2 hétéroatomes choisis parmi N, O ou S, éventuellement substitué par un atome d'halogène, un groupe CN, CF₃, alkyle en C₁-C₆, alcényle en C₂-C₆, alcoxy en C₁-C₄, ou peuvent former ensemble un cycle à 5 ou 6 chaînons renfermant 1 ou 2 hétéroatomes choisis parmi N, O ou S;
un énantiomère ou un sel de celui-ci.

2. Composé selon la revendication 1, dans lequel X est O.

3. Composé selon la revendication 1, dans lequel X est 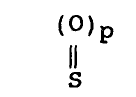 et p est égal à 0, 1 ou 2.

4. Composé selon l'une quelconque des revendications 1, 2 ou 3, dans lequel R₃ est NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants indépendamment choisis parmi les halogènes, les groupes CN, CF₃, alkyle en C₁-C₆, alcényle en C₂-C₆ ou alcoxy en C₁-C₄, soit (ii) fusionné au niveau de deux atomes de carbone adjacents à un cycle aryle, ledit cycle aryle étant éventuellement substitué par un ou plusieurs substituants indépendamment choisis parmi les halogènes, les groupes CN, CF₃, alkyle en C₁-C₆, alcényle en C₂-C₆ ou alcoxy en C₁-C₄; R₇ est un atome d'hydrogène, un groupe alkyle en C₁-C₆, alcényle en C₂-C₆, alcoxy en C₁-C₄, NR₈R₉ ou aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄; X, p, R, R₁, R₂, R₄, R₅, R₆, R₈ et R₉ sont tels que définis dans la revendication 1, un énantiomère ou un sel pharmaceutiquement acceptable de celui-ci, à utiliser en thérapie.

5. Composé selon l'une quelconque des revendications 1-4, dans lequel R₁ et R₂ sont identiques ou différents et sont choisis parmi l'hydrogène, le n-propyle, l'i-propyle et le cyclopropyle.

6. Composé selon l'une quelconque des revendications 1-5, dans lequel R₃ est COR₇.

7. Composé selon la revendication 6, dans lequel R₇ est un groupe alkyle en C₁-C₄, phényle, furanyle ou thiényle éventuellement substitué par un halogène, NR₈R₉ où R₈ et R₉ sont chacun indépendamment un atome d'hydrogène ou un groupe alkyle en C₁-C₄, ou alcoxy en C₁-C₄.

8. Composé selon la revendication 6, dans lequel R et R₄ sont des atomes d'hydrogène, R₁ et R₂ sont des groupes n-propyle et R₇ est un groupe méthyle, éthyle, n-propyle, i-propyle, cyclopropyle, n-butyle, i-butyle, t-butyle, cyclobutyle, thiényle, furanyle, phényle, N-méthylamino, méthoxy ou fluorophényle.

9. Composé selon l'une quelconque des revendications 1-5, dans lequel R₃ est un groupe phényle, furanyle, thiényle ou fluorophényle.

10. Composé selon l'une quelconque des revendications 1-7 et 9, dans lequel R₄ est un atome d'halogène en position 8.

11. Composé selon la revendication 1, qui est le 3-dipropylamino-5-acétylchroman, un énantiomère ou un sel de celui-ci.

12. Composé selon la revendication 1, qui est le 3-dipropylamino-5-carbamoylchroman, un énantiomère ou un sel de celui-ci.

13. Composé selon la revendication 1, qui est le 3-dipropylamino-5-N-méthylcarbamoylchroman, un énantiomère ou un sel de celui-ci.

14. Composé selon la revendication 1, qui est le 3-dipropylamino-5-(2-thiénylcarbonyl)chroman, un énantiomère ou un sel de celui-ci.

15. Composé selon la revendication 1, dans lequel R₃ est CN, COOH, COCl, COBr, N₃ ou SO₃CF₃, et X, R, R₁, R₂ et R₄ sont tels que définis dans la revendication 1, un énantiomère ou un sel de celui-ci.

16. Composé selon la revendication 15, dans lequel R₁ et R₂ sont identiques ou différents et sont choisis parmi l'hydrogène, le n-propyle, l'i-propyle et le cyclopropyle.

17. Composé selon la revendication 16, dans lequel R et R₄ sont des atomes d'hydrogène, R₁ et R₂ sont des n-propyle et R₃ est SO₃CF₃.

18. Préparation pharmaceutique contenant, comme principe actif, un composé selon la formule I 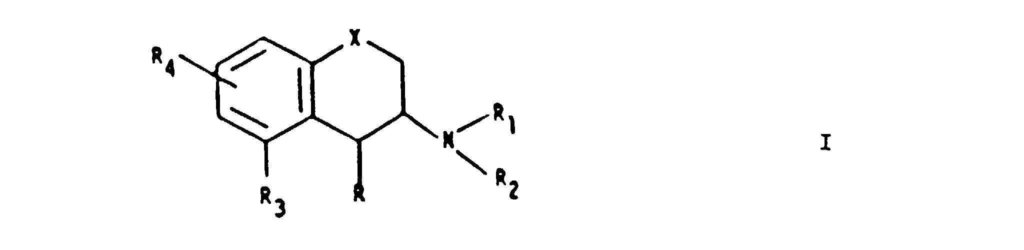 dans laquelle
X est O ou 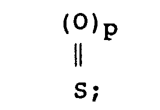
p est un nombre entier égal à 0, 1 ou 2;
R est un atome d'hydrogène, de fluor ou un groupe alkyle en C₁-C₆;
R₁ est un atome d'hydrogène, un groupe alkyle en C₁-C₆ ou alcényle en C₂-C₆;
R₂ est un atome d'hydrogène, un groupe alkyle en C₁-C₆, alcényle en C₂-C₆, alkylaryl en C₁-C₄, où le groupe aryle peut renfermer 1 ou 2 hétéroatomes choisis parmi N, O ou S, éventuellement substitué par un halogène, un groupe CN, CF₃, alkyle en C₁-C₆, alcényle en C₂-C₆ ou alcoxy en C₁-C₄; ou
R₃ est NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄ soit (ii) fusionné au niveau de deux atomes de carbone adjacents à un cycle aryle, ledit cycle aryle étant éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄;
R₄ est un atome d'hydrogène ou d'halogène;
R₅ est un atome d'hydrogène, un groupe alkyle en C₁-C₆ ou alcényle en C₂-C₆;
R₆ est un groupe alkyle en C₁-C₆ ou alcényle en C₂-C₆; ou
R₅ et R₆ peuvent former ensemble un cycle à 5 ou 6 chaînons qui peut renfermer 1 ou 2 hétéroatomes choisis parmi N, O ou S;
R₇ est un atome d'hydrogène, un groupe alkyle en C₁-C₆, alcényle en C₂-C₆, alcoxy en C₁-C₄, NR₈R₉ ou aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
R₈ et R₉ représentent chacun indépendamment un atome d'hydrogène, un groupe alkyle en C₁-C₆, alcényle en C₂-C₆, aryle à 5 ou 6 chaînons qui peut renfermer 1 ou 2 hétéroatomes choisis parmi N, O ou S, éventuellement substitué par un atome d'halogène, un groupe CN, CF₃, alkyle en C₁-C₆, alcényle en C₂-C₆, alcoxy en C₁-C₄, ou peuvent former ensemble un cycle à 5 ou 6 chaînons renfermant 1 ou 2 hétéroatomes choisis parmi N, O ou S;
un énantiomère ou un sel pharmaceutiquement acceptable de celui-ci.

19. Préparation pharmaceutique selon la revendication 18, dans laquelle X, R, R₁, R₂, R₃, R₄, R₇, R₈ et R₉ sont tels que définis dans l'une quelconque des revendications 2-3, 5-14.

20. Composé selon l'une quelconque des revendications 4-14, destiné au traitement des troubles dans le système nerveux central, en particulier, des troubles ayant pour médiateur la 5-hydroxytryptamine.

21. Composé selon la revendication 20, destiné au traitement de la dépression, de l'anxiété, de l'anorexie, de la démence sénile, de la migraine, de la maladie d'Alzheimer, de l'hypertension, de la thermorégulation et des troubles sexuels, de la douleur et des troubles du système cardio-vasculaire.

22. Utilisation d'un composé selon l'une quelconque des revendications 4-14, pour la fabrication d'un médicament destiné au traitement des troubles du système nerveux central, en particulier, des troubles ayant pour médiateur la 5-hydroxytryptamine.

23. Utilisation selon la revendication 22, pour la fabrication d'un médicament destiné au traitement de la dépression, de l'anxiété, de l'anorexie, de la démence sénile, de la migraine, de la maladie d'Alzheimer, de la thermorégulation et des troubles sexuels, de la douleur et des troubles du système cardio-vasculaire.

24. Procédé de préparation d'un composé de formule I selon l'une quelconque des revendications 1-17, par
a) transformation d'un composé de formule II 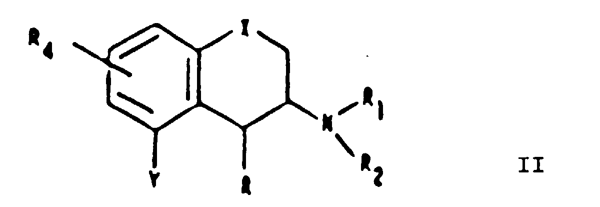 dans laquelle Y est un groupe partant et X, R, R₁, R₂ et R₄ sont tels que définis pour la formule I, à l'aide d'un cycle catalytique en utilisant un métal de transition non-valent (M°), qui subit une addition par oxydation sur les liaisons aryl-Y, traitement avec le monoxyde de carbone suivi par Z-H, où Z est Cl, Br, OH, ORₚ, où Rₚ est un groupe alkyle en C₁-C₆, et le complexe carbonylé o-aryl-métal-Y obtenu initialement, pour former un composé de formule I dans laquelle R₃ est COZ (IA), ou
b) réaction à l'aide d'un cycle catalytique en utilisant un métal de transition non-valent (M⁰) qui subit une addition par oxydation sur Z-Y, où Z est Cl, Br, OH, ORₚ, où Rₚ est un groupe alkyle en C₁-C₆, et Y est un groupe partant, traitement avec le monoxyde de carbone, suivi de l'addition du composé de formule III 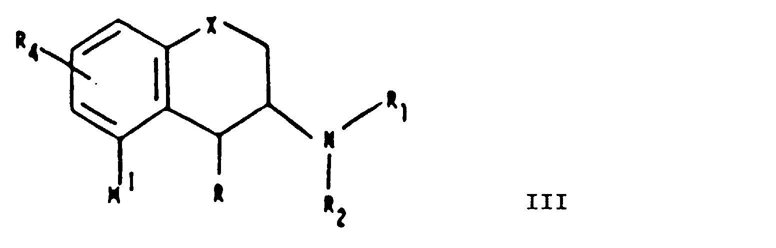 dans laquelle X, R, R₁, R₂ et R₄ sont tels que définis pour la formule I et M^{I} est un métal de transition, pour former un composé de formule I dans laquelle R₃ est COZ (IA), ou
c) transformation d'un composé de formule Il 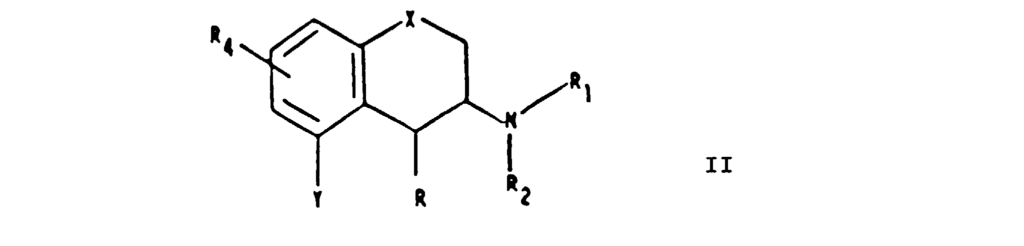 dans laquelle X, R, R₁, R₂ et R₄ sont tels que définis pour la formule I et Y est un groupe partant tel que SO₃CF₃, un halogénure, par traitement avec un réactif cyanuré, pour former un composé de formule I dans laquelle R₃ est CN (IB);
d) amination d'un composé de formule lA 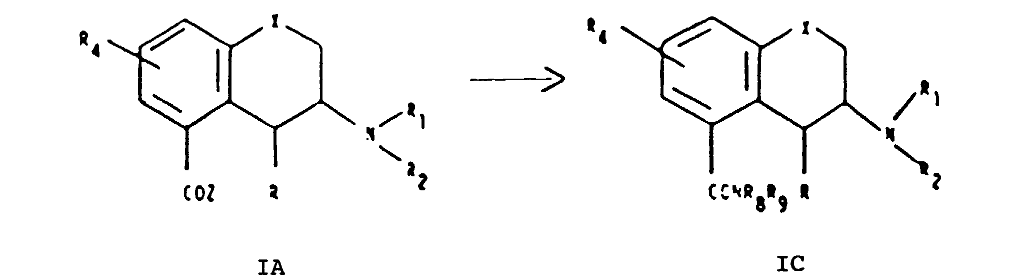 dans laquelle X, R, R₁, R₂ et R₄ sont tels que définis pour la formule I et Z est Cl, OH ou ORₚ, où Rₚ est un groupe alkyle en C₁-C₆, par réaction avec NHR₈R₉ pour donner l'amide correspondant de formule I, où R₃ est CONR₈R₉ (IC), ou
e) substitution d'un dérivé 5-bromochroman/thiochroman 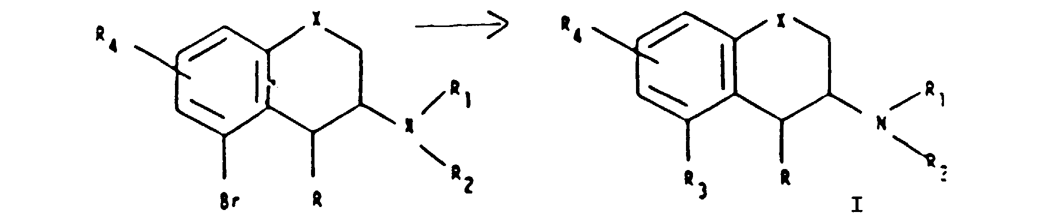 où X, R, R₁, R₂ et R₄ sont tels que définis pour la formule I, par traitement avec un réactif trialkylstannique, en présence d'un métal non-valent, par exemple, Pd°, pour obtenir un composé de formule I dans laquelle R₃ est un groupe aryle, ou en présence de monoxyde de carbone pour former un composé où R₃ est COR₇, où R₇ est un groupe alkyle en C₁-C₆, alcényle en C₂-C₆ ou aryle, ou
f) transformation du dérivé 5-carboxychroman/thiochroman de formule IA 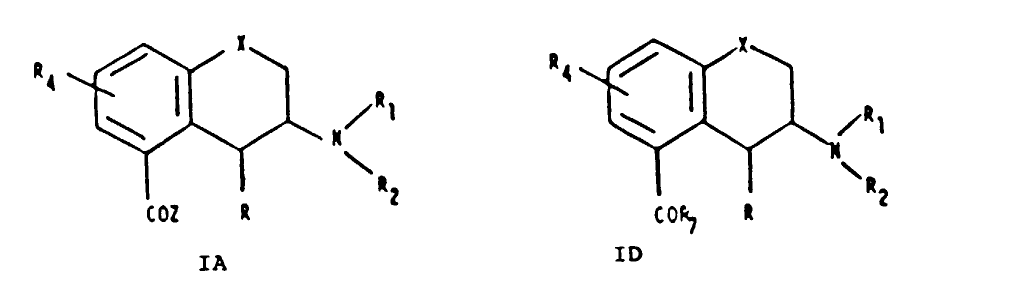 dans laquelle X, R, R₁, R₂ et R₄ sont tels que définis pour la formule I et Z est Cl, Br, en utilisant R₇Li pour obtenir le composé de formule I correspondant où R₃ est COR₇ (ID), où R₇ représente un groupe alkyle en C₁-C₆, alcényle en C₂-C₆ ou aryle, ou
g) hydrolyse d'un composé de formule I dans laquelle R₃ est CN (IB) 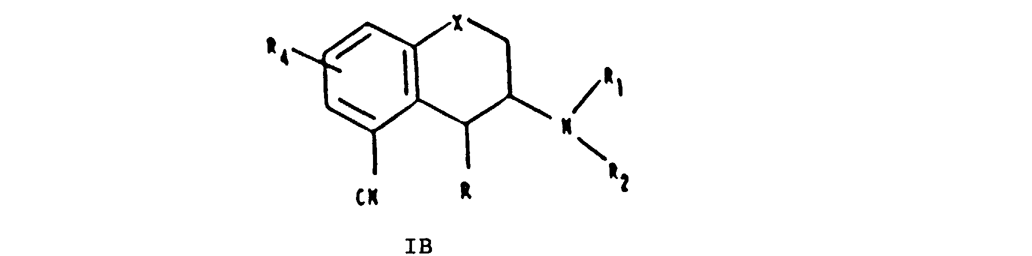 où X, R, R₁, R₂ et R₄ sont tels que définis pour la formule I, suivie éventuellement d'un traitement avec un halogénure de thionyle pour obtenir un composé de formule I dans laquelle R₃ est COZ, où Z est OH, Cl ou Br (IA), ou
h) substitution d'un composé de formule I dans laquelle R₃ est CN 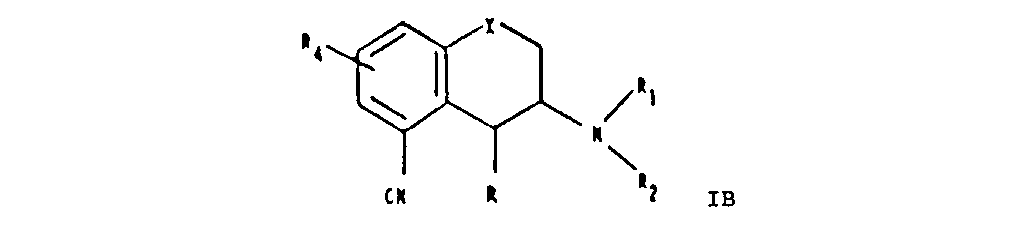 où X, R, R₁, R₂ et R₄ sont tels que définis pour la formule I, par traitement avec un réactif organométallique, suivi d'une hydrolyse pour obtenir un composé de formule I dans laquelle R₃ est COR₇, où R₇ est un groupe alkyle en C₁-C₆, alcényle en C₂-C₆ ou aryle (ID), ou
i) transformation d'un composé de formule II 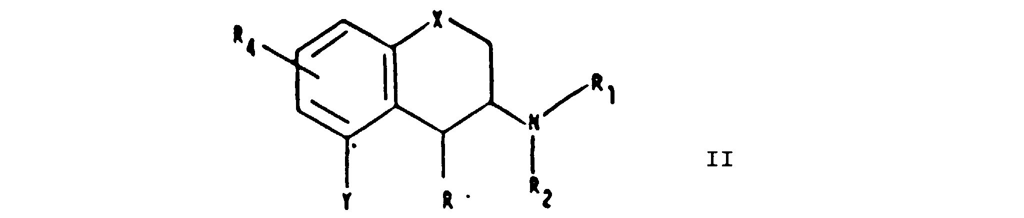 dans laquelle Y est un groupe partant et X, R, R₁, R₂ et R₄ sont tels que définis pour la formule I , par réaction avec un métal de transition pour former un complexe de ligands, qui subit une addition par oxydation par traitement avec un trialkylarylstannane ou des réactifs à l'acide aryl-borique, pour former un composé de formule I dans laquelle R₃ est un groupe aryle à 5 ou 6 chaînons, qui peut renfermer 1 ou 2 hétéroatomes choisis parmi N, O ou S et qui est soit substitué soit fusionné au niveau de deux atomes de carbone adjacents; suivi éventuellement de la transformation d'une base obtenue en un sel d'addition d'acide, ou d'un sel obtenu en la base ou en un sel d'addition d'acide différent, ou éventuellement de la séparation d'un mélange d'isomères obtenu en un énantiomère pur.

25. Composé selon l'une quelconque des revendications 11-14, destiné à être utilisé en thérapie.

## Revendications (Revendications pour l'(les) Etat(s) contractant(s) suivant(s): ES)

1. Procédé de préparation d'un composé de formule 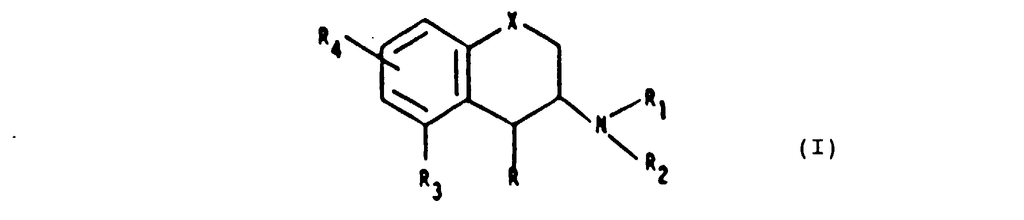 dans laquelle
X est O ou 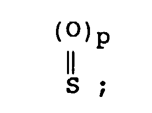
p est un nombre entier égal à 0, 1 ou 2;
R est un atome d'hydrogène, de fluor ou un groupe alkyle en C₁-C₆;
R₁ est un atome d'hydrogène, un groupe alkyle en C₁-C₆ ou alcényle en C₂-C₆;
R₂ est un atome d'hydrogène, un groupe alkyle en C₁-C₆, alcényle en C₂-C₆, alkylaryle en C₁-C₄, où le groupe aryle peut renfermer 1 ou 2 hétéroatomes choisis parmi N, O ou S, éventuellement substitué par un halogène, un groupe CN, CF₃, alkyle en C₁-C₆, alcényle en C₂-C₆ ou alcoxy en C₁-C₄; ou
R₃ est CN, SO₃CF₃, N₃, NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄, soit (ii) fusionné au niveau de deux atomes de carbone adjacents à un cycle aryle, ledit cycle aryle étant éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄;
R₄ est un atome d'hydrogène ou d'halogène;
R₅ est un atome d'hydrogène, un groupe alkyle en C₁-C₆ ou alcényle en C₂-C₆;
R₆ est un groupe alkyle en C₁-C₆ ou alcényle en C₂-C₆; ou
R₅ et R₆ peuvent former ensemble un cycle à 5 ou 6 chaînons qui peut renfermer 1 ou 2 hétéroatomes choisis parmi N, O ou S;
R₇ est un atome d'hydrogène, un hydroxy, un atome de chlore, de brome, un groupe alkyle en C₁-C₆, alcényle en C₂-C₆, alcoxy en C₁-C₄, NR₈R₉ ou un groupe aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
R₈ et R₉ représentent chacun indépendamment un atome d'hydrogène, un groupe alkyle en C₁-C₆, alcényle en C₂-C₆, aryle à 5 ou 6 chaînons qui peut renfermer 1 ou 2 hétéroatomes choisis parmi N, O ou S, éventuellement substitué par un atome d'halogène, un groupe CN, CF₃, alkyle en C₁-C₆, alcényle en C₂-C₆, alcoxy en C₁-C₄, ou peuvent former ensemble un cycle à 5 ou 6 chaînons renfermant 1 ou 2 hétéroatomes choisis parmi N, O ou S;
un énantiomère ou un sel de celui-ci, par
a) transformation d'un composé de formule II 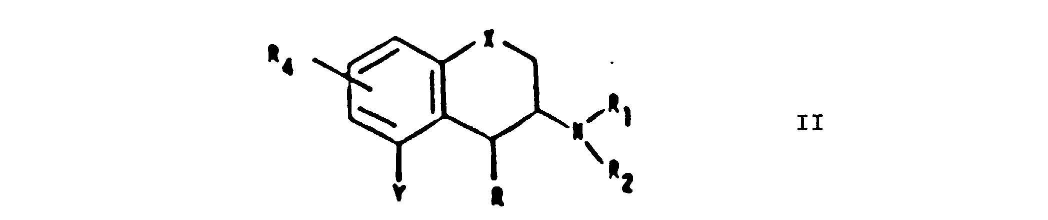 dans laquelle Y est un groupe partant et X, R, R₁, R₂ et R₄ sont tels que définis pour la formule I, à l'aide d'un cycle catalytique en utilisant un métal de transition non-valent (M°), qui subit une addition par oxydation sur les liaisons aryl-Y, traitement avec le monoxyde de carbone suivi par Z-H, où Z est Cl, Br, OH, ORₚ, où Rₚ est un groupe alkyle en C₁-C₆, et le complexe carbonylé o-aryl-métal-Y obtenu initialement, pour former un composé de formule I dans laquelle R₃ est COZ (IA), ou
b) réaction à l'aide d'un cycle catalytique en utilisant un métal de transition non-valent (M°) qui subit une addition par oxydation sur Z-Y, où Z est Cl, Br, OH, ORₚ, où Rₚ est un groupe alkyle en C₁-C₆, et Y est un groupe partant, traitement avec le monoxyde de carbone, suivi de l'addition du composé de formule III 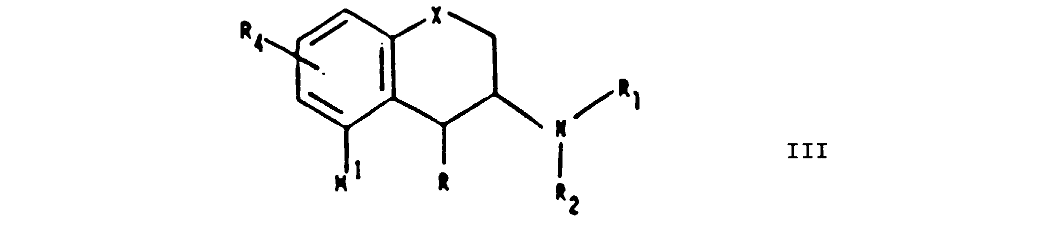 dans laquelle X, R, R₁, R₂ et R₄ sont tels que définis pour la formule I et M^{I} est un métal de transition, pour former un composé de formule I dans laquelle R₃ est COZ (IA), ou
c) transformation d'un composé de formule II 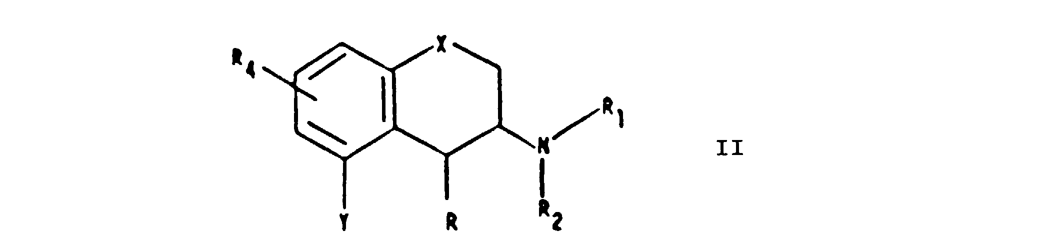 dans laquelle X, R, R₁, R₂ et R₄ sont tels que définis pour la formule I et Y est un groupe partant tel que SO₃CF₃, un halogénure, par traitement avec un réactif cyanuré, pour former un composé de formule I dans laquelle R₃ est CN (IB);
d) amination d'un composé de formule IA 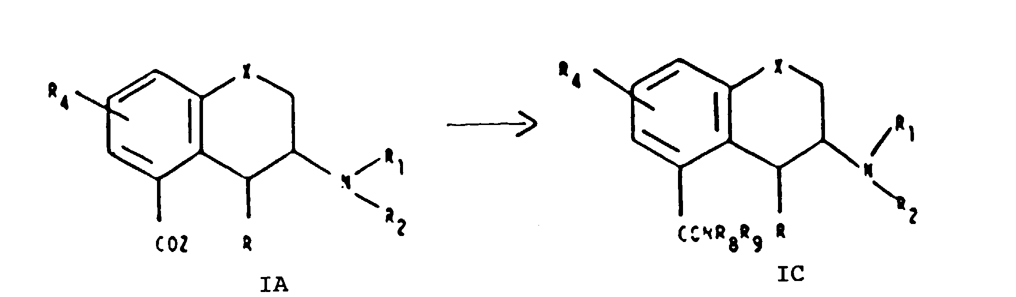 dans laquelle X, R, R₁, R₂ et R₄ sont tels que définis pour la formule I et Z est Cl, OH ou ORₚ, où Rₚ est un groupe alkyle en C₁-C₆, par réaction avec NHR₈R₉ pour donner l'amide correspondant de formule I, où R₃ est CONR₈R₉ (IC), ou
e) substitution d'un dérivé 5-bromochroman/thiochroman 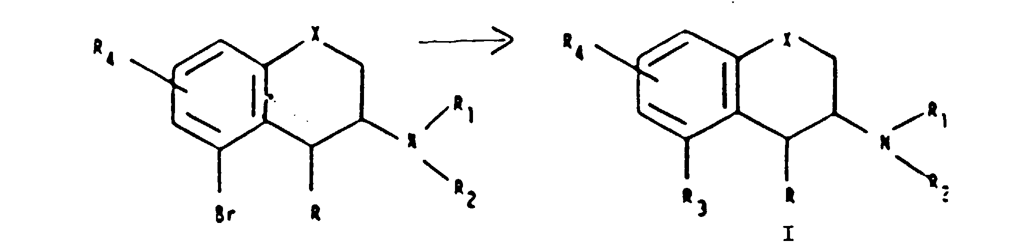 où X, R, R₁, R₂ et R₄ sont tels que définis pour la formule I, par traitement avec un réactif trialkylstannique, en présence d'un métal non-valent, par exemple, Pd°, pour obtenir un composé de formule I dans laquelle R₃ est un groupe aryle, ou en présence de monoxyde de carbone pour former un composé où R₃ est COR₇, où R₇ est un groupe alkyle en C₁-C₆, alcényle en C₂-C₆ ou aryle, ou
f) transformation du dérivé 5-carboxychroman/thiochroman de formule IA 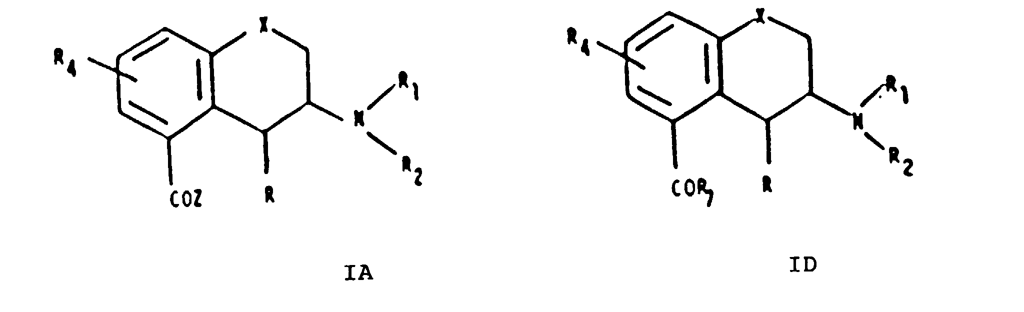 dans laquelle X, R, R₁, R₂ et R₄ sont tels que définis pour la formule I et Z est Cl, Br, en utilisant R₇Li pour obtenir le composé de formule I correspondant où R₃ est COR₇ (ID), où R₇ représente un groupe alkyle en C₁-C₆, alcényle en C₂-C₆ ou aryle, ou
g) hydrolyse d'un composé de formule I dans laquelle R₃ est CN (IB) 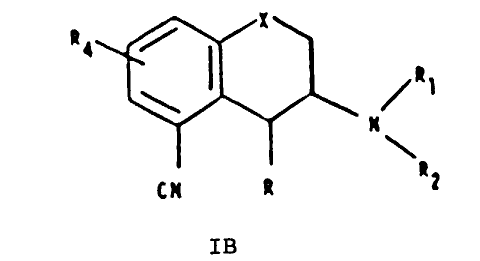 où X, R, R₁, R₂ et R₄ sont tels que définis pour la formule I, suivie éventuellement d'un traitement avec un halogénure de thionyle pour obtenir un composé de formule I dans laquelle R₃ est COZ, où Z est OH, Cl ou Br (IA), ou
h) substitution d'un composé de formule I dans laquelle R₃ est CN 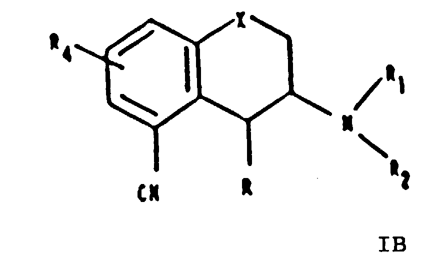 où X, R, R₁, R₂ et R₄ sont tels que définis pour la formule I, par traitement avec un réactif organométallique, suivi d'une hydrolyse pour obtenir un composé de formule I dans laquelle R₃ est COR₇, où R₇ est un groupe alkyle en C₁-C₆, alcényle en C₂-C₆ ou aryle (ID), ou
i) transformation d'un composé de formule II 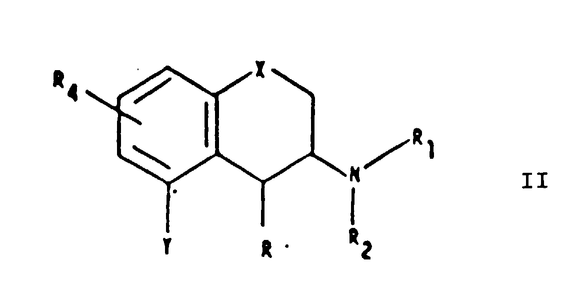 dans laquelle Y est un groupe partant et X, R, R₁, R₂ et R₄ sont tels que définis pour la formule I , par réaction avec un métal de transition pour former un complexe de ligands, qui subit une addition par oxydation par traitement avec un trialkylarylstannane ou des réactifs à l'acide aryl-borique, pour former un composé de formule I dans laquelle R₃ est un groupe aryle à 5 ou 6 chaînons, qui peut renfermer 1 ou 2 hétéroatomes choisis parmi N, O ou S et qui est soit substitué soit fusionné au niveau de deux atomes de carbone adjacents; suivi éventuellement de la transformation d'une base obtenue en un sel d'addition d'acide, ou d'un sel obtenu en la base ou en un sel d'addition d'acide différent, ou éventuellement de la séparation d'un mélange d'isomères obtenu en un énantiomère pur.

2. Procédé selon la revendication 1, pour la préparation d'un composé selon la revendication 1, dans lequel X est O.

3. Procédé selon la revendication 1, pour la préparation d'un composé selon la revendication 1, dans lequel X est 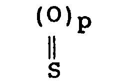 et p est égal à 0, 1 ou 2.

4. Procédé selon la revendication 1, pour la préparation d'un composé selon l'une quelconque des revendications 1, 2 ou 3, dans lequel R₃ est NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants indépendamment choisis parmi les halogènes, les groupes CN, CF₃, alkyle en C₁-C₆, alcényle en C₂-C₆ ou alcoxy en C₁-C₄, soit (ii) fusionné au niveau de deux atomes de carbone adjacents à un cycle aryle, ledit cycle aryle étant éventuellement substitué par un ou plusieurs substituants indépendamment choisis parmi les halogènes, les groupes CN, CF₃, alkyle en C₁-C₆, alcényle en C₂-C₆ ou alcoxy en C₁-C₄; R₇ est un atome d'hydrogène, un groupe alkyle en C₁-C₆, alcényle en C₂-C₆, alcoxy en C₁-C₄, NR₈R₉ ou aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
X, p, R, R₁, R₂, R₄, R₅, R₆, R₈ et R₉ sont tels que définis dans la revendication 1, un énantiomère ou un sel pharmaceutiquement acceptable de celui-ci, à utiliser en thérapie.

5. Procédé selon la revendication 1, pour la préparation d'un composé selon l'une quelconque des revendications 1-4, dans lequel R₁ et R₂ sont identiques ou différents et sont choisis parmi l'hydrogène, le n-propyle, l'i-propyle et le cyclopropyle.

6. Procédé selon la revendication 1, pour la préparation d'un composé selon l'une quelconque des revendications 1-5, dans lequel R₃ est COR₇.

7. Procédé selon la revendication 1, pour la préparation d'un composé selon la revendication 6, dans lequel R₇ est un groupe alkyle en C₁-C₄, phényle, furanyle ou thiényle éventuellement substitué par un halogène, NR₈R₉ où R₈ et R₉ sont chacun indépendamment un atome d'hydrogène ou un groupe alkyle en C₁-C₄, ou alcoxy en C₁-C₄.

8. Procédé selon la revendication 1, pour la préparation d'un composé selon la revendication 6, dans lequel R et R₄ sont des atomes d'hydrogène, R₁ et R₂ sont des groupes n-propyle et R₇ est un groupe méthyle, éthyle, n-propyle, i-propyle, cyclopropyle, n-butyle, i-butyle, t-butyle, cyclobutyle, thiényle, furanyle, phényle, N-méthylamino, méthoxy ou fluorophényle.

9. Procédé selon la revendication 1, pour la préparation d'un composé selon l'une quelconque des revendications 1-5, dans lequel R₃ est un groupe phényle, furanyle, thiényle ou fluorophényle.

10. Procédé selon la revendication 1, pour la préparation d'un composé selon l'une quelconque des revendications 1-7 et 9, dans lequel R₄ est un atome d'halogène en position 8.

11. Procédé selon la revendication 1, pour la préparation du 3-dipropylamino-5-acétylchroman, un énantiomère ou un sel de celui-ci.

12. Procédé selon la revendication 1, pour la préparation du 3-dipropylamino-5-carbamoylchroman, un énantiomère ou un sel de celui-ci.

13. Procédé selon la revendication 1, pour la préparation du 3-dipropylamino-5-N-méthylcarbamoylchroman, un énantiomère ou un sel de celui-ci.

14. Procédé selon la revendication 1, pour la préparation du 3-dipropylamino-5-(2-thiénylcarbonyl)chroman, un énantiomère ou un sel de celui-ci.

15. Procédé selon la revendication 1, pour la préparation d'un composé selon la revendication 1, dans lequel R₃ est CN, COOH, COCl, COBr, N₃ ou SO₃CF₃, et X, R, R₁, R₂ et R₄ sont tels que définis dans la revendication 1, un énantiomère ou un sel de celui-ci.

16. Procédé selon la revendication 1, pour la préparation d'un composé selon la revendication 15, dans lequel R₁ et R₂ sont identiques ou différents et sont choisis parmi l'hydrogène, le n-propyle, l'i-propyle et le cyclopropyle.

17. Procédé selon la revendication 1, pour la préparation d'un composé selon la revendication 16, dans lequel R et R₄ sont des atomes d'hydrogène, R₁ et R₂ sont des n-propyle et R₃ est SO₃CF₃.

18. Utilisation d'un composé selon l'une quelconque des revendications 4-14, pour la fabrication d'un médicament destiné au traitement des troubles du système nerveux central, en particulier, des troubles ayant pour médiateur la 5-hydroxytryptamine.

19. Utilisation selon la revendication 18, pour la fabrication d'un médicament destiné au traitement de la dépression, de l'anxiété, de l'anorexie, de la démence sénile, de la migraine, de la maladie d'Alzheimer, de la thermorégulation et des troubles sexuels, de la douleur et des troubles du système cardio-vasculaire.

## Revendications (Revendications pour l'(les) Etat(s) contractant(s) suivant(s): GR)

1. Procédé de préparation d'un composé de formule 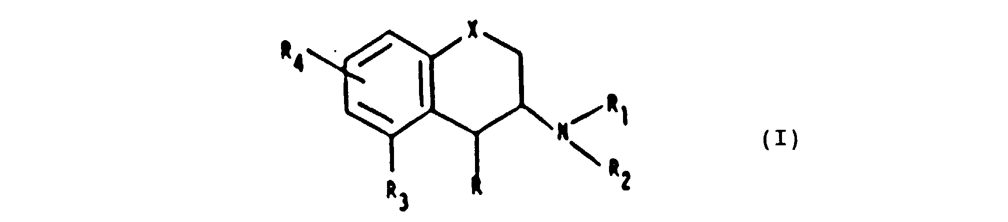 dans laquelle
X est O ou 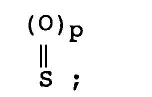
p est un nombre entier égal à 0, 1 ou 2;
R est un atome d'hydrogène, de fluor ou un groupe alkyle en C₁-C₆;
R₁ est un atome d'hydrogène, un groupe alkyle en C₁-C₆ ou alcényle en C₂-C₆;
R₂ est un atome d'hydrogène, un groupe alkyle en C₁-C₆, alcényle en C₂-C₆, alkylaryle en C₁-C₄, où le groupe aryle peut renfermer 1 ou 2 hétéroatomes choisis parmi N, O ou S, éventuellement substitué par un halogène, un groupe CN, CF₃, alkyle en C₁-C₆, alcényle en C₂-C₆ ou alcoxy en C₁-C₄; ou
R₃ est CN, SO₃CF₃, N₃, NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄, soit (ii) fusionné au niveau de deux atomes de carbone adjacents à un cycle aryle, ledit cycle aryle étant éventuellement substitué par un ou plusieurs substituants choisis indépendamment parmi les halogènes, les groupes CN, CF₃, alkyle en C₁-C₆, alcényle en C₂-C₆ ou alcoxy en C₁-C₄;
R₄ est un atome d'hydrogène ou d'halogène;
R₅ est un atome d'hydrogène, un groupe alkyle en C₁-C₆ ou alcényle en C₂-C₆;
R₆ est un groupe alkyle en C₁-C₆ ou alcényle en C₂-C₆; ou
R₅ et R₆ peuvent former ensemble un cycle à 5 ou 6 chaînons qui peut renfermer 1 ou 2 hétéroatomes choisis parmi N, O ou S;
R₇ est un atome d'hydrogène, un hydroxy, un atome de chlore, de brome, un groupe alkyle en C₁-C₆, alcényle en C₂-C₆, alcoxy en C₁-C₄, NR₈R₉ ou un groupe aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
R₈ et R₉ représentent chacun indépendamment un atome d'hydrogène, un groupe alkyle en C₁-C₆, alcényle en C₂-C₆, aryle à 5 ou 6 chaînons qui peut renfermer 1 ou 2 hétéroatomes choisis parmi N, O ou S, éventuellement substitué par un atome d'halogène, un groupe CN, CF₃, alkyle en C₁-C₆, alcényle en C₂-C₆, alcoxy en C₁-C₄, ou peuvent former ensemble un cycle à 5 ou 6 chaînons renfermant 1 ou 2 hétéroatomes choisis parmi N, O ou S;
un énantiomère ou un sel de celui-ci, par
a) transformation d'un composé de formule II 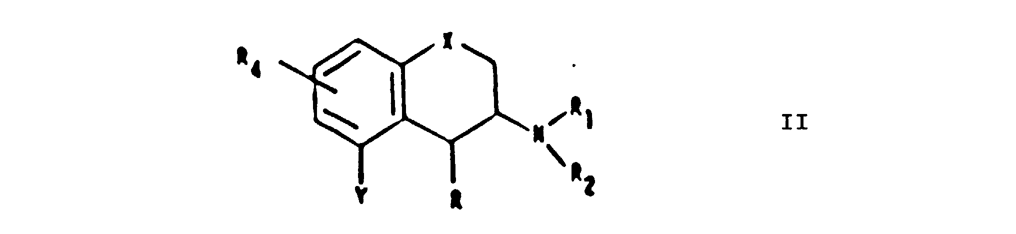 dans laquelle Y est un groupe partant et X, R, R₁, R₂ et R₄ sont tels que définis pour la formule I, à l'aide d'un cycle catalytique en utilisant un métal de transition non-valent (M°), qui subit une addition par oxydation sur les liaisons aryl-Y, traitement avec le monoxyde de carbone suivi par Z-H, où Z est Cl, Br, OH, ORₚ, où Rₚ est un groupe alkyle en C₁-C₆, et le complexe carbonylé o-aryl-métal-Y obtenu initialement, pour former un composé de formule I dans laquelle R₃ est COZ (IA), ou
b) réaction à l'aide d'un cycle catalytique en utilisant un métal de transition non-valent (M°) qui subit une addition par oxydation sur Z-Y, où Z est Cl, Br, OH, ORₚ, où Rₚ est un groupe alkyle en C₁-C₆, et Y est un groupe partant, traitement avec le monoxyde de carbone, suivi de l'addition du composé de formule III 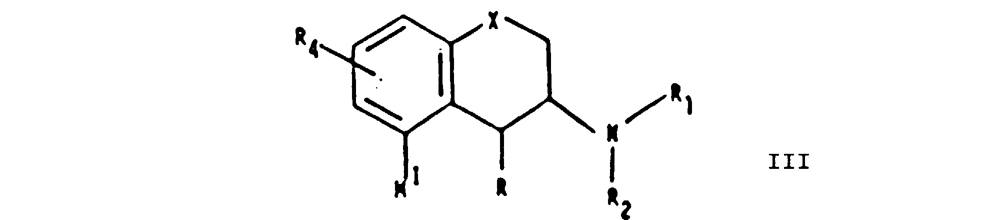 dans laquelle X, R, R₁, R₂ et R₄ sont tels que définis pour la formule I et M^{I} est un métal de transition, pour former un composé de formule I dans laquelle R₃ est COZ (IA), ou
c) transformation d'un composé de formule II 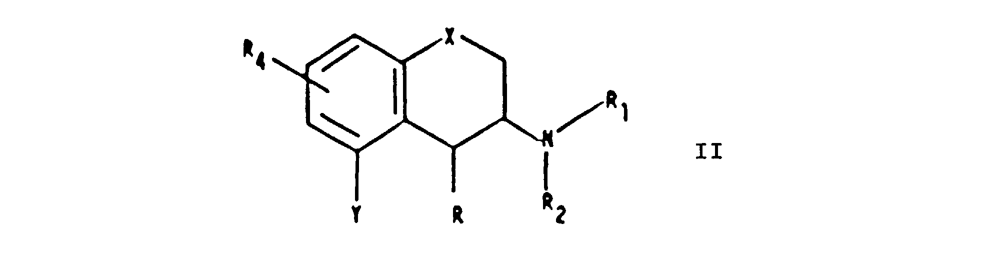 dans laquelle X, R, R₁, R₂ et R₄ sont tels que définis pour la formule I et Y est un groupe partant tel que SO₃CF₃, un halogénure, par traitement avec un réactif cyanuré, pour former un composé de formule I dans laquelle R₃ est CN (IB);
d) amination d'un composé de formule IA 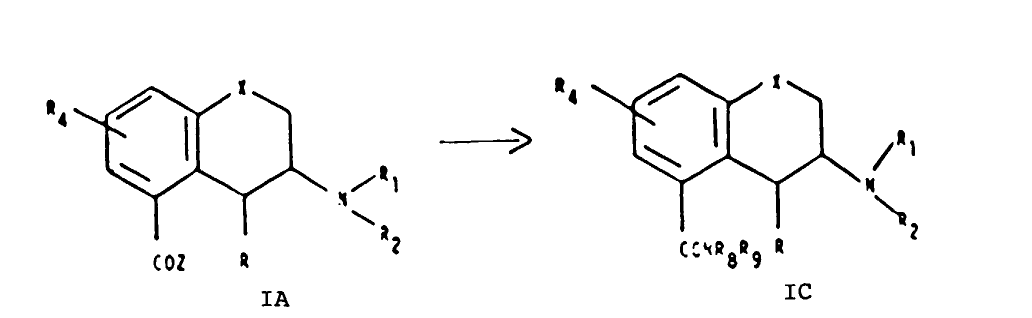 dans laquelle X, R, R₁, R₂ et R₄ sont tels que définis pour la formule I et Z est Cl, OH ou ORₚ, où Rₚ est un groupe alkyle en C₁-C₆, par réaction avec NHR₈R₉ pour donner l'amide correspondant de formule I, où R₃ est CONR₈R₉ (IC), ou
e) substitution d'un dérivé 5-bromochroman/thiochroman 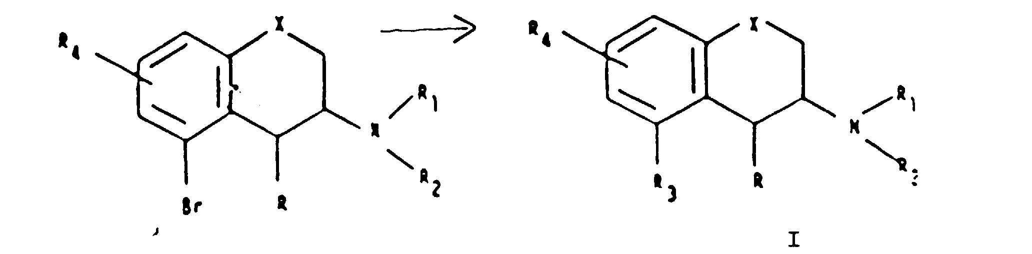 où X, R, R₁, R₂ et R₄ sont tels que définis pour la formule I, par traitement avec un réactif trialkylstannique, en présence d'un métal non-valent, par exemple, Pd°, pour obtenir un composé de formule I dans laquelle R₃ est un groupe aryle, ou en présence de monoxyde de carbone pour former un composé où R₃ est COR₇, où R₇ est un groupe alkyle en C₁-C₆, alcényle en C₂-C₆ ou aryle, ou
f) transformation du dérivé 5-carboxychroman/thiochroman de formule IA 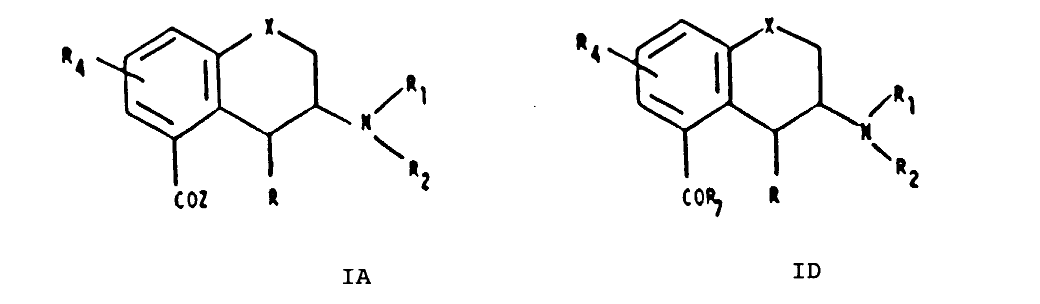 dans laquelle X, R, R₁, R₂ et R₄ sont tels que définis pour la formule I et Z est Cl, Br, en utilisant R₇Li pour obtenir le composé de formule I correspondant où R₃ est COR₇ (ID), où R₇ représente un groupe alkyle en C₁-C₆, alcényle en C₂-C₆ ou aryle, ou
g) hydrolyse d'un composé de formule I dans laquelle R₃ est CN (IB) 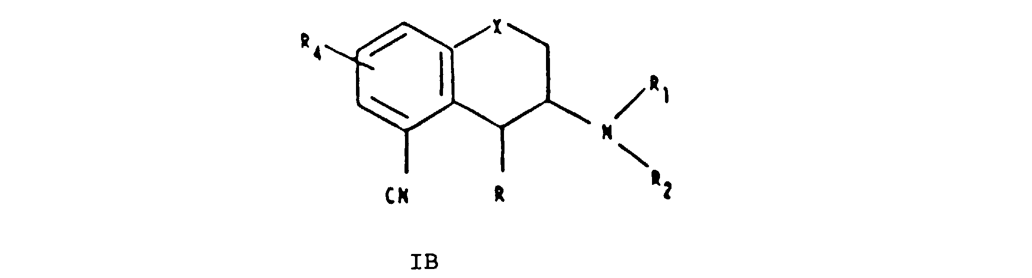 où X, R, R₁, R₂ et R₄ sont tels que définis pour la formule I, suivie éventuellement d'un traitement avec un halogénure de thionyle pour obtenir un composé de formule I dans laquelle R₃ est COZ, où Z est OH, Cl ou Br (IA), ou
h) substitution d'un composé de formule I dans laquelle R₃ est CN 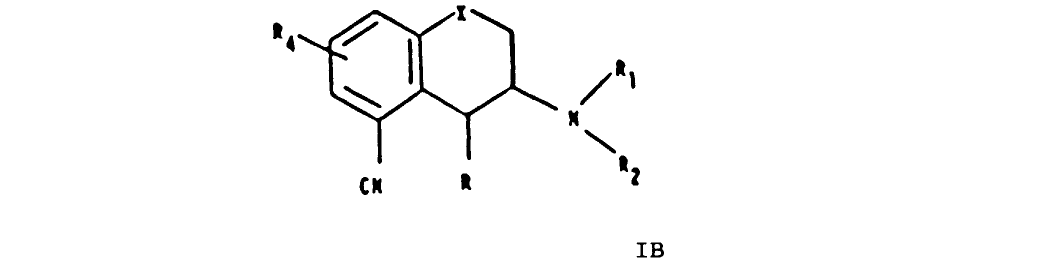 où X, R, R₁, R₂ et R₄ sont tels que définis pour la formule I, par traitement avec un réactif organométallique, suivi d'une hydrolyse pour obtenir un composé de formule I dans laquelle R₃ est COR₇, où R₇ est un groupe alkyle en C₁-C₆, alcényle en C₂-C₆ ou aryle (ID), ou
i) transformation d'un composé de formule II 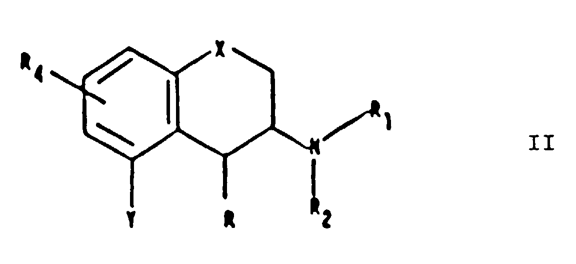 dans laquelle Y est un groupe partant et X, R, R₁, R₂ et R₄ sont tels que définis pour la formule I , par réaction avec un métal de transition pour former un complexe de ligands, qui subit une addition par oxydation par traitement avec un trialkylarylstannane ou des réactifs à l'acide aryl-borique, pour former un composé de formule I dans laquelle R₃ est un groupe aryle à 5 ou 6 chaînons, qui peut renfermer 1 ou 2 hétéroatomes choisis parmi N, O ou S et qui est soit substitué soit fusionné au niveau de deux atomes de carbone adjacents; suivi éventuellement de la transformation d'une base obtenue en un sel d'addition d'acide, ou d'un sel obtenu en la base ou en un sel d'addition d'acide différent, ou éventuellement de la séparation d'un mélange d'isomères obtenu en un énantiomère pur.

2. Procédé selon la revendication 1, pour la préparation d'un composé selon la revendication 1, dans lequel X est O.

3. Procédé selon la revendication 1, pour la préparation d'un composé selon la revendication 1, dans lequel
X est 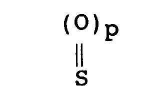 et p est égal à 0, 1 ou 2.

4. Procédé selon la revendication 1, pour la préparation d'un composé selon l'une quelconque des revendications 1, 2 ou 3, dans lequel R₃ est NR₅R₆, COR₇, un groupe aryle à 5 ou 6 chaînons qui peut renfermer 1 ou 2 hétéroatomes choisis parmi N, O ou S et qui est soit (i) éventuellement substitué par un ou plusieurs substituants indépendamment choisis parmi les halogènes, les groupes CN, CF₃, alkyle en C₁-C₆, alcényle en C₂-C₆ ou alcoxy en C₁-C₄, soit (ii) fusionné au niveau de deux atomes de carbone adjacents à un cycle aryle, ledit cycle aryle étant éventuellement substitué par un ou plusieurs substituants indépendamment choisis parmi les halogènes, les groupes CN, CF₃, alkyle en C₁-C₆, alcényle en C₂-C₆ ou alcoxy en C₁-C₄; R₇ est un atome d'hydrogène, un groupe alkyle en C₁-C₆, alcényle en C₂-C₆, alcoxy en C₁-C₄, NR₈R₉ ou aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
X, p, R, R₁, R₂, R₄, R₅, R₆, R₈ et R₉ sont tels que définis dans la revendication 1, un énantiomère ou un sel pharmaceutiquement acceptable de celui-ci, à utiliser en thérapie.

5. Procédé selon la revendication 1, pour la préparation d'un composé selon l'une quelconque des revendications 1-4, dans lequel R₁ et R₂ sont identiques ou différents et sont choisis parmi l'hydrogène, le n-propyle, l'i-propyle et le cyclopropyle.

6. Procédé selon la revendication 1, pour la préparation d'un composé selon l'une quelconque des revendications 1-5, dans lequel R₃ est COR₇.

7. Procédé selon la revendication 1, pour la préparation d'un composé selon la revendication 6, dans lequel R₇ est un groupe alkyle en C₁-C₄, phényle, furanyle ou thiényle éventuellement substitué par un halogène, NR₈R₉ où R₈ et R₉ sont chacun indépendamment un atome d'hydrogène ou un groupe alkyle en C₁-C₄, ou alcoxy en C₁-C₄.

8. Procédé selon la revendication 1, pour la préparation d'un composé selon la revendication 6, dans lequel R et R₄ sont des atomes d'hydrogène, R₁ et R₂ sont des groupes n-propyle et R₇ est un groupe méthyle, éthyle, n-propyle, i-propyle, cyclopropyle, n-butyle, i-butyle, t-butyle, cyclobutyle, thiényle, furanyle, phényle, N-méthylamino, méthoxy ou fluorophényle.

9. Procédé selon la revendication 1, pour la préparation d'un composé selon l'une quelconque des revendications 1-5, dans lequel R₃ est un groupe phényle, furanyle, thiényle ou fluorophényle.

10. Procédé selon la revendication 1, pour la préparation d'un composé selon l'une quelconque des revendications 1-7 et 9, dans lequel R₄ est un atome d'halogène en position 8.

11. Procédé selon la revendication 1, pour la préparation du 3-dipropylamino-5-acétylchroman, un énantiomère ou un sel de celui-ci.

12. Procédé selon la revendication 1, pour la préparation du 3-dipropylamino-5-carbamoylchroman, un énantiomère ou un sel de celui-ci.

13. Procédé selon la revendication 1, pour la préparation du 3-dipropylamino-5-N-méthylcarbamoylchroman, un énantiomère ou un sel de celui-ci.

14. Procédé selon la revendication 1, pour la préparation du 3-dipropylamino-5-(2-thiénylcarbonyl)chroman, un énantiomère ou un sel de celui-ci.

15. Utilisation d'un composé selon l'une quelconque des revendications 4-14 pour la fabrication d'un médicament destiné au traitement des troubles du système nerveux central, en particulier, des troubles ayant pour médiateur la 5-hydroxytryptamine.

16. Utilisation selon la revendication 15, pour la fabrication d'un médicament destiné au traitement de la dépression, de l'anxiété, de l'anorexie, de la démence sénile, de la migraine, de la maladie d'Alzheimer, de la thermorégulation et des troubles sexuels, de la douleur et des troubles du système cardio-vasculaire.

17. Composé de formule 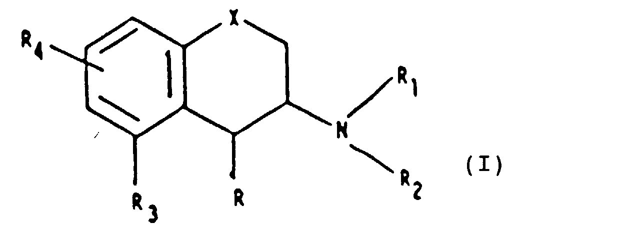 dans laquelle
X est O ou 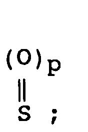
p est un nombre entier égal à 0, 1 ou 2;
R est un atome d'hydrogène, de fluor ou un groupe alkyle en C₁-C₆;
R₁ est un atome d'hydrogène, un groupe alkyle en C₁-C₆ ou alcényle en C₂-C₆;
R₂ est un atome d'hydrogène, un groupe alkyle en C₁-C₆, alcényle en C₂-C₆, alkylaryle en C₁-C₄, où le groupe aryle peut renfermer 1 ou 2 hétéroatomes choisis parmi N, O ou S, éventuellement substitué par un halogène, un groupe CN, CF₃, alkyle en C₁-C₆, alcényle en C₂-C₆ ou alcoxy en C₁-C₄; ou
R₃ est CN, COOH, COCl, COBr, N₃ ou SO₃CF₃;
R₄ est un atome d'hydrogène ou d'halogène;
R₅ est un atome d'hydrogène, un groupe alkyle en C₁-C₆ ou alcényle en C₂-C₆;
R₆ est un groupe alkyle en C₁-C₆ ou alcényle en C₂-C₆; ou
R₅ et R₆ peuvent former ensemble un cycle à 5 ou 6 chaînons qui peut renfermer 1 ou 2 hétéroatomes choisis parmi N, O ou S;
R₇ est un atome d'hydrogène, un hydroxy, un atome de chlore, de brome, un groupe alkyle en C₁-C₆, alcényle en C₂-C₆, alcoxy en C₁-C₄, NR₈R₉ ou un groupe aryle à 5 ou 6 chaînons qui peut renfermer 1 ou 2 hétéroatomes choisis parmi N, O ou S, éventuellement substitué par un ou plusieurs substituants parmi les halogènes, les groupes CN, CF₃, alkyle en C₁-C₆, alcényle en C₂-C₆ ou alcoxy en C₁-C₄;
R₈ et R₉ représentent chacun indépendamment un atome d'hydrogène, un groupe alkyle en C₁-C₆, alcényle en C₂-C₆, aryle à 5 ou 6 chaînons qui peut renfermer 1 ou 2 hétéroatomes choisis parmi N, O ou S, éventuellement substitué par un atome d'halogène, un groupe CN, CF₃, alkyle en C₁-C₆, alcényle en C₂-C₆, alcoxy en c₁-C₄, ou peuvent former ensemble un cycle à 5 ou 6 chaînons renfermant 1 ou 2 hétéroatomes choisis parmi N, O ou S;
un énantiomère ou un sel de celui-ci.

18. Composé selon la revendication 17, dans lequel R₁ et R₂ sont identiques ou différents et sont choisis parmi l'hydrogène, le n-propyle, l'i-propyle et le cyclopropyle.

19. Composé selon la revendication 18, dans lequel R et R₄ sont des atomes d'hydrogène, R₁ et R₂ sont des groupes n-propyle et R₃ est SO₃CF₃.
